# EUROPEAN PATENT SPECIFICATION

(11) **EP 1 654 272 B1**
(45) Date of publication and mention of the grant of the patent: **09.09.2009**
(21) Application number: 04763685.7
(22) Date of filing: 30.07.2004
(51) Int. Cl.: C07K 1/22, C07K 1/32, C07K 14/47, G01N 33/569, A61K 38/00, A61K 39/00

(54) **RA ANTIGENIC PEPTIDES**
RA ANTIGENE PEPTIDE
PEPTIDES ANTIGENIQUES DE L'ARTHRITE RHUMATOIDE

(30) Priority: 07.08.2003 EP 03017551
(43) Date of publication of application: 10.05.2006
(73) Proprietor: F. Hoffmann-La Roche AG, 4070 Basel (CH)
(72) Inventor: BERNTENIS, Nikolaos, F-68300 St. Louis (FR); BUURMAN, Gerrit, 79576 Weil am Rhein (DE); KROPSHOFER, Harald, 79539 Loerrach (DE); MUELLER, Bernd, Christian, 79576 Weil am Rhein (DE); SPINDELDREHER, Sebastian, Thomas, 79539 Loerrach (DE); VOGT, Anne, 79539 Loerrach (DE); ZOLG, Werner, 82362 Weilheim (DE)
(74) Representative: Heiroth, Ulrike Hildegard
(86) International application number: PCT/EP2004/008609
(87) International publication number: WO 2005/014622

(56) References cited:
- WO-A1-93/18153
- DATABASE Geneseq [Online] 29 January 2004 (2004-01-29), "Human Protein P13284, SEQ ID NO 10787." XP002305086 retrieved from EBI accession no. GSN:ADD45354 Database accession no. ADD45354 & WO 03/016475 A (BAYER AG ; D URSO DONATELLA (DE); BEFORT KATIA (FR); GEN HOSPITAL CORP) 27 February 2003 (2003-02-27)
- DATABASE Geneseq [Online] 22 April 2004 (2004-04-22), "Human apoptosis-associated protein SEQ ID 154." XP002304913 retrieved from EBI accession no. GSN:ADI62711 Database accession no. ADI62711 & WO 03/058021 A (XANTOS BIOMEDICINE AG ; KESPER BJOERN (DE); SCHAEFER ROLF (DE); KAZINS) 17 July 2003 (2003-07-17)
- DATABASE Geneseq [Online] 19 August 2002 (2002-08-19), "Human peptide encoded by genome-derived single exon probe SEQ ID 30218." XP002315338 retrieved from EBI accession no. GSN:ABG40553 Database accession no. ABG40553 & WO 01/86003 A2 (MOLECULAR DYNAMICS, INC., USA) 15 November 2001 (2001-11-15)
- DI BARTOLO, V. ET AL: "Binding of human GM-CSF to synthetic peptides of the alpha subunit of its receptor" JOURNAL OF RECEPTOR AND SIGNAL TRANSDUCTION RESEARCH , 16(1 & 2), 77-92 CODEN: JRETET; ISSN: 1079-9893, 1996, XP009043176
- CARR P D ET AL: "Structure of the complete extracellular domain of the common beta subunit of the human GM-CSF, IL-3, and IL-5 receptors reveals a novel dimer configuration." CELL. 26 JAN 2001, vol. 104, no. 2, 26 January 2001 (2001-01-26), pages 291-300, XP002315334 ISSN: 0092-8674
- TSARK ELEANOR C ET AL: "Differential MHC class II-mediated presentation of rheumatoid arthritis autoantigens by human dendritic cells and macrophages." JOURNAL OF IMMUNOLOGY (BALTIMORE, MD. : 1950) 1 DEC 2002, vol. 169, no. 11, 1 December 2002 (2002-12-01), pages 6625-6633, XP002304908 ISSN: 0022-1767 cited in the application
- SANTAMBROGIO L ET AL: "Extracellular antigen processing and presentation by immature dendritic cells" PROCEEDINGS OF THE NATIONAL ACADEMY OF SCIENCES OF USA, NATIONAL ACADEMY OF SCIENCE. WASHINGTON, US, vol. 96, no. 26, 21 December 1999 (1999-12-21), pages 15056-15061, XP002222004 ISSN: 0027-8424
- FRIEDE T ET AL: "Natural ligand motifs of closely related HLA-DR4 molecules predict features of rheumatoid arthritis associated peptides." BIOCHIMICA ET BIOPHYSICA ACTA. 7 JUN 1996, vol. 1316, no. 2, 7 June 1996 (1996-06-07), pages 85-101, XP002304909 ISSN: 0006-3002
- DONGRE A R ET AL: "In vivo MHC class II presentation of cytosolic proteins revealed by rapid automated tandem mass spectrometry and functional analyses." EUROPEAN JOURNAL OF IMMUNOLOGY. MAY 2001, vol. 31, no. 5, May 2001 (2001-05), pages 1485-1494, XP002304910 ISSN: 0014-2980 cited in the application
- CHICZ R M ET AL: "SPECIFICITY AND PROMISCUITY AMONG NATURALLY PROCESSED PEPTIDES BOUND TO HLA-DR ALLELES" JOURNAL OF EXPERIMENTAL MEDICINE, TOKYO, JP, vol. 178, no. 1, 1 July 1993 (1993-07-01), pages 27-47, XP002069888 ISSN: 0022-1007 cited in the application
- ARUNACHALAM B ET AL: "Intracellular formation and cell surface expression of a complex of an intact lysosomal protein and MHC class II molecules." JOURNAL OF IMMUNOLOGY (BALTIMORE, MD. : 1950) 15 JUN 1998, vol. 160, no. 12, 15 June 1998 (1998-06-15), pages 5797-5806, XP002304911 ISSN: 0022-1767
- DATABASE UniProt [Online] 1 November 1996 (1996-11-01), "Cytokine receptor complex common beta chain h beta c (Fragment)." XP002315339 retrieved from EBI accession no. UNIPROT:Q63968 Database accession no. Q63968
- CANTAGREL A ET AL: "Interleukin-1beta, interleukin-1 receptor antagonist, interleukin-4, and interleukin-10 gene polymorphisms: relationship to occurrence and severity of rheumatoid arthritis." ARTHRITIS AND RHEUMATISM. JUN 1999, vol. 42, no. 6, June 1999 (1999-06), pages 1093-1100, XP002315335 ISSN: 0004-3591
- RAMMENSEE H-G ET AL: "MHC LIGANDS AND PEPTIDE MOTIFS: FIRST LISTING" IMMUNOGENETICS, SPRINGER VERLAG, BERLIN, DE, vol. 41, no. 4, February 1995 (1995-02), pages 178-228, XP000673045 ISSN: 0093-7711

## Description

The present invention provides novel naturally-processed RA antigenic peptides which are candidate markers for erosive and non-erosive RA. These antigenic peptides are presented by human MHC class II HLA-DR molecules. Moreover, these antigenic peptides linked to MHC class II molecules, as well as antibodies reactive with said antigenic peptides, nucleic acids encoding said antigenic peptides, and nucleic acid constructs and host cells for expressing said antigenic peptides are provided. The antigenic peptides of the invention as well as the polypeptides they are derived from can be used as markers in diagnosis of RA and in therapy as anti-RA vaccines.

Rheumatoid Arthritis (RA), originally termed chronic polyarthritis, is a systemic autoimmune disease and one of the most debilitating forms of articular inflammation (Feldmann, M. et al., Cell 85 (1996) 307-310; Dedhia, H.V. & DiBartolomeo, A., Critical care clinics 18 (2002) 841-854). Typically, RA causes joint pain, deformities and severe joint stiffness. The disease can also have its manifestation outside the joints, especially in patients who are positive for an autoantibody, termed "rheumatoid factor" (RF) (Mageed, R.A., in: van Venrooij, W.J. & Maini, RN. eds., Manual of biological markers of disease, Kluwer Academic Publishers (1996) 1-18). RA occurs quite frequently in the Caucasian population with the susceptibility to RA being influenced by genetic and environmental factors. Both have a crucial effect on the onset and the progression of this autoimmune disease. Approximately 4% of the total population has an increased genetic susceptibility to RA, roughly 20% of which (around 1% of the total population) develops RA as a result of, as yet, uncharacterized non-inheritable factors. Beyond that, RA shows a significant bias in the sex ratio: women have a three fold higher risk for RA than men, indicating that sex hormones may also be involved in the pathogenesis.

In the beginning, RA progresses slowly. Typical early stage symptoms are palm sweating, morning stiffness of fingers and symmetrical joint inflammation. In addition, rheumatoid nodules can appear which is an indication for tissue affection outside the joints. In a simplified model, the immune system produces autoantibodies against healthy tissue. These autoantibodies attack the articular cartilage in the joint leading to its inflammation and later on to its destruction. This destruction stimulates the immune system to produce more autoantibodies. In addition, cytokines like tumor necrosis-factor alpha (TNF-α) and Interleukin-1 (IL-1) are produced which enhance the inflammatory reaction even further (Houssiau, F.A., Clin Rheumatol 14 Suppl 2 (1995) 10-13). The synovium begins to swell due to infiltration of additional cells of the immune system, such as macrophages and T cells. These cells are actively involved in causing further cell death and in driving joint inflammation (Fox, D.A., Arthritis Rheum 40 (1997) 598-609; Choy, E.H. & Panayi, G.S., N Engl J Med 344 (2001) 907-916). This process resembles a vicious circle of autoantibody production, joint inflammation and joint destruction.

Typically, RA progresses chronically, with 85-90% of all RA patients showing a mild to moderate disease development. Aggressive disease forms leading to complete loss of joint function up to the degree of invalidity is experienced by 10-15% of the patients. In this advanced RA state, patients have a permanent articular inflammation and display rheumatoid nodules. They suffer from strong chronical pain and the inflammation leads to severe finger stiffness and irreversible joint deformations or dislocations.

### Diagnosis

There is growing evidence that therapeutic intervention early in the disease can reduce the extent of joint damage (Egsmose, C. et al., J Rheumatol 22 (1995) 2208-2213; Van der Heide, A. et al., Ann Intern Med 124 (1996) 699-707). Since treatment with disease-modifying antirheumatic drugs (DMARDs) is only justified when the risk:benefit or cost:effectiveness ratios are favorable, it is mandatory to be able to differentiate between RA and other forms of arthritis shortly after onset of the disease (Kirwan, J.R. & Quilty, B., Clin Exp Rheumatol 15 (1997) 15-25). The diagnosis is made by established criteria based on clinical history, physical examination and laboratory tests. The American Society of Rheumatism published a catalog of criteria to help gaining objective evidence for RA (Arnett, F.C. et al., Arthritis Rheum 31 (1987) 315-324). But so far, not a single test is available which is specific for RA. Several biological and biochemical markers, e.g. C-reactive protein (CRP), erythrocyte sedimentation rate (ESR), antinuclear antibody (ANA) or RF are utilized for the evaluation of RA. However, these markers are non-specific, as they appear in other inflammatory or autoimmune diseases as well. The RF, for instance, is an autoantibody that is present in the serum of approximately 50% of RA patients. Since increased levels of the same autoantibody can also be found in the context of other inflammatory diseases, such as Sjögren syndrome, endokarditis or chronical hepatitis, RF is unsuitable to serve as a diagnostic marker for RA. Rather than being of diagnostic value per se, the above mentioned biochemical and biological markers are useful for assessing disease activity and prognosis as well as in the treatment and management of RA patients (Nakamura, R.M., J Clin Lab Anal 14 (2000) 305-313).

Recently, a diagnostic set of criteria was developed that consists of clinical and biochemical aspects which were claimed to discriminate, at an early state, between self-limiting, persistent non-erosive, and persistent erosive RA (Visser, H. et al., Arthritis Rheum 46 (2002) 357-365). Self-limiting arthritis was characterized by natural remission: there was no arthritis on examination in a patient for a certain period of time. Erosive arthritis was defined based on the presence of erosions on radiographs of the hands and/or feet. In particular, the use of antibodies recognizing cyclic citrullinated peptides appears to be promising and suggests an important role for citrullinated antigens in the early diagnosis and prognosis of erosive RA (Schellekens, G.A. et al., J Clin Invest 101 (1998) 273-281; Vincent, C. et al., J Rheumatol 25 (1998) 838-846). The early recognition of erosive RA allows early intervention with DMARDs, which will lead to earlier disease control and improvement of disease outcome (Symmons, D.P.M. et al., J Rheumatol 25 (1998) 1072-1077; Anderson, J.J. et al., Arthritis Rheum 43 (2000) 22-29). Likewise, early recognition of self-limiting and non-erosive arthritis will prevent unnecessary treatment with potentially toxic therapeutics (Fries, J.F. et al., Arthritis Rheum 36 (1993) 297-306.

### Therapy

The goal of any anti-rheumatic therapy is to relieve pain in order to ease the activities of every day life. So far, complete healing of RA is not possible, but by applying modern therapies the progression of the disease can be slowed down or even stopped. Due to individual differences, each patient requires an individualized therapy and early diagnosis, as mentioned before, is desirable. RA therapy is complex and includes lifelong medicinal treatment as well as physio- and radiotherapy. DMARDs used in RA therapy are basic therapeutics (e.g. Methotrexate, Sulfasalazin, Hydroxychloroquin, Leflunomid, Azathioprin), cortisone, non-steroidal anti-inflammatory drugs (NSAID) or monoclonal antibodies against the pro-inflammatory cytokines TNF-α, IL-1β or their respective receptors (http://rheuma-online.de). These drugs have all in common that they are inhibitors of inflammation by suppressing the immune response. The main disadvantage is their lack of specificity for RA, their adverse effects and their inability to effectively target the causes of RA.

### Autoimmunity

Autoimmunity starts when a specific adaptive immune response is initiated against self antigens (autoantigens) manifested by the development of self-reactive T or B cells. The normal consequence of an adaptive immune response against a foreign antigen is the clearance of the antigen from the body. When an adaptive immune response develops against a self antigen, however, the antigen can in most cases not be completely removed from the body, leading to a sustained immune response. As a consequence, the effector mechanisms of immunity cause chronic inflammatory injury to tissues. The mechanisms of tissue damage are essentially the same in autoimmune disease as those that operate in protective immunity and in hypersensitivity. Even though it is not well understood what triggers autoimmunity, several events which are nowadays believed to contribute to the induction of autoimmune diseases and selection of autoantigenic targets have been summarized most recently (Marrack, P. et al., Nat Med 7 (2001) 899-905).

Autoimmune diseases are controlled by properties of particular genes of each individual and environmental factor. The host's genes affect the susceptibility to autoimmunity at least at three levels. First, some of the genes affect the overall reactivity of the immune system and, thus, can predispose the individual to certain or to several different types of autoimmune diseases. Second, this altered immunoreactivity is funneled to particular autoantigens and tissues by other genes that affect recognition of antigenic peptides by T cells. Third, still other genes act on the ability of target tissues to modulate immune attack for instance by influencing the activity of effector cells of the immune system which are destined to initiate an autoaggressive attack. The latter two sets of genes dictate which antigens will be the targets of autoimmunity and hence which organs will be attacked and what damage will occur.

In addition, signals from the environment influence the development of autoimmunity at the same three levels, by affecting the overall reactivity of the immune system, the antigen-specificity and the state of the potential target tissue. And finally, there is cross-talk between genetic and environmental factors.

### Major histocompatibility complex (MHC)

Population studies, genotyping and modern approaches at the molecular level have unanimously shown that certain genes encoded by the major histocompatibility complex (MHC) confer a significantly higher risk for the development of RA (Stastny, P., Tissue Antigens 4 (1974) 571-579; Wordsworth, P. et al., PNAS 86 (1989) 10049-10053; Wordsworth, P. & Bell, J., Springer Semin Immunopathol 14 (1992) 59-78). In particular, the class II MHC alleles *HLA-DRB1*0101, *0401, *0404* and **0405* in several ethnic groups increase the susceptibility to RA (Reveille, J., Curr Opin Rheumatol 10 (1998) 187-200). E.g. more than 90% of RF-positive RA patients carry one of these susceptibility alleles. HLA class II molecules are MHC-surface proteins that bind antigenic peptides within the cell and present them on the surface of antigen-presenting cells for interaction with the T cell receptors of CD4⁺ helper T lymphocytes, thereby initiating a cellular immune response (Banchereau, J. & Steinman, R.M., Nature 392 (1998) 245-252). The RA-association of particular HLA class II molecules together with the presence of large numbers of activated CD4⁺ T cells in synovial tissue has supported the model of disease induction in which disease-associated HLA-DR molecules present disease-relevant (e.g. synovial) autoantigens and cause stimulation and expansion of synovial T cells, which then drive the inflammatory process (Striebich, C.C. et al., J Immunol 161 (1998) 4428-4436).

MHC class II HLA-DR (short: DR) proteins are heterodimers consisting of monomorphic α- and extremely polymorphic β-chains that bind peptide antigens in a peptide binding groove. This groove generally has four major pockets to accept side chains at relative positions 1, 4, 6 and 9 of the peptide (Stern, L.J. et al., Nature 368 (1994) 215-221). The allelic variations between HLA class II molecules account for the differential ability to bind antigenic peptides. This is the rationale why individuals differing in their HLA alleles have divergent antigenic peptide repertoirs, thereby leading to differences in the quality of immune responses (Messaoudi, I. et al., Science 298 (2002) 1797-1800).

Peptids bound by class II MHC molecules are typically longer and more heterogeneous in size (11-25 amino acids) than the peptides bound by class I MHC molecules (8-10 amino acids). This difference arises because the peptide binding groove of class II proteins is open and while peptides are gripped in the midle, their ends can extend out of the groove in a variable fashion (Jones, E.Y., Curr Opin Immunol 9 (1997) 75-79). As a consequence, class II molecules typically bind sets of overlapping peptides that share a common core sequence, termed "T cell epitope", but have different lengths.

More than a decade ago, it was recognized that the DRβ chains encoded by RA-linked DRB1 alleles, although exhibiting polymorphic differences, all share a stretch of identical or almost identical amino acids at positions 67-74, known as the "shared epitope" (Gregersen, P.K. et al., Arthritis Rheum 30 (1987) 1205-1213). Since immunity to autoantigens has been regarded central to the pathogenesis of RA, it was hypothesized that the shared epitope could impose disease linkage on the respective DR molecules by at least two different mechanisms: first, by selecting the relevant autoantigenic peptides for presentation, and second, by selecting the appropriate autoreactive T cell specificities during ontogeny. The three-dimensional structure of DR molecules has indeed revealed that the shared epitope is located in the center of the α-helix flanking one side of the peptide binding groove (Stern, L.J. et al., Nature 368 (1994) 215-221). Thus, strategically this shared epitope region is positioned in such a way that it can interact with both bound peptide and T cell receptor.

However, one of the unresolved mysteries in rheumatology research is the question what are the key arthritogenic antigens and epitopes in man that trigger the onset and the development of RA. Although autoantibodies of different specificity have been identified in serum and synovial fluid of patients it is often unclear whether the antigens which were released at the time of cartilage degradation, were initiating pathogenicity or whether they are merely a consequence of antigen spreading as a result of inflammation (Corrigall, V.M. & Panayi G.S., Crit Rev Immunol 22 (2002) 281-293). Furthermore it is difficult to define pathogenic mechanisms in which the antigen is present throughout the body, including the joint, but the pathology is targeted solely or predominately to the joint.

### Autoantigens

The large number of possible autoantigens in RA is derived from studies using sera or, less frequently, T cells from patients with established chronic RA. One of the most convincing joint-specific antigen that has been proposed in the context of DR molecules, is type II collagen (CII), the predominant protein in articular cartilage. Autoantibodies against CII were found in elevated concentrations in the serum and joints of RA patients although it is not yet clear whether anti-CII antibodies are pathogenic in RA (Banerjee, S. et al., Clin Exp Rheumatol 6 (373-380). Snowden and coworkers have shown that peripheral blood T cells from RA patients proliferated to CII, most pronounced in those patients with anti-CII antibodies. However, the response was seen only in 50% of patients (Snowden, N. et al., Rheumatology 40 (1997) 1210-1218). In a mouse model immunization with CII was shown to induce arthritis in mice expressing the class II MHC alleles *DRB1*0401* and *0101 (Rosloniec, E.F. et al., J Exp Med 185 (1997) 1113-1122; Rosloniec, E.F. et al., J immunol 160 (1998) 2573-2578). The immunodominant epitope in both *0401 and *0101 transgenic mice was localized to peptides within residues 261-273 of human CII (Fugger, L. et al., Eur J Immunol 26 (1996) 928-933). The same epitope of CII was capable of stimulating a T cell response in RA patients, particularly in the early stages of disease. Synovial fluid T cells were especially responsive (Kim, H.Y. et al., Arthritis Rheum 42 (1999) 2085-2093).

Although other cartilage proteins have been proposed as RA candidate antigens, DR4-binding epitopes have been defined only for human cartilage glycoprotein 39 (HCgp39). This protein is secreted by synovial cells and articular chondrocytes and its expression is upregulated in plasma and joints during inflammation (Vos, K. et al., Ann Rheum Dis 59 (2000) 544-548). Similar to CII, HCgp39 treatment induces arthritis in mice. In addition a HCgp39 response of peripheral blood T cells from RA patients was detected (Verheijden, G.F. et al., Arthritis Rheum 40 (1997) 1115-1125). The predominant epitope recognized by T cells in DR4 patients was defined between residues 263-275 and identical to the immunodominant epitope found in *DRB1***0401*-transgenic mice after immunization with native HCgp39 (Cope, A.P. et al., Arthritis Rheum 42 (1999) 1497-1507). Although not disease specific, responses to this peptide did correlate with disease activity in RA patients (Baeten, D. et al., Arthritis Rheum 43 (2000) 1233-1243). Antibodies to HCgp39, however, have also been detected in the sera of patients with inflammatory diseases, such as inflammatory bowel disease and systemic lupus erythematosus (SLE), albeit at a lower level than in RA.

In an attempt to track antigen-specific T cells in RA, soluble peptide-DR4 tetrameric complexes were used to detect synovial CD4⁺ T cells reactive with CII or HCgp39 in DR4⁺ patients (Kotzin, B.L. et al., PNAS 97 (2000) 291-296). The CII-DR4 complex bound in a specific manner to CII peptide-reactive T cell hybridomas, but did not stain a detectable fraction of synovial CD4⁺ cells. Almost similar results were obtained with the HCgp39-DR4 complex suggesting that the major oligoclonal CD4⁺ T cell expansions present in RA joints are not specific for the dominant CII and HCgp39 determinants described above.

In summary, despite some strong indications for a CII and HCgp39 association with RA, the evidence that they are important antigens in RA is scanty. A direct proof that peptides of CII or HCgp39 are presented in a class II MHC-restricted manner by antigen-presenting cells with subsequent stimulation and activation of synovial CD4⁺ T cells is still lacking. Furthermore a major problem of animal models is their unknown relevance to RA as CII-induced arthritis by immunizing rats or mice differs in many respects from RA.

### Naturally processed MHC class II-associated peptides

An alternative strategy to the identification of RA-specific autoantibodies and T cells relies on the sequence analysis of naturally processed peptide antigens bound to MHC class II molecules. With the help of monoclonal antibodies, class II MHC molecules conferring susceptibility to RA can be purified from cognate cells. RA-associated peptide antigens can be acid-eluted from purified HLA class II molecules. The mixture of small peptides can be separated by HPLC and the peptide sequence be determined by Edman sequencing or mass spectrometry. Due to limitations with peptide purification and sequencing techniques, peptide sequences were, as yet, only obtained from MHC molecules that have been isolated from cultured B cell lines or large amounts of tissue, and the analysis was restricted to a few abundant peptides (Kropshofer et al., J.Exp.Med. 175 (1992) 1799-1803; Chicz, R.M. et al., J Exp Med 178 (1993) 27-47). As a result of the development of high-resolution microcapillary HPLC columns and more sensitive mass spectrometers, MHC-bound peptides can be analyzed more efficiently (Dongre, A.R. et al., Eur J Immunol 31 (2001) 1485-1494; Engelhard, V.H. et al., Mol Immunol 39 (2002) 127-137).

In the present invention a modified peptide isolation and sequencing technique was used to investigate the peptide antigen repertoire of HLA-DR4 molecules derived from autologous dendritic cells (DCs) which were pulsed with serum or synovial fluid derived from RA patients. The main advantage of this innovative approach is the usage of human DCs that are professionals in RA-relevant antigen processing and presentation, instead of using transgenic animal models or artificial B cell lines.

DCs are enriched in rheumatoid synovial fluid and tissue and are derived from circulating immature precursors (Thomas, R. et al., J Immunol 152 (1994) 2613-2623). They are the most potent antigen-presenting cells which express high levels of MHC molecules together with a variety of accessory molecules (Mellman, I. et al., Trends Cell Biol 8 (1998) 231-237). In a most recent study, it was shown that *ex vivo* differentiated human DCs and macrophages that are phenotypically similar to antigen-presenting cells from RA synovial joints, were capable of generating and presenting immunodominant epitopes from CII and HCgp39 (Tsark, E.C. et al., J Immunol 169 (2002) 6625-6633). DC have the capacity to prime CD4⁺ helper T cells and to effectively activate cytotoxic CD8⁺ T cells (Ridge, T. et al., Nature 393 (1998) 474-478). Thus, peptides bound to MHC class II molecules and presented by DCs play a superior role in the pathogenesis of diseases involving T cell-driven immune responses.

Therefore, the problem posed by the lack of knowledge of MHC class II restricted antigenic peptides for RA is solved by providing novel naturally-processed MHC class II associated RA antigenic peptides and the polypeptides they are derived from as markers for RA.

The present invention provides novel naturally-processed antigenic peptides which are candidate RA markers in erosive and non-erosive RA. These antigenic peptides are presented by human MHC class II HLA-DR molecules derived from dendritic cells which were pulsed with serum or synovial fluid derived from patients with established erosive or non-erosive RA. The invention provides a MHC class II antigenic peptide comprising (a) at least the amino acid sequence of the peptide binding motif of SEQ ID NO. 49, or (b) at least the amino acid sequence of the peptide binding motif of SEQ ID NO. 49 with additional N-and C-terminal flanking sequences of a corresponding sequence selected from the group consisting of SEQ ID NOs. 1 to 3, and originate from interferon-γ-inducible lysosomal thiol reductase. The present invention also provides these antigenic peptides and the proteins they are derived from as markers for erosive and/or non-erosive RA. Moreover, these antigenic peptides linked to MHC class II molecules, as well as antibodies reactive with said antigenic peptides, nucleic acids encoding said antigenic peptides, and nucleic acid constructs, host cells and methods for expressing said antigenic peptides are provided. Further methods are provided for isolating and identifying RA antigenic peptides.

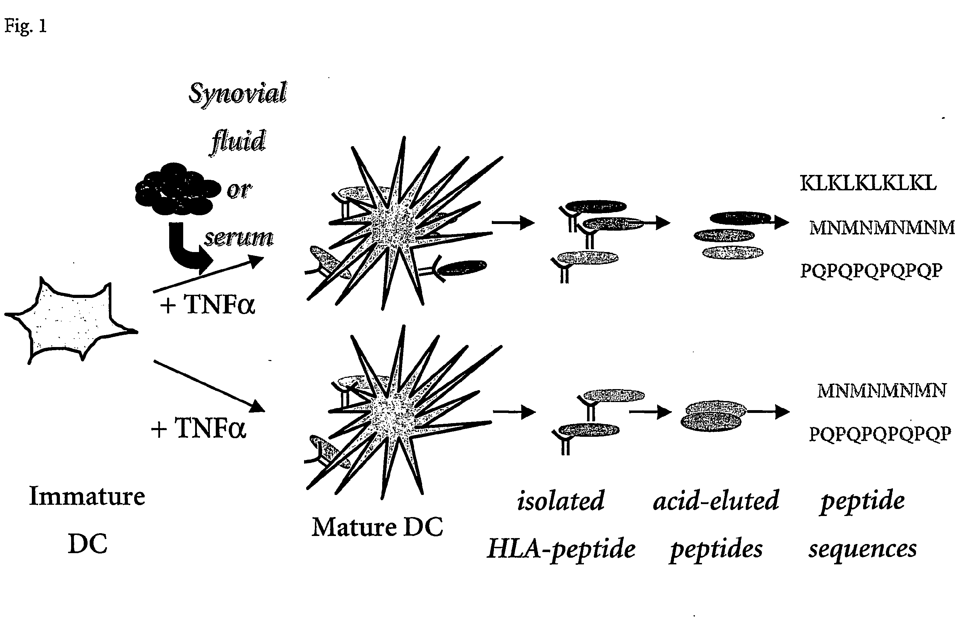

**Fig. 1****: Diagram of Dendritic cell (DC)-mediated analysis of tissue samples:** Dendritic cells (DCs), the most specialized antigen-presenting cells (APCs), are brought in contact with an antigen source (e.g. synovial fluid) under optimal conditions for antigen uptake and antigen processing. As a control, DCs are cultured under the same conditions in the absence of synovial fluid antigens. After maturation of DCs, antigen-loaded MHC class II molecules are purified and the respective MHC class II-associated antigenic peptides are isolated and identified.

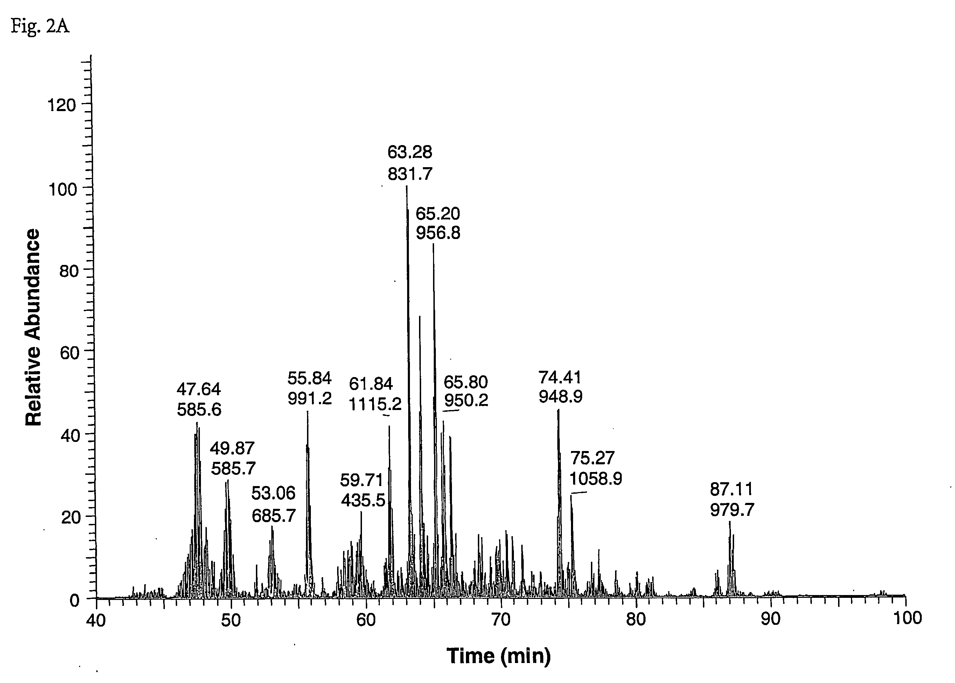

**Fig. 2A****: ION-TRAP MS Base Peak Chromatogram of MHC class II-associated antigenic** peptides that were isolated from dendritic cells pulsed with the serum of a RA patient. The peptides were eluted directly from a RP-C18-HPLC column into the ion trap mass spectrometer for immediate MS/MS identification. The numbers indicate the retention times (upper value) and the molecular masses (lower value) of the most prominent peptide peaks in the mixture at the respective time.

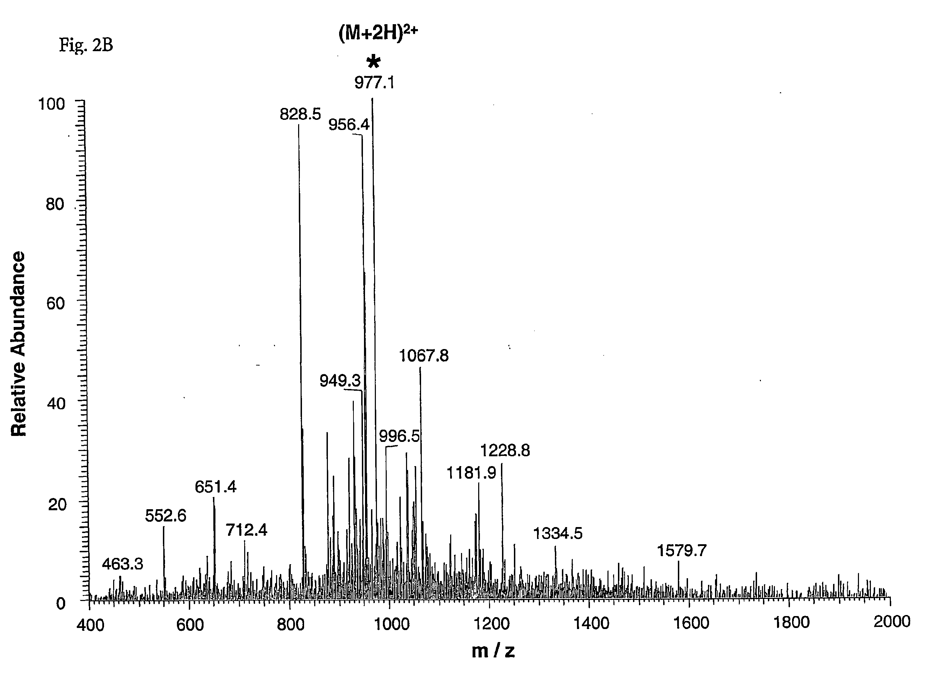

**Fig. 2B****: ION-TRAP MS spectrum** of antigenic peptides at a retention time of 65.4 min. The marked peak was further fragmented and corresponded to a doubly charged peptide ion from the inter-alpha-trypsin inhibitor ITIH4 (cf. table 3).

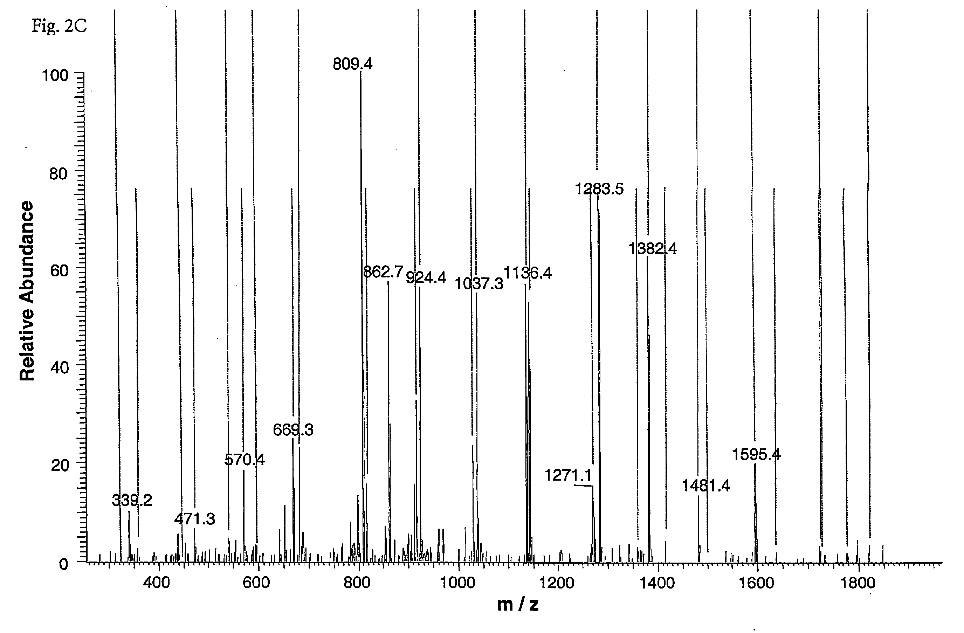

**Fig. 2C****: ION-TRAP MS/MS spectrum** of the doubly charged peptide ion at m/z 977.1. The fragmentation masses, together with the mass of the parent ion, were searched against a non-redundant human database by using the SEQUEST algorithm. The retrieved sequence MPKNVVFVIDKSGSMSGR (one-letter-code) corresponded to the dominant epitope ITIH4 (271-288) of the inter-alpha-trypsin inhibitor. The positions of the assigned series of N-terminal B-ions and C-terminal Y-ions are marked.

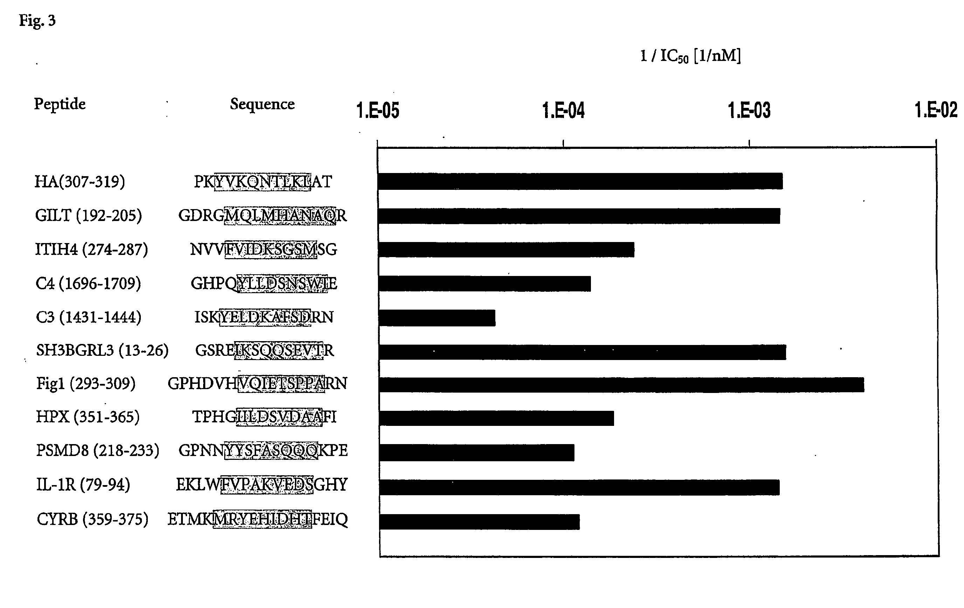

Fig. 3: Summary of the differential binding capacity of the tested candidate RA antigens in the context of binding to the *HLA-DRB1*0401* allele. The putative *HLA-DRB1*0401* binding motif is boxed in grey and. As a measure for affinity, the peptide concentration was determined that was needed to reduce binding of a fixed amount of biotinylated HA(307-319) peptide by 50% (IC₅₀) through competition. The reciprocal (1/ IC₅₀) directly correlates with peptide affinity. As a reporter biotinylated HA(307-319) peptide from influenza hemagglutinin (Rothbard, J.B. et al., Cell 52 (1988) 515-523) was included in the study.

The antigenic peptides of the invention are peptides, which are associated with and presented by MHC molecules and thereby can have the potential to activate or tolerize T cells. Antigenic peptides presented by MHC class II molecules are therefore MHC class II associated or MHC class II antigenic peptides, whereas antigenic peptides presented by MHC class I molecules are MHC class I associated or MHC class I antigenic peptides.

Peptides which are derived from proteins that are encoded in the genome of the body or an APC are denoted as "self-peptides". The main function of self-peptides presented by DCs in the peripheral lymphoid organs is thought to be the induction of T cell tolerance to self-proteins. Tolerance is the failure to respond to an antigen; when that antigen is borne by self tissues, tolerance is called self tolerance.

Antigens which are derived from an individual's own body are called "self antigens" or "autoantigens". An adaptive immune response directed against self antigens is called an autoimmune response. Likewise, adaptive immunity specific for self antigens is called autoimmunity. Autoreactivity describes immune responses directed against self antigens. RA is probably due to an autoimmune response that is based on the involvement of autoreactive T cells and/ or autoreactive antibodies. Immunogenic peptide includes, but is not limited to, an antigenic peptide capable of causing or stimulating a cellular or humoral immune response. Such peptides may also be reactive with antibodies.

Peptides derived from proteins encoded in the genome of bacteria, viruses or other foreign invaders and which differ from self-proteins are called "foreign antigenic" or "foreign" peptides. They are able to elicit a T cell response against foreign proteins they are derived from.

RA antigenic peptides are self-peptides that function as self antigens and as a consequence of the disease erroneously trigger autoreactivity against self tissues.

Preferably, the MHC class II antigenic peptide has a length of less than 26 amino acids, more preferably a length of 11 to 25 amino acids. Even more preferred is the antigenic peptide of the invention with a length of 11 to 19 amino acids. Most preferred is the antigenic peptide of the invention consisting of the peptide binding motif comprising the four anchor amino acids.

The present invention also provides a MHC class II antigenic peptide comprising (a) at least the amino acid sequence of the peptide binding motif of SEQ ID NO. 49, or (b) at least the amino acid sequence of the peptide binding motif of SEQ ID NO. 49 with additional N-and C-terminal flanking sequences of a corresponding sequence selected from the group consisting of SEQ I D NOs. 1 to 3.

MHC class II associated novel antigenic peptides originate from interferon-γ-inducible lysosomal thiol reductase (SEQ ID NOs. 1 to 3), integrin beta-2 (SEQ ID NOs. 58 and 59), phosphatitylinositol-4,5-bisphosphate 3-kinase (SEQ ID NO: 60), urokinase-type plasminogen activator (SEQ ID NO: 61), immunoglobulin heavy chain V-III region (V_{H}26) (SEQ ID NO: 62), DJ-1 protein (SEQ ID NO: 63), apolipoprotein B-100 (SEQ ID NOs. 4 and 5), 26S proteasome non-ATPase regulatory subunit 8 (SEQ ID NOs. 64 to 67), interleukin-1 receptor (SEQ ID NO: 68), fibromodulin (SEQ ID NOs.: 69 and 70), GM-CSF/IL-3/IL-5 receptor (SEQ ID NOs. 71 and 72), sorting nexin 3 (SEQ ID NO: 73), inter-α-trypsin inhibitor heavy chain H4 (SEQ ID NOs. 6 to 12), complement C4 (SEQ ID NOs. 13 to 18), complement C3 (α-chain) (SEQ ID NOs. 19 to 23, 74 and 75), complement C3 (β-chain) (SEQ ID NOs. 76 and 77), SH3 domain-binding glutamic acid-rich-like protein 3 (SEQ ID NOs. 24 to 27), interleukin-4-induced protein 1 (SEQ ID NOs. 28 to 30), hemopexin (SEQ ID NOs. 31 to 35 and 78), Hsc70-interacting protein (SEQ ID NOs. 36 to 39), invariant chain (Ii) (SEQ ID NOs. 79 to 83), retinoic acid receptor responder protein 2 (SEQ ID NOs. 84 to 86), fibronectin (SEQ ID NOs. 87 to 91), cathepsin B (SEQ ID NO: 92), tripeptidyl-peptidase II (SEQ ID NOs. 93 and 94), legumain (SEQ ID NO: 95), platelet activating factor receptor (SEQ ID NO: 96), poly-alpha-2.8-sialyltransferase (SEQ ID NO: 97), and ras-leated protein Rab-11B (SEQ ID NOs. 98 to 102).

The single peptide binding groove of MHC class II molecules is about 25 Å long, but in contrast to MHC class I molecules, both sides are open (Stern LJ et al., Nature 1994; 368, 215-221). Thus, naturally processed antigenic peptides eluted from human MHC class II molecules have a minimal length of about 11 residues and attain a maximal length of about 25 residues (Chicz RM et al., J Exp Med 1993; 178, 27-47).

The stability of the MHC-peptide interaction is determined by more than a dozen hydrogen bonds involving the peptide backbone and the complementarity between specificity pockets of the binding groove and appropriately located amino acid side-chains of the peptide. The amino acids of the peptide fitting into the respective pockets were named "anchor" residues. With regard to most HLA-DR alleles, these anchors are located at relative positions P1, P4, P6 and P9. The combination of amino acids at these 4 anchor positions conferring high-stability binding to the respective HLA-DR allelic product and vary from allele to allele. The peptide binding motif is defined herein as the sequence of nine amino acids comprising the four anchor amino acids. The peptide binding motif of the MHC class II antigenic peptide is depicted in SEQ ID NO. 49 for the peptides derived from interferon-γ-inducible lysosomal thiol reductase (SEQ ID NOs. 1 to 3), in SEQ ID NO. 103 for the peptides derived from integrin beta-2 (SEQ ID NOs. 58 and 59), in SEQ ID NO. 104 for the peptides derived from phosphatitylinositol-4,5-bisphosphate 3-kinase (SEQ ID NO: 60), in SEQ ID NO. 105 for the peptides derived from urokinase-type plasminogen activator (SEQ ID NO: 61), in SEQ ID NO. 106 for the peptides derived from immunoglobulin heavy chain V-III region (V_{H}26) (SEQ ID NO: 62), in SEQ ID NO. 107 for the peptides derived from DJ-1 protein (SEQ ID NO: 63), in SEQ ID NO. 50 for the peptides derived from apolipoprotein B-100 (SEQ ID NOs. 4 and 5), in SEQ ID NO. 108 for the peptides derived from 26S proteasome non-ATPase regulatory subunit 8 (SEQ ID NOs. 64 to 67), in SEQ ID NO. 109 for the peptides derived from interleukin-1 receptor (SEQ ID NO: 68), in SEQ ID NO. 110 for the peptides derived from fibromodulin (SEQ ID NOs.: 69 and 70), in SEQ ID NO. 111 for the peptides derived from GM-CSF/IL-3/IL-5 receptor (SEQ ID NOs. 71 and 72), in SEQ ID NO. 112 for the peptides derived from sorting nexin 3 (SEQ ID NO: 73), in SEQ ID NO. 51 for the peptides derived from inter-α-trypsin inhibitor heavy chain H4 (SEQ ID NOs. 6 to 12), in SEQ ID NO. 52 for the peptides derived from complement C4 (SEQ ID NOs. 13 to 18), in SEQ ID NO. 53 for the peptides derived from complement C3 (α-chain) (SEQ ID NOs. 19 to 23, 74 and 75), in SEQ ID NO. 113 for the peptides derived from complement C3 (β-chain) (SEQ ID NOs. 76 and 77), in SEQ ID NO. 54 for the peptides derived from SH3 domain-binding glutamic acid-rich-like protein 3 (SEQ ID NOs. 24 to 27), in SEQ ID NO. 55 for the peptides derived from interleukin-4-induced protein 1 (SEQ ID NOs. 28 to 30), in SEQ ID NO. 56 for the peptides derived from hemopexin (SEQ ID NOs. 31 to 35 and 78), in SEQ ID NO. 57 for the peptides derived from Hsc70-interacting protein (SEQ ID NOs. 36 to 39), in SEQ ID NO. 114 for the peptides derived from invariant chain (Ii) (SEQ ID NOs. 79 to 83), in SEQ ID NO. 115 for the peptides derived from retinoic acid receptor responder protein 2 (SEQ ID NOs. 84 to 86), in SEQ ID NO. 116 for the peptides derived from fibronectin (SEQ ID NOs. 87 to 91), in SEQ ID NO. 117 for the peptides derived from cathepsin B (SEQ ID NO: 92), in SEQ ID NO. 118 for the peptides derived from tripeptidyl-peptidase II (SEQ ID NOs.93 and 94), in SEQ ID NO. 119 for the peptides derived from legumain (SEQ ID NO: 95), in SEQ ID NO. 120 for the peptides derived from platelet activating factor receptor (SEQ ID NO: 96), in SEQ ID NO. 121 for the peptides derived from poly-alpha-2.8-sialyltransferase (SEQ ID NO: 97) and in SEQ ID NO. 122 for the peptides derived from Ras-related protein Rab-11B (SEQ ID NO: 98 to 102).

The peptide binding motif may also comprise at least one, at least two, at least three, at least four or at least five modifications of the amino acid sequence while still attaining the binding capacity of the non-modified peptide binding motif. Preferably, the modified peptide binding motif comprises at least three of the four anchor amino acids of the non-modified peptide binding motif. The amino acid modification may be a conservative amino acid substitution as described below.

Additional binding energy is provided by hydrogen bonds involving residues in front of the P1 anchor and behind the P9 anchor. In agreement with that, in most naturally processed peptides the nonameric core-region (P1-P9) is N- and C-terminally flanked by 3-4 residues. Hence, the majority of peptides are 15-17-mers. Longer peptides protrude from the groove, thereby allowing access of exopeptidases which are trimming both ends.

Therefore, the MHC class II antigenic peptide of the invention comprising (a) at least the amino acid sequence of the peptide binding motif of SEQ ID NO. 49, or (b) at least the amino acid sequence of the peptide binding motif of SEQ ID NO. 49 with additional N-and C-terminal flanking sequences of a corresponding sequence selected from the group consisting of SEQ ID NOs. 1 to 3, preferably comprises additional N- and C- terminal flanking amino acid residues providing additional binding energy.

Preferably, the MHC class II antigenic peptide of the present invention has a binding capacity to the corresponding MHC class II molecule of between one tenth and ten-fold the IC₅₀ of a corresponding peptide selected from the group consisting of SEQ ID NOs. 1 to 3. The binding capacity of a peptide is measured by determining the concentration necessary to reduce binding of a labelled reporter peptide by 50%. This value is called IC₅₀. A MHC class II antigenic peptide of the invention maintains its binding capacity to the relevant HLA class II molecules as long as it attains IC₅₀ values between one tenth and 10-fold the IC₅₀ of the established reference peptides.

Since peptide trimming occurs in an individual fashion both before and after binding into the peptide binding groove, the occurrence of several truncation variants sharing a common nonameric core region is a common feature of MHC class II-bound peptides. Importantly, it was shown that C- or N-terminal truncation variants of the same epitope can trigger divergent T cell responses (Arnold et al., (2002) J. Immunol. 169, 739-749).

Several parameters can be envisaged that have an influence on the relative abundance of truncation variants of a particular epitope, e.g. the abundance and integrity of the antigen of relevance, antigen-associated proteins, the abundance of proteases, the type of proteases available and the supply with competitive antigens and/or peptides. Since the antigen supply is a major characteristic that may correlate with the origin of a sample, the ratio of particular truncation variants of an epitope can be of diagnostic value.

A peptide of the invention is a peptide which either has no naturally-occurring counterpart (e.g., such as an mutated peptide antigen), or has been isolated, i.e., separated or purified from components which naturally accompany it, e.g., in tissues such as pancreas, liver, spleen, ovary, testis, muscle, joint tissue, neural tissue, gastrointestinal tissue, or body fluids such as blood, serum, synovial fluid or urine. Typically, the peptide is considered "isolated" when a preparation comprising a peptide of the invention consists to at least 70%, by dry weight of said peptide and to less than 30% of the proteins and naturally-occurring organic molecules with which it is naturally associated. Preferably, a preparation of a peptide of the invention consists of at least 80%, more preferably at least 90%, and most preferably at least 99%, by dry weight, the peptide of the invention. Since a peptide that is chemically synthesized is, by its nature, separated from the components that naturally accompany it, the synthetic peptide is "isolated".

The invention further provides analogs of the antigenic peptide of the invention. The term analog includes any peptide which displays the functional aspects of these antigenic peptides comprising the binding capacity IC₅₀ and the recognition by antibodies and cells of the immune system. Analogs exhibit essentially the same IC₅₀ as the corresponding reference peptide. The term analog also includes conservative substitutions or chemical derivatives of the peptides.

The term "analog" includes any polypeptide having an amino acid residue sequence substantially identical to the sequences described herein in which one or more residues have been conservatively substituted with a functionally similar residue and which displays the functional aspects of the peptides as described herein. Examples of conservative substitutions include the substitution of one non-polar (hydrophobic) residue such as phenylalanine, tyrosine, isoleucine, valine, leucine or methionine for another, the substitution of one polar (hydrophilic) residue for another such as between arginine and lysine, between glutamine and asparagine, between threonine and serine, the substitution of one basic residue such as lysine, arginine or histidine for another, or the substitution of one acidic residue, such as aspartic acid or glutamic acid for another.

The phrase "conservative substitution" also includes the use of a chemically derivatized amino acid in place of a non-derivatized amino acid. "Chemical derivative" refers to a subject polypeptide having one or more amino acids chemically derivatized by reaction of a functional side group. Examples of such derivatized molecules include for example, those molecules in which free amino groups have been derivatized to form amine hydrochlorides, p-toluene sulfonyl groups, carbobenzoxy groups, t-butyloxycarbonyl groups, chloroacetyl groups, acetyl groups or formyl groups. Free carboxyl groups may be derivatized to form salts, methyl and ethyl esters or other types of esters or hydrazides. Free hydroxyl groups may be derivatized to form O-acyl or O-alkyl derivatives. The imidazole nitrogen of histidine may be derivatized to form N-im-benzylhistidine. Also included as chemical derivatives are those proteins or peptides, which contain one or more naturally-occurring amino acid derivative of the twenty standard amino acids. For examples: 4-hydroxyproline may be substituted for proline; 5-hydroxylysine may be substituted for lysine; 3-methylhistidine may be substituted for histidine; homoserine may be substituted for serine; and ornithine or citrulline may be substituted for lysine.

The MHC class II antigenic peptides of the invention and the proteins they are derived from can be used as markers in diagnosis of RA and in therapy as anti-RA vaccines. The term marker as used herein refers to a biomolecule, preferably a peptide or a polypeptide, which is expressed in a group of patients with a diagnosed disease, e.g. RA, and attains an abundance that is significantly increased or decreased as compared to a control group.

The marker of the present invention may be used as a prognostic marker to predict the susceptibility to a disease, e.g., to predict the susceptibility to RA, as a diagnostic marker for the diagnosis of a disease, e.g. for the diagnosis of RA, as a differential diagnostic marker to differentiate between different forms of a disease, e.g., to differentiate between different forms of RA, as a prognostic marker for the prediction of the outcome of a disease, e.g., for the prognosis of RA, and as a response marker to determine the efficacy of a therapeutic regime, e.g., as a response marker in the treatment of RA.

In a further embodiment, the MHC class II antigenic peptide comprising (a) at least the amino acid sequence of the peptide binding motif of SEQ ID NO. 49, or (b) at least the amino acid sequence of the peptide binding motif of SEQ ID NO. 49 with additional N-and C-terminal flanking sequences of a corresponding sequence selected from the group consisting of SEQ ID NOs. 1 to 3 is used as a marker for non-erosive RA.

In a further embodiment, the MHC class II antigenic peptides of the invention as described above are provided linked to a MHC class II molecule.

Multimers (e.g., dimers, trimers, tetramers, pentamers, hexamers or oligomers) of a class II MHC molecule containing a covalently or non-covalently bound peptide according to the present invention, if conjugated with a detectable label (e.g., a fluorescent moiety, a radionuclide, or an enzyme that catalyzes a reaction resulting in a product that absorbs or emits light of a defined wavelength) can be used to quantify T cells from a subject (e.g. a human patient) bearing cell surface receptors that are specific for, and therefore will bind, such complexes. Relatively high numbers of such T cells are likely to be diagnostic of disease or an indication that the T cells are involved in immunity to the disease. In addition, continuous monitoring of the relative numbers of multimer-binding T cells can be useful in establishing the course of a disease or the efficacy of therapy. Such assays have been developed using tetramers of class I MHC molecules containing an HIV-1-derived or an influenza virus-15 derived peptide (Altman et al. (1996), Science 274:94-96; Ogg et al. (1998), Science 279:2103- 21061), and corresponding class II MHC multimers would be expected to be similarly useful. Such complexes could be produced by chemical cross-linking of purified class II MHC molecules assembled in the presence of a peptide of interest or by modification of already established recombinant techniques for the production of class II MHC molecules containing a single defined peptide (Kazono et al. (1994), Nature 369:151-154; Gauthier et al. (1998), Proc. Natl. Acad. Sci. U.S.A. 95:11828-118331). The class II MHC molecule monomers of such multimers can be native molecules composed of full-length alpha and beta chains. Alternatively, they can be molecules containing either the extracellular domains of the alpha and beta chains or the alpha and beta chain domains that form the "walls" and "floor" of the peptide-binding cleft.

The invention also relates to an antibody, fragments or derivatives thereof, directed to and reactive with the above-described MHC class II antigenic peptides. The general methodology for producing antibodies is well known and is disclosed per example in Kohler and Milstein, 1975, Nature 256,494 or in J. G. R. Hurrel, Monoclonal Hybridoma Antibodies: Techniques and Applications, CRC Press Inc., Boco Raron, FL (1982). The antibodies can be polyclonal or, preferably, monoclonal, or antibody fragments like be F (ab') 2, Fab, Fv or scFv. The antibodies of the present invention may also be humanized (Merluzzi S. et al., (2000), Adv. Clin. Path., 4(2): 77-85) or human antibodies (Aujame L. et al., Hum. Antibodies, (1997), 8(4): 155-168).

The present invention also provides a nucleic acid molecule encoding a MHC class II antigenic peptide of the invention comprising (a) at least the amino acid sequence of the peptide binding motif of SEQ ID NO. 49, or (b) at least the amino acid sequence of the peptide binding motif of SEQ ID NO. 49, with additional N- and C-terminal flanking sequences of a corresponding sequence selected from the group consisting of SEQ ID NOs. 1 to 3. Preferably, the nucleic acid molecule is a DNA molecule.

Furthermore, a nucleic acid molecule is provided encoding a MHC class II antigenic peptide of the invention linked to a MHC class II molecule.

This invention also provides a recombinant nucleic acid construct comprising the nucleic acid molecules as described above, operably linked to an expression vector. Expression vectors suitable for use in the present invention comprise at least one expression control element operably linked to the nucleic acid sequence encoding the antigenic peptide or the antigenic peptide linked to a MHC class II molecule. The recombinant expression construct may be a DNA construct.

The expression control elements are inserted in the vector to control and regulate the expression of the nucleic acid sequence encoding the antigenic peptide of the invention. Examples of expression control elements include, but are not limited to, lac system, operator and promoter regions of phage lambda, yeast promoters and promoters derived from polyoma, adenovirus, retrovirus or SV40. Additional preferred or required operational elements include, but are not limited to, leader sequence, termination codons, polyadenylation signals and any other sequences necessary or preferred for the appropriate transcription and subsequent translation of the nucleic acid sequence in the host system. It will be understood by one skilled in the art that the correct combination of required or preferred expression control elements will depend on the host system chosen. It will further be understood that the expression vector should contain additional elements necessary for the transfer and subsequent replication of the expression vector containing the nucleic acid sequence in the host system. Examples of such elements include, but are not limited to, origins of replication and selectable markers. It will further be understood by one skilled in the art that such vectors are easily constructed using conventional methods ("DNA Isolation and Sequencing", Bruce A. Roe, Judy S. Crabtree and Akbar S. Khan, Published by John Wiley & Sons, 1996) or are commercially available.

Another aspect of this invention relates to a host organism or a host cell into which a recombinant nucleic acid construct comprising the nucleic acid molecules as described above, operably linked to an expression vector, has been inserted. The host cells transformed with the nucleic acid constructs encompassed by this invention include eukaryotes, such as animal, plant, insect and yeast cells and prokaryotes, such as E. coli.

The means by which the nucleic acid construct carrying the nucleic acid sequence may be introduced into the cell include, but are not limited to, microinjection, electroporation, transduction, or transfection using DEAE-dextran, lipofection, calcium phosphate or other procedures known to one skilled in the art (Sambrook et al. (1989) in "Molecular Cloning. A Laboratory Manual", Cold Spring Harbor Press, Plainview, New York).

In a preferred embodiment, eukaryotic expression vectors that function in eukaryotic cells are used. Examples of such vectors include, but are not limited to, retroviral vectors, vaccinia virus vectors, adenovirus vectors, herpes virus vector, fowl pox virus vector, plasmids, or the baculovirus transfer vectors. Preferred eukaryotic cell lines include, but are not limited to, COS cells, CHO cells, HeLa cells, NIH/3T3 cells, 293 cells (ATCC# Cry:15731); T2 cells, dendritic cells, monocytes or Epstein-15 Barr Virus transformed B cells.

An antigenic peptide of the invention can be obtained, for example, by extraction from a natural source (e.g., elution from MHC II molecules); by expression of a recombinant nucleic acid encoding the peptide; or by chemical synthesis. A peptide that is produced in a cellular system different from the source from which it naturally originates is "isolated," because it will be separated from components which naturally accompany it. The recombinant peptide expressed by a host organism can be obtained as a crude lysate or can be purified by standard protein purification procedures known in the art which may include differential precipitation, size exclusion chromatography, ion-exchange chromatography, isoelectric focusing, gel electrophoresis, affinity, and immunoaffinity chromatography and the like. The extent of isolation or purity can be measured by any appropriate method, e.g. mass spectrometry or HPLC analysis. The peptides maybe prepared synthetically by procedures described in Merrifield, (1986) Science 232: 341-347, and Barany andMerrifield, The Peptides, Gross and Meienhofer, eds (N. Y., Academic Press), pp. 1-284 (1979). The synthesis can be carried out in solution or in solid phase or with an automatized synthesizer (Stewart and Young, Solid Phase Peptide Synthesis, 2nd ed., Rockford Ill., Pierce Chemical Co. (1984)).

Therefore, the present invention further provides a method for producing a MHC class II antigenic peptide comprising (a) at least the amino acid sequence of the peptide binding motif of SEQ ID NO. 49, or (b) at least the amino acid sequence of the peptide binding motif of SEQ ID NO. 49 with additional N-and C-terminal flanking sequences of a corresponding sequence selected from the group consisting of SEQ ID NOs. 1 to 3, comprising the steps of culturing the host cell containing a recombinant nucleic acid construct as described above under conditions allowing expression of said peptide and recovering the peptide from the cells or the culture medium.

A method is herewith disclosed for isolating and identifying MHC class II associated RA antigenic peptides in femtomolar amounts, which method comprises (a) providing immature dendritic cells in a number comprising 0.1 to 5 µg MHC class II molecules; (b) contacting the cells of (a) with serum or synovial fluid and inducing maturation of dendritic cells by adding TNFalpha; (c) isolating class II MHC molecule-antigenic peptide complexes from the cells with methods comprising solubilization of the cells and sequestration of the complexes of MHC class II molecules with antigenic peptides by immunoprecipitation or immunoaffinity chromatography; (d) washing the sequestered complexes of MHC class II molecules with antigenic peptides with water in an ultrafiltration tube; (e) eluting the associated antigenic peptides from the MHC class II molecules at 37°C with diluted trifluoro acetic acid, and (f) separating, detecting and identifying the isolated peptides by liquid chromatography and mass spectrometry. Furthermore, in step (f) of the method, the liquid chromatography comprises a first linear elution step from the reversed-phase material with a volume sufficient to elute the majority of contaminants prior to peptide elution. Moreover, the method may further comprise (g) analyzing the identified peptides by methods comprising a database and a software developed to perform comparative data analysis across multiple datasets.

The amount of tissue or bodily fluid necessary to obtain e.g. 100 ng MHC class II molecules depends on the number of cells that do express MHC class II and on the expression rate of MHC class II molecules: e.g. 100 ng of MHC class II are equivalent to about 2 x 10⁵ mature DCs or 5 to 10 x 10⁶ peripheral blood monocytes or about 5 x 10⁷ peripheral blood mononuclear cells which can be obtained from about 50 ml of blood.

For the purification of class II MHC molecule-antigenic peptide complexes from cells or tissue, the membranes of the cells or tissue have to be solubilized. Cell lysis may be carried out with methods known in the art, e.g. freeze-and-thaw cycles and the use of detergents, and combinations thereof. Preferred lysis methods are solubilization using detergents, preferably TX-100, NP40, n-octylglucoside, Zwittergent, Lubrol, CHAPS, most preferably TX-100 or Zwittergent 3-12. Cell debris and nuclei have to be removed from cell lysates containing the solubilized receptor-peptide complexes by centrifugation. Therefore, the complexes of class II MHC molecules with antigenic peptides are isolated from the cells with methods comprising solubilization with a detergent.

Furthermore, the MHC class II molecule-peptide complexes are purified from cell lysates by methods comprising immunoprecipitation or immunoaffinity chromatography. For the immunoprecipitation or immunoaffinity chromatography, antibodies specific for MHC class II molecules and suitable for these methods are used. The specific antibodies are preferably monoclonal antibodies, and are covalently or non-covalently e.g. via Protein A, coupled to beads, e.g. sepharose or agarose beads. A selection of the broad panel of anti-HLA antibodies used in the prior art comprises: anti-HLA-DR antibodies: L243, TU36, DA6.147, preferably L243; anti-HLA-DQ antibodies: SPVL3, TU22, TU169, preferably TU22 and TU169; anti-HLA-DP antibody B7/21 and anti-HLA-A,B,C antibodies W6/32 and B9.12.

Monoclonal antibodies specific for different MHC class II molecules may be commercially obtained (e.g. Pharmingen, Dianova) or purified from the supernatant of the respective hybridoma cells using Protein A- or Protein G- affinity chromatography. Purified monoclonal antibodies may be coupled by various methods known in the art, preferably by covalently coupling antibody amino groups to CNBr-activated sepharose.

Immunoisolation of MHC molecules may be performed by incubating the antibody-beads with the cell lysate under rotation for several hours or chromatographically by pumping the cell lysate through a micro-column. Washing of the antibody-beads may be performed in eppendorf tubes or in the microcolumn. The efficacy of the immunoprecipitation may be analysed by SDS-PAGE and western blotting using antibodies recognizing denatured MHC molecules (anti-HLA-DRalpha: 1B5; anti-HLA class I: HC10 or HCA2).

The sequestered MHC class II molecule-peptide complexes are washed with water or low-salt buffer before elution in order to remove residual detergent contaminants. The low salt buffer may be a Tris, phosphate or acetate buffer in a concentration range of 0.5 -10 mM, preferably in a concentration of 0.5 mM. In a more preferred embodiment, the MHC class II molecule -peptide complexes are washed with ultrapure water (sequencing grade) conventionally used for HPLC analysis, preferably with ultrapure (sequencing grade) water from MERCK. The washing step may be carried out by ultrafiltration. The ultrafiltration may be carried out in an ultrafiltration tube with a cut-off of 30 kD, 20 kD, 10 kD or 5 kD, preferably of 30 kD and a tube volume of 0.5 -1.0 ml ("Ultrafree" tubes; Millipore). The washing in the ultrafiltration tube may be carried out 4 to 12 times, preferably 6 to 10 times, with a volume of 10 to 20 times the volume of the beads carrying the receptor-peptide complexes, preferably with a volume of 15 times the beads. The eluted peptides may be separated from the remaining MHC class II molecules using the same ultrafiltration tube. The eluted peptides may then be lyophilized.

By eluting the peptides from the MHC class II molecules, a complex mixture of naturally processed peptides derived from the source of potential antigen and from polypeptides of intra- or extracellular origin, is obtained. Only after elution, peptides can be separated and subjected to sequence analysis.

The antigenic peptides in the method of the present invention may be eluted by a variety of methods known in the art, preferably by using diluted acid, e.g., diluted acetonitrile (Jardetzky TS et al., Nature 1991 353, 326-329), diluted acetic acid and heating (Rudensky AY et al., Nature 1991,353, 622-626; Chicz RM et al., Nature 1992, 358, 764-768) or diluted trifluoro acetic acid at 37°C (Kropshofer H et al., J Exp Med 1992,175,1799-1803). Most preferably, the peptides are eluted at 37°C with diluted trifluoro acetic acid.

The isolated antigenic peptides are then separated, detected and identified. By detecting it is understood that the amino acid sequence of the individual peptides in the mixture of isolated antigenic peptides is elucidated by methods adequate to detect and sequence femtomolar amounts of peptides. By identifying it is understood that it is established from which proteins or polypeptides the antigenic peptides are derived and which sequence they constitute within these proteins or polypeptides.

In a first step, the complex mixture of eluted peptides may be separated by one of a variety of possible chromatographic methods, e.g. by reversed phase, anion exchange, cation exchange chromatography or a combination thereof. Preferably, the separation is performed by C18-reverse phase chromatography or by reversed-phase / cation exchange two-dimensional HPLC, denoted as MudPit (Washburn MP et al., Nat Biotechnol., (2001), 19, 242-247).

The separation is done in a HPLC mode utilizing fused-silica micro-capillary columns which are either connected to a nano-flow electrospray source of a mass spectrometer or to a micro-fractionation device which spots the fractions onto a plate for MALDI analysis.

Liquid chromatography comprises peptide fractionation by the use of a strong ion exchange material and a hydrophobic reversed-phase material. For the elution of the peptides from the ion exchange and reversed-phase material different elution programs are run one after another comprising elutions with salt and with organic solvents, e.g., acetonitrile. The elution from the reversed-phase material is conducted in several steps of linear gradients of different lengths and slopes. A contamination in the sample to be fractionated may be any contamination whose elution competes with the detection of the peptide peaks in the mass spectrometer. Therefore, in order to prevent simultaneous elution, the contaminants have to be eluted with a sufficient solvent volume prior to the peptide elution step. Depending on the column used for liquid chromatography the solvent volume sufficient to elute the contaminants prior to the peptide elution step may be 100 to 200 times the column volume.

A variety of mass spectrometric techniques are suitable, preferably MALDI-post source decay (PSD) MS or electrospray ionization tandem mass spectrometry (ESI-MS), most preferably ion-trap ESI-MS.

The sequences of the individual peptides can be determined by means known in the art. Preferably, sequence analysis is performed by fragmentation of the peptides and computer-assisted interpretation of the fragment spectra using algorithms, e.g. MASCOT or SEQUEST. Both computer algorithms use protein and nucleotide sequence databases to perform cross-correlation analyses of experimental and theoretically generated tandem mass spectra. This allows automated high through-put sequence analysis.

The isolated and identified antigenic peptides of the invention can be validated by the MHC binding motif, the MHC binding capacity and/or by T cell recognition.

### MHC binding motif

Peptides associated to a particular MHC molecule (allelic variant) have common structural characteristics, denoted as binding motifs, necessary to form stable complexes with MHC molecules. Peptide ligands eluted from MHC class I molecules are relatively short, ranging from 8-11 amino acids. Moreover, 2 or 3 side chains of the peptide are relevant for binding. The position of the respective amino acid side chains varies with the HLA allele, most often two of these so-called "anchor" residues are located at positions 2 and 9. With respect to a particular anchor position, only 1 or 2 amino acids normally can function as anchor amino acids e.g. leucine or valine V at position 2 in the case of HLA-A2.

In the case of MHC class II molecules, the peptide length varies from 11 to 25 amino acids, as longer peptides can bind since both ends of the peptide binding groove are open. Most HLA class II molecules accommodate up to 4 anchor residues at relative positions P1, P4, P6 and P9 contained in a nonameric core region. This core region, however, can have variable distance from the N-terminus of the peptide. In the majority of cases, 2-4 N-terminal residues precede the core region. Hence, the P1 anchor residues is located at positions 3, 4 or 5 in most HLA class II associated peptides. Peptides eluted from HLA-DR class II molecules share a big hydrophobic P1 anchor, represented by tyrosine, phenylalanine, tryptophane, methionine, leucine, isoleucine or valine.

The position and the exact type of anchor residues constitute the peptide binding motif which is known for most of the frequently occurring HLA class II allelic products. A computer algorithm allowing motif validation in peptide sequences is "Tepitope", available by vaccinome.

### MHC binding capacity

Peptides identified by the method of the invention may be tested for their ability to bind to the appropriate MHC class II molecule by methods known in the art using, for example, isolated MHC class II molecules and synthetic peptides with amino acid sequences identical to those identified by the method of the invention (Kropshofer H et al., J. Exp. Med. 1992; 175, 1799-1803; Vogt AB et al., J. Immunol. 1994; 153,1665-1673; Sloan VS et al., Nature 1995; 375, 802-806). Alternatively, a cellular binding assay using MHC class II expressing cell lines and biotinylated peptides can be used to verify the identified epitope (Arndt SO et al., EMBO J., 2000; 19, 1241-1251)

In both assays, the relative binding capacity of a peptide is measured by determining the concentration necessary to reduce binding of a labelled reporter peptide by 50%. This value is called IC₅₀. Peptide binding with a reasonable affinity to the relevant HLA class II molecules attain IC₅₀ values not exceeding 10-fold the IC₅₀ of established reference peptides.

The same binding assays can also be used to test the ability of peptides to bind to alternative class II MHC molecules, i.e., class II MHC molecules other than those from which they were eluted using the method of the invention. The diagnostic methods of the invention using such peptides and therapeutic methods of the invention, using either the peptides or peptides derived from them, can be applied to subjects expressing such alternative class II MHC molecules.

### T cell recognition

The epitope verification procedure may involve testing of peptides identified by the method of the invention for their ability to activate CD4+ T cell populations. Peptides with amino acid sequences either identical to those identified in the present invention or corresponding to a core sequence derived from a nested group of peptides identified in the present invention are synthesized. The synthetic peptides are then tested for their ability to activate CD4+ T cells from (a) test subjects expressing the MHC class II molecule of interest and having at least one symptom of the disease; and (b) control subjects expressing the MHC class II molecule of interest and having no symptoms of the disease. Additional control subjects can be those with symptoms of the disease and not expressing the MHC class II molecule of interest.

In some diseases (e.g., those with an autoimmune component) responsiveness in the CD4+ T cells of test subjects but not in CD4+ T cells of the control subjects described in (b) provides confirmatory evidence that the relevant peptide is an epitope that activates CD4+ T cells that can initiate, promote, or exacerbate the relevant disease. In other diseases (e.g., cancer or infectious diseases without an autoimmune component), a similar pattern of responsiveness and non-responsiveness to that described in the previous sentence would indicate that the relevant peptide is an epitope that activates CD4+ T cells that can mediate immunity to the disease or, at least, a decrease in the symptoms of the disease.

CD4+ T cell responses can be measured by a variety of in *vitro* methods known in the art. For example, whole peripheral blood mononuclear cells (PBMC) can be cultured with and without a candidate synthetic peptide and their proliferative responses measured by, e.g., incorporation of [³H] -thymidine into their DNA. That the proliferating T cells are CD4+ T cells can be tested by either eliminating CD4+ T cells from the PBMC prior to assay or by adding inhibitory antibodies that bind to the CD4+ molecule on the T cells, thereby inhibiting proliferation of the latter. In both cases, the proliferative response will be inhibited only if CD4+ T cells are the proliferating cells. Alternatively, CD4+ T cells can be purified from PBMC and tested for proliferative responses to the peptides in the presence of APC expressing the appropriate MHC class II molecule. Such APC can be B-lymphocytes, monocytes, macrophages, or dendritic cells, or whole PBMC. APC can also be immortalized cell lines derived from B-lymphocytes, monocytes, macrophages, or dendritic cells. The APC can endogenously express the MHC class II molecule of interest or they can express transfected polynucleotides encoding such molecules. In all cases the APC can, prior to the assay, be rendered non-proliferative by treatment with, e.g., ionizing radiation or mitomycin-C.

As an alternative to measuring cell proliferation, cytokine production by the CD4+ T cells can be measured by procedures known to those in art. Cytokines include, without limitation, interleukin-2 (IL-2), interferon-gamma (IFN-gamma), interleukin-4 (IL-4), TNF-alpha, interleukin-6 (IL-6), interleukin-10 (IL-10), interleukin-12 (oil- 12) or TGF-beta. Assays to measure them include, without limitation, ELISA, and bio-assays in which cells responsive to the relevant cytokine are tested for responsiveness (e.g., proliferation) in the presence of a test sample.

Alternatively, cytokine production by CD4+ lymphocytes can be directly visualized by intracellular immunofluorescence staining and flow cytometry.

Moreover, the MHC class II antigenic peptides of the present invention may be used in the diagnosis of RA. Therefore, a further embodiment of the invention is the use of an antigenic peptide according to the present invention as a marker for RA.

Preferably, a MHC class II antigenic peptide comprising (a) at least the amino acid sequence of the peptide binding motif of SEQ ID NO. 49, or (b) at least the amino acid sequence of the peptide binding motif of SEQ ID NO. 49 with additional N-and C-terminal flanking sequences of a corresponding sequence selected from the group consisting of SEQ ID NOs. 1 to 3, is used as a marker for RA.

In another embodiment, the antigenic peptides of the invention may be used as response markers to track the efficacy of a therapeutic regime. Essentially, a baseline value for an antigenic peptide can be determined, then a given therapeutic agent is administered, and the levels of the antigenic peptide are monitored subsequently, whereas a change in the level of the antigenic peptide is indicative of the efficacy of a therapeutic treatment.

Furthermore, the antigenic peptides which are only found in certain stages or phases of a disease, preferably of RA, may be utilized as stage-specific markers. Essentially, the levels of the antigenic peptides which have been linked to a certain disease stage are monitored regularly, thereby providing information about the stage of the disease and its progression.

The invention also includes the use of the polypeptides the RA antigenic peptides are derived from as markers for the diagnosis and monitoring of a disease, preferably of RA, and in particular, of erosive versus non-erosive RA. The rationale for the use of the respective proteins is that DCs reside in most tissues where they capture exogenous antigens via specific receptors and via specialized endocytotic mechanisms (e.g. macropinocytosis) followed by presentation of the processed antigens as peptides on MHC class II molecules. Previous studies have shown that the frequency of a peptide epitope found in the context of MHC class II molecules, e.g. the RA antigenic peptides, in the majority of cases mirrors the abundance of the protein from which this particular peptide was derived from. Therefore, not only the RA antigenc peptides but also the corresponding proteins can serve as markers for RA.

The diagnosis of RA can be made by examining expression and/or composition of a polypeptide or peptide marker for RA, by a variety of methods, including enzyme linked immunosorbent assays (ELISAs), Western blots, immunoprecipitations and immunofluorescence. A test sample from an individual is assessed for the presence of an alteration in the expression and/or an alteration in composition of a polypeptide or a peptide of the present invention. An alteration in expression of a polypeptide or peptide can be, for example, an alteration in the quantitative polypeptide expression (*i.e*., the amount of polypeptide produced); an alteration in the composition of a polypeptide is an alteration in the qualitative polypeptide expression (*e.g*., expression of a mutant polypeptide or of a different splicing variant).

Both such alterations (quantitative and qualitative) can also be present. An "alteration" in the polypeptide expression or composition, as used herein, refers to an alteration in expression or composition in a test sample, as compared with the expression or composition of the peptide or polypeptide in a control sample. A control sample is a sample that corresponds to the test sample (e.g., is from the same type of cells), and is from an individual who is not affected by RA. An alteration in the expression or composition of the peptide or polypeptide in the test sample, as compared with the control sample, is indicative of RA or a susceptibility to RA. Various means of examining expression or composition of a peptide or polypeptide of the present invention can be used, including spectroscopy, colorimetry, electrophoresis, isoelectric focusing, and immunoassays (*e.g*., David et al., U.S. Pat. No. 4,376,110) such as immunoblotting (see also Current Protocols in Molecular Biology, particularly chapter 10). For example, in one embodiment, an antibody capable of binding to the polypeptide (*e.g*., as described above), preferably an antibody with a detectable label, can be used. Antibodies can be polyclonal, or more preferably, monoclonal. An intact antibody, or a fragment thereof (*e.g*., Fab or F(ab')₂) can be used. The term "labeled", with regard to the probe or antibody, is intended to encompass direct labeling of the probe or antibody by coupling (*i.e*., physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin.

Western blotting analysis, using an antibody as described above that specifically binds to a peptide or polypeptide of the present invention, may be used to measure the level or amount of a peptide or polypeptide in a test sample and comparing it with the level or amount of the peptide or polypeptide in a control sample. Preferably the peptide or polypeptide in a test sample is measured in a homogenous or a heterogenous immuno assay. A level or amount of the polypeptide in the test sample that is higher or lower than the level or amount of the polypeptide in the control sample, such that the difference is statistically significant, is indicative of an alteration in the expression of the polypeptide, and is diagnostic for a RA or a susceptibility to RA.

Therefore, the present invention also relates to a diagnostic composition comprising an antibody reactive with a MHC class II antigenic peptide of the invention.

In a further embodiment the antigenic peptides of the invention or the proteins they are derived from may be used in the prevention and treatment of a disease, preferably of RA.

One aspect of the invention is a therapeutic purpose, wherein one or more of the identified antigenic peptides are used to vaccinate patients against RA, preferably against erosive and/or non-erosive RA. In the course of the vaccination the antigenic peptide would induce an antigen-specific T cell tolerance in the patient which would ultimately lead to regression of the disease or to an attenuation of disease development.

A promising strategy to induce specific immune tolerance in future clinical trials is the use of DNA tolerizing vaccines. DNA tolerizing vaccines encoding autoantigens alone were shown to reduce T cell proliferative responses (Ruiz, P. et al., Immunol. 162 (1999) 3336-3341), while DNA tolerizing vaccines co-delivering autoantigen plus IL-4 also induced protective T_{H}2 responses (Garren, H. et al., Immunity 15 (2001) 15-22). Examples of non-polynucleotide-specific tolerizing therapies under development include protein antigens, naturally processed peptides, altered peptide ligands, other biomolecules, such as DNA, or proteins and peptides containing posttranslational modifications, and antigens delivered orally to induce "oral tolerance" (reviewed in: Robinson, W.H. et al., Clin Immunol 103 (2002) 7-12). A potential adverse effect with regard to tolerizing therapies is the development of autoimmunity.

To this end, the relevant RA antigenic peptides may be directly administered to the patient in an amount sufficient for the peptides to bind to the MHC molecules, and provoke peripheral tolerance of T cells.

Alternatively, the antigenic peptides of the invention may be utilized for the generation of vaccines based on DCs. In this case, autologous DCs derived from patients' monocytes may be pulsed with the relevant peptides or recombinant proteins containing the relevant peptide sequences.

Therefore, the present invention provides a pharmaceutical composition comprising a MHC class II antigenic peptide comprising (a) at least the amino acid sequence of the peptide binding motif of SEQ ID NO. 49, or (b) at least the amino acid sequence of the peptide binding motif of SEQ ID NO. 49 with additional N-and C-terminal flanking sequences of a corresponding sequence selected from the group consisting of SEQ ID NOs. 1 to 3, or an antibody reactive with said antigenic peptide, and optionally a pharmaceutically acceptable excipient, diluent or carrier. The antigenic peptide has to be present in an amount sufficient to tolerize specific lymphocytes. Such an amount will depend on the peptide used, the administration, the severity of the disease to be treated and the general conditions of the patient and will usually range from 1 to 50 mg/ml, for example in case of peptides being loaded on dendritic cells.

An acceptable excipient, diluent or carrier may be phosphate buffered saline for in *vitro* studies and physiological salt solutions for in vivo applications.

"Vaccination" herein means both active immunization, i. e. the *in vivo* administration of the peptides to elicit an *in vivo* immune tolerance directly in the patient and passive immunization, i. e. the use of the peptides to tolerize in *vitro* CD4+ T lymphocytes or to stimulate autologous or allogeneic dendritic cells, which are subsequently re-inoculated into the patient.

The present invention also provides the antigenic peptides, antibodies, nucleic acids, host cells, methods, compositions and uses substantially as herein before described especially with reference to the Examples.

Having now generally described this invention, the same will become better understood by reference to the specific examples, which are included herein for purpose of illustration only and are not intended to be limiting unless otherwise specified, in connection with the following figures.

### Examples

The examples below are illustrated in connection with the figures described above and based on the methodology summarized in Fig 1, as described in the following. Commercially available reagents referred to in the examples, were used according to manufacturer's instructions unless otherwise indicated.

### Methodology of the invention

### Dendritic cells and culturing

The study was performed with human dendritic cells which were differentiated from monocytes, as described below. Monocytes were purified from human peripheral blood. The blood was taken from healthy donors with the following haplotypes: (1) *HLA-DRB1*0401, *03011,* (2) *HLA-DRB1*0401, *0304,* (3) *HLA-DRB1*0401, *1301,* (4) *HLA-DRB1*0401, *0701, HLA-DRB1 *0401, *0407.*

All cells were cultured in RPMI 1640 medium (short: RPMI) supplemented with 1 mM Pyruvate, 2 mM Glutamine and 10% heat-inactivated fetal calf serum (Gibco BRL, Rockville, MD).

### Isolation of peripheral blood mononuclear cells (PBMCs)

Peripheral blood was obtained from the blood bank in Mannheim, Germany as standard buffy coat preparations from healthy donors. Heparin (200 I.U./ml blood, Liquemine, Roche) was used to prevent clotting. Peripheral blood mononuclear cells (PBMCs) were isolated by centrifugation in LSM^{®} (1.077-1.080 g/ml; ICN, Aurora, OH) at 800g (room temperature) for 30 min. PBMCs were collected from the interphase and washed twice in RPMI containing 20 mM Hepes (500g for 15 min, 300g for 5 min). In order to remove erythrocytes, PBMCs were treated with ALT buffer (140 mM ammonium chloride, 20 mM Tris, pH 7.2) for 3 min at 37°C. PBMCs were washed twice with RPMI containing 20 mM Hepes (200g for 5 min).

### Generation of dendritic cells from peripheral blood monocytes.

Monocytes were isolated from PBMCs by positive sorting using anti-CD 14 magnetic beads (Miltenyi Biotech, Auburn, CA) according to the manufacturer's protocol. Monocytes were cultured in RPMI supplemented with 1% non-essential amino acids (Gibco, BRL, Rockville, MD), 50 ng/ml recombinant human granulocyte macrophage-colony stimulating factor (GM-CSF; S.A. 1.1x10⁷ U/mg) (Leucomax; Novartis, Basel Switzerland) and 3 ng/ml recombinant human IL-4 (S.A. 2.9x10⁴ U/µg) (R&D Systems, Minneapolis, MN). Monocytes were seeded at 0.3 x 10⁶/ml in 6-well plates (Costar) for 5 days to obtain immature dendritic cells.

The quality of monocyte-derived immature dendritic cells was routinely monitored by flow-cytometric analysis and assessed to be appropriate when they displayed the following phenotype: CD1a (high), CD3 (neg.), CD14 (low), CD19 (neg.), CD56 (neg.), CD80 (low), CD83 (neg.), CD86 (low) and HLA-DR (high). In contrast, mature dendritic cells (cf. below) display the following phenotype: CD1a (low), CD80 (high), CD83 (high), CD86 (high) and HLA-DR (high). Monoclonal antibodies against CD1a, CD3, CD14, CD19, CD56, CD80, CD83, CD86 as well as the respective isotype controls were purchased from Pharmingen (San Diego, CA).

### Exposure of dendritic cells to serum or synovial fluid

Serum and synovial fluid were irradiated for 30 min with ¹³⁷Cs (70 TBq). To feed dendritic cells with serum- or synovia-derived antigen, 6 x 10⁶ immature dendritic cells were pulsed with either 1 ml serum or 0.6 ml synovial fluid. At the same time maturation of dendritic cells was induced by adding 10 ng/ml recombinant human tumor necrosis factor alpha (TNFα; S.A. 1.1x10⁵ U/µg). As a control, 6 x 10⁶ immature dendritic cells were incubated with TNFα alone.

After 24 hrs in culture, mature dendritic cells were harvested by centrifugation at 300g for 10 min. Cells were washed with PBS and transferred to an eppendorf tube. After centrifugation at 400g for 3 min, the supernatant was completely removed and the cells were frozen at -70°C.

### Generation of anti-HLA class II beads

The anti-HLA-DR monoclonal antibody (mAb) L243 (ATCC, Manassas, VA) was produced by culturing the respective mouse hybridoma cell line. mAb L243 was purified using ProteinA sepharose (Pharmacia, Uppsala, Sweden) and immobilized to CNBr-activated sepharose beads (Pharmacia) at a final concentration of 2.5 mg/ml, according to the manufacturer's protocol. L243 beads were stored in PBS containing 0.1% Zwittergent 3-12 (Calbiochem, La Jolla, CA).

### Nano-scale purification of HLA-DR-peptide complexes

Pellets of frozen dendritic cells were resuspended in 10-fold volume of ice cold lysis buffer (1% Triton-X-100, 20 mM Tris, pH 7.8, 5 mM MgCl₂, containing protease inhibitors chymostatin, pepstatin, PMSF and leupeptin (Roche, Mannheim, Germany)) and lysed in a horizontal shaker at 1000 rpm, 4°C for 1 h. The cell lysate was cleared from cell debris and nuclei by centrifugation at 10000g, 4°C for 10 min. The lysate was co-incubated with L243 beads (5-10 µl L243 beads per 100 µl cell lysate) in a horizontal shaker at 1000 rpm, 4°C for 2 hrs. Immunoprecipitated HLA-DR-peptide complexes bound to L243 beads were sedimented by centrifugation at 1000g, 4°C for 1 min and washed four times with 500 µl 0.1% Zwittergent 3-12 (Calbiochem) in PBS.

The efficacy of depletion of HLA-DR-peptide complexes was monitored by analyzing the respective cell lysates before and after immunoprecipitation and aliquots of the beads by western blotting using the anti-HLA-DRα-specific mAb 1B5 (Adams, T.E. et al., Immunology 50 (1983) 613-624).

### Elution of HLA-DR-associated peptides

HLA-DR-peptide complexes bound to L243 beads were resuspended in 100 µl H₂O (HPLC-grade; Merck, Darmstadt, Germany), transferred to an ultrafiltration tube, Ultrafree MC, 30 kD cut-off (Millipore, Bedford, MA) and washed 10 times with 100 µl H₂O (HPLC-grade) by centrifugation for 1-2 min at 10000g at RT. For eluting the bound peptides, 60 µl 0.1% trifluoracetic acid (Fluka, Buchs, Switzerland) in H₂O (HPLC-grade) was added and incubation was performed for 30 min at 37°C. Eluted peptides were collected in a new eppendorf tube by centrigugation of the Ultrafree unit at 10000g for 3 min at RT and immediately lyophilized in a Speed-Vac™ vacuum centrifuge.

### Fractionation by two-dimensional nanoflow LC

To perform high-throughput sequencing of complex peptide mixtures, the MudPIT (multidimensional protein identification technology) was used (Washburn, M.P. et al., Nat Biotechnol 19 (2001), 242-247) which is based on liquid chromatographic fractionation followed by mass spectrometric sequence determination.

To this end, lyophilized peptides eluted from HLA molecules were resuspended in a buffer containing 5% (v/v) acetonitrile (ACN), 0.5% (v/v) acetic acid, 0.012% (v/v) heptafluoro butyric acid (HFBA) and 1% (v/v) formic acid. The peptide mixture was fractionated on a fused-silica microcapillary column (100 µm i.d. x 375 µm) generated by a Model P-2000 laser puller (Sutter Instrument Co., Novato, CA). The microcolumn was packed with 3 µm / C18 reversed-phase material (C18-ACE 3 µm [ProntoSIL 120-3-C18 ACE-EPS, Leonberg, Germany]) followed by 3 cm of 5 µm cation exchange material (Partisphere SCX; Whatman, Clifton, USA).

A fully automated 8-step gradient separation on a LC Packings UltiMate HPLC (LC Packings, San Francisco, USA) was carried out, using the following buffers: 5% ACN / 0.012% HFBA / 0.5% acetic acid (buffer A), 80% ACN / 0.012% HFBA / 0.5% acetic acid (buffer B), 250 mM ammonium acetate / 5% ACN / 0.012% HFBA / 0.5% acetic acid (buffer C), and 1.5 M ammonium acetate / 5% ACN / 0.012% HFBA / 0.5% acetic acid (buffer D). The first 116 min step consisted of a 75 min gradient from 0 to 40% buffer B followed by a 10 min gradient from 40 to 80% buffer B, a 6 min hold at 80% buffer B and a 10 min equilibration step with 100% buffer A. The next 5 steps (146 min each) were characterized by the following profile: 5 min 100% buffer A, 5 min gradient from 0 to x% buffer C, 5 min 100% buffer A, 30 min gradient from 0 to 10% buffer B, 55 min gradient from 10 to 35% buffer B, 20 min gradient from 35 to 50% buffer B, 10 min gradient from 50 to 80% buffer B, a 6 min hold at 80% buffer B, and a 10 min equilibration step with 100% buffer A. The buffer C percentages (x) in steps 2-6 were as follows: 20, 40, 60, 80, and 90%. The 30 min gradient from 0 to 10% buffer B, which is the first linear elution step from the reversed-phase material, was needed in order to sufficiently separate peptide elution from the elution of a major contaminant (m/z=945) which otherwise would have led to the loss of the more hydrophilic peptide peaks. Step 7 consisted of the following profile: 5 min 100% buffer A, 20 min 100% buffer C, 5 min gradient from 0 to 10% buffer B, 35 min gradient from 10 to 35% buffer B, 50 min gradient from 35 to 50% buffer B, 10 min gradient from 50 to 80% buffer B, a 5 min hold at 80% buffer B and a 10 min equilibration step with 100% buffer A. Step 8 was identical to step 7 with the exception of using buffer D instead of buffer C.

### Ion trap MS/MS mass spectrometry

The HPLC column was directly coupled to a Finnigan LCQ Deca XP Plus ion trap mass spectrometer (Thermo Finnigan, San Jose, USA) equipped with a nano-LC electrospray ionization source. Mass spectrometry in the MS/MS mode was performed according to the manufacturer's protocol. Peptides were identified by the SEQUEST algorithm (U.S. patents 6,017,693 and 5,538,897).

### MALDI-TOF mass spectrometry

Peptides spotted onto an AnchorChip plate were co-cristallized with matrix (5 mg/ml; α-cyano-4-hydroxy-cinnamic acid (Merck, Darmstadt, Germany), 50% acetonitrile, 0.1% trifluoroacetic acid). For qualitative analysis of the whole peptide repertoire, samples were analyzed on an Ultraffex^{™} MALDI-TOF mass spectrometer (Bruker, Bremen, Germany), according to the manufacturer's protocol.

### Sequence identification by SEQUEST and differential dataset analysis.

MS/MS fragmentation data were analyzed with the software SEQUEST (Thermo Finnigan, San Jose, USA). From an in-house protein database, which was created based on the public databases Swiss-Prot and TrEMBL, SEQUEST extracted for each spectrum all peptide sequences that corresponded to the molecular mass of the parent ion and measured the degree of similarity between the experimental spectrum and the theoretical, *in silico* generated, spectrum. Only the top-scoring candidate sequence was listed.

The peptide sequences derived from the SEQUEST analysis and their accompanying information on mass accuracy, scoring parameters and peptide origin were stored in an appropriately designed relational database and further processed. Certain constraints were enforced in order to guarantee the storage of only significant sequences with satisfying SEQUEST scores. The two most important constraints were: (i) keep only those sequences that have a cross correlation coefficient (CC) higher than a certain value and (ii) from the remaining sequences keep those ones which have a predefined delta cross correlation coefficient (ΔCC). For both criteria the minimum chosen values are based on empirical knowledge of interpreting SEQUEST results.

A dataset was defined as the sum of data from a particular set of spectra. The design of database and software allowed queries on a single dataset as well as comparisons of multiple datasets. Such a database and software design enables comparative sample analysis, which is not provided by SEQUEST. For instance, possible queries on a single dataset could provide information on the score distribution among the stored spectra, on the existence of further sequence length variants or common subsequences, or on the protein origin of peptide sequences. Since the occurrence of truncation variants of the same epitope is a general characteristic of class II MHC-bound peptides, the existence of length variants in a dataset provides additional strong evidence for the presence of an epitope in a set of spectra.

The most important feature in the analysis of multiple datasets is the possibility to extract a common subset of sequences that satisfies a given criterion. Such a criterion could be based on sequence similarity, e.g., within all sequences of a collection of datasets, those sequences were selected that had at least one subsequence in common with any other sequence. Such comparisons across different datasets constitute the differential approach (RA samples versus control samples) and thereby optimize the search for candidate RA marker peptides.

The pairwise similarity scores between sequences were calculated by a software routine, which is an implementation of a standard string-comparison algorithm. Subsequently, these scores were used to group closely-related sequences (sequences sharing a common subsequence) in well-separated clusters by an additionally developed software routine, which is based on a well-established algorithm (hierarchical clustering, UPGMA).

The generated clusters (e.g. of peptide truncation variants) were then used to identify closely-related sequences across different datasets.

Overall, the data evaluation software provided the ability to perform swiftly and reproducibly the following:
- Select from the sequence output generated by SEQUEST those sequences that satisfy reliable empirical criteria.
- Store the data in a database appropriately designed for the discovery process at hand.
- Extract information about the sequence content of each stored dataset. This information is valuable in assessing the importance of individual sequences within the given dataset and, consequently, across multiple datasets.
- Provide, by virtue of the multiple dataset comparisons, a tool that realizes the differential approach, namely the study of the actual sequence content of one sample versus other(s).

### Purification of HLA-DR molecules

HLA-DR molecules were purified from 10¹⁰ EBV-transformed B cell lines or T2-transfectants by affinity chromatography, using anti-DR monoclonal antibody L243, as previously described (Kropshofer H. et al., PNAS 92 (1995) 8313-8317).

### In vitro peptide binding assay

HA(307-319), PKYVKQNTLKLAT, is an immunodominant epitope from influenza virus hemagglutinin that binds well to HLA-DR4 molecules and was used as a reporter - peptide in an *in vitro* peptide binding assay (Rothbard, J.B. et al., Cell (1988) 52:515-523).

Purified detergent-solubilized HLA-DR4 molecules (200 nM) were co-incubated with biotinylated HA(307-319) peptide (200 nM) and graded amounts of competitor peptide (100 nM-10 µM) for 24 hrs at 37°C in binding buffer (50 nM sodium phosphate, 50 mM sodium citrate, pH 4.8, 0.1% Zwittergent 3-12) in a total volume of 50 µl. The competitor peptides were derived from the candidate RA antigens identified in this study and purchased as synthetic peptides from Medprobe (Lund, Sweden).

3 x 10 µl were then diluted 10-fold in PBS containing 0.05% Tween-20 and 1% BSA and incubated for 2 hrs in a microtiterplate (Nalge Nunc), which has been coated over night with anti-DR monoclonal antibody L243. Afterwards the samples were developed by incubation with 0.1 µg/ml EU-labeled streptavidin (Wallay Oy, Turku, Finland) for 45 min according to the manufacturer's protocol. After intensive washing with 0.05% Tween-20 in PBS, Europium fluorescence was measured with a time-resolved fluorometer (VICTOR 1420, Wallac/Perkin Elmer Life Sciences) to quantify the binding of biotinylated HA(307-319) peptide to HLA-DR4 molecules (Arndt, S.O. et al., EMBO J. 19 (2000) 1241-1251).

### Example 1

In this example, the technique mentioned in Figure 1 was used to identify novel HLA-DR-associated peptide markers derived from serum and synovial fluid of patients with non-erosive RA.

6 x 10⁶ immature dendritic cells were pulsed with either 1 ml serum (5 samples) or 0.6 ml synovial fluid (2 samples) of patients with non-erosive RA and cultured for 24 hrs in the presence of 10 ng/ml TNFα. As a control, 6 x 10⁶ dendritic cells were cultured in the presence of TNFα (10 ng/ml) without adding serum but 1 ml of PBS. In an additional experiment 6 x 10⁶ dendritic cells were pulsed with 1 ml serum from 2 healthy test persons and cultured for 24 hrs in the presence of TNFα (10 ng/ml).

Dendritic cells were lysed in detergent TX-100 and HLA-DR molecules were isolated using mAb L243. HLA-DR-associated peptides were eluted with 0.1% TFA and analyzed by high-throughput 2D-LC-MS/MS technology. Peptide identification was achieved by using the SEQUEST algorithm. The peptide sequences derived from the SEQUEST analysis and accompanying information on mass accuracy, scoring parameters and peptide origin were stored in a database and further processed.

The peptide sequences identified from unpulsed DCs (control 1) and from DCs pulsed with the serum of healthy test persons (control 2) were compared with the peptide sequences identified from DCs pulsed with the serum of non-erosive RA patients. Among the RA-specific sequences, only those peptides were selected for further evaluation that re-occurred in at least three of five non-erosive RA samples.

In each serum sample roughly 600±150 individual peptide sequences (cross correlation coefficient CC > 3.0 and ΔCC > 0.15) were identified. In the synovia samples the number of individual peptide sequences was slightly smaller (400±30). Approximately 80-85% of the peptides found in RA samples were also identified in control samples, underlining the high reproducibility of the analysis. In the majority of cases, several length variants of the same epitope could be identified which is a typical characteristic of class II MHC-bound antigens and supports the validity of the results (Jones, E.Y., Curr Opin Immunol 9 (1997) 75-79). Further confidence in the quality of the data relies on the fact that several of the identified peptides or proteins have already been described in the context of MHC class II molecules: epitopes derived from ubiquitous proteins like Hsp70, enolase, annexin II, cathepsin C or collagen II, as well as from MHC molecules (HLA-A, -B, -C, -E, -G, and β₂-microglobulin) and CLIP (Chicz, R.M. et al., J Exp Med 178 (1993) 27-47; Sinigaglia, F. & Hammer, J., Curr Opin Immunol 6 (1994) 52-56; Arnold-Schild, D. et al., J Immunol 162 (1999) 3757-3760; Vogt, A.B. & Kropshofer, H., Trends Biochem Sci 4 (1999) 150-154) were frequently detected.

RA-specific peptide sequences were further validated with regard to binding to the RA susceptibility allele *DRB1**0401 by using the TEPITOPE software (Hammer, J. et al., Adv Immunol 66 (1997) 67-100). This software provides means for the qualitative and quantitative prediction of T cell epitopes.

The output of the study consists of an epitope that occurred, apart from one exception, only in non-erosive RA samples (Table 1).

### Interferon-gamma-inducible lysosomal thiol reductase

A very interesting epitope which was identified in 3 out of 7 non-erosive RA samples from serum and synovia is derived from the interferon-gamma-inducible lysosomal thiol reductase (GILT): the 16-mer GILT (192-207) with the amino acid sequence of SEQ ID NO: 3 (Table 1). Further length variants in three other samples support the relevance of this epitope (Table 1): the 14-mer GILT (192-205; SEQ ID NO: 1) and the 17-mer GILT (192-208; SEQ ID NO: 2).

As judged from the shortest length variant, GILT (192-205), the epitope contains a suitable binding motif, with regard to binding to the RA susceptibility allele *DRB1*0401:* 196M serves as a P1 anchor, 199M as a P4 anchor and 201A as a P6 anchor. According to TEPITOPE scoring, the epitope has a binding score (threshold value) of 1% which is similar to the binding score of an epitope from influenza haemagglutinin (307-319) that was shown to be a strong *DRB1*0401* binder (Table 1) (Rothbard, J.B. et al., Cell 52 (1988) 515-523).

GILT is constitutively expressed in antigen-presenting cells, such as dendritic cells, macrophages and B cells, and facilitates unfolding of endocytosed antigens in MHC class II-containing compartments (MIIC) by enzymatically reducing disulfide bonds (Phan, U.T. et al., J Biol Chem 275 (2000) 25907-25914). Direct binding of GILT to HLA-DR molecules has been reported for B cells (Arunachalam, B. et al., J Immunol 160 (1998) 5797-5806). A rather long second epitope of GILT was found to bind to HLA-DR3 molecules: the 22-mer GILT (38-59) having the amino acid sequence SPLQALDFFGNGPPVNYKTGNL (Chicz, R.M. et al., J Exp Med 178 (1993) 27-47).

In addition to GILT (192-207), another epitope of the same protein was identified in several RA samples, but also in control samples: GILT (210-227) with the amino acid sequence QPPHEYVPWVTVNGKPLE. This epitope was accompanied by 3 other length variants: the 16-mer GILT (210-225), the 17-mer GILT (210-226) and the 19-mer GILT (210-228).

As indicated by its name, GILT expression can be induced by the pro-inflammatory cytokine interferon gamma (IFN-γ) in various types of cells, including macrophages, endothelial cells and fibroblasts (Luster, A.D. et al., J Biol Chem 263 (1988) 12036-12043). As IFN-γ is known to be present in inflamed joints of RA patients, GILT could become over-expressed in synovia and serum and, hence, could be taken up by DCs as an exogenous antigen. GILT (192-207) maybe derived from exogenous GILT. The other GILT epitope, which is also present in the control samples, may be derived from endogenous GILT, expressed by DCs. Alternatively, both GILT (192-207) and GILT (210-227) may be derived from endogenous GILT, in case that GILT processing and GILT-derived epitope presentation by DCs were critically altered upon contact with RA-associated material.

The identified epitope of Interferon-gamma-inducible lysosomal thiol reductase GILT (192-205) which has been described already in detail, was further analysed in an *in vitro* binding assay using synthetic GILT (192-205) peptide and purified HLA-DR4 molecules (Fig. 3): In agreement with TEPITOPE scoring, the peptide was shown to bind to HLA-DR4 with high affinity, comparable to the viral HA(307-319) peptide.

### Integrin beta-2

The extended analysis revealed the presence of an epitope which was identified in two out of seven non-erosive RA samples from serum and synovia and which is derived from the beta subunit of Integrin (ITB2): the 17-mer ITB2 (315-331; SEQ. ID NO: 58) with the amino acid sequence NIQPIFAVTSRMVKTYE (Table 1). One length variant was found: the 19-mer ITB2 (313-331; SEQ. ID NO: 59) supporting the validity of the identified epitope (Table 1).

The peptide sequence contains a moderate *HLA-DRB1*0401* binding motif with 3161 and 319I serving as P1 and P4 anchor, respectively (TEPITOPE binding score: 2%).

Integrins are a family of cell surface receptors that play important roles in embryogenesis, wound healing, immune responses, and cell adhesion. ITB2 is a heterodimeric receptor for intercellular and vascular adhesion molecules (ICAMs and VCAMs) and exclusively expressed on leukocytes. ICAMs and VCAMs are members of the immunoglobulin superfamily that has a central function in humoral and cell-mediated immune responses. Many cytokines, such as interferon-γ, IL-1 and TNFα, which are upregulated in inflammatory diseases like RA, induce expression of ICAMs on the surface of endothelial cells. It was shown that the expression of ICAM-1 and VCAM-1 is higher in synovial tissue of RA patients as compared to osteoarthritis patients (Furuzawa-Carballeda, J. et al., Scand J Immunol 50 (1999) 215-222).

Binding of ITB2 and other integrins to ICAMs allows leukocytes to infiltrate into their target tissues, e.g. the sites of inflammation. In order to function in a non-adherent or circulating mode, leukocytes constitutively express ITB2 and other integrins with low ligand-binding capacity. The ITB2/ICAM-1 interaction has become an important strategic target to approach inflammation, autoimmune diseases and cancer (Yusuf-Makagiansar, H. et al., Med Res Rev 22 (2002) 146-167), hence these findings strongly support the validity of the identified ITB2 epitope as a candidate marker for RA.

### Phosphatidylinositol-4,5-bisphosphate 3-kinase

Another epitope with a moderate *HLA-DRB1*0401* binding motif was identified in two non-erosive serum samples: the 17-mer PI3K (792-808; SEQ. ID NO: 60) with the amino acid sequence NKVFGEDSVGVIFKNGD derived from Phosphatidylinositol 3-kinase (PI3K) (Table 1). In this peptide 794V could serve as a hydrophobic P1 anchor, 797E as a negatively charged P4 anchor, and 799S as a typical DR4-P6 anchor (TEPITOPE binding score: 3%).

PI3K is ubiquitously expressed in many cell types and phosphorylates lipids, predominantly phosphatidylinositol-4,5-bisphosphate. The protein has emerged as a key signal transducer for survival factor receptors, including growth factors, cytokines, and integrins (reviewed by Toker, A. & Cantley, L., Nature 387 (1997) 673-676). In addition, PI3K plays an important role in the signaling pathway of Toll-like receptors (TLRs) that recognize a variety of microbial products, collectively termed pathogen-associated molecular patterns (PAMPs) (Fukao, T. & Koyasu, S., Trends Immunol 24 (2003) 358-363). Stimulation through TLRs by PAMPs, such as lipopolysaccharide (LPS) (endotoxin) triggers production of various cytokines, including IL-12, which is a key cytokine in TLR-mediated Th1 responses. It was shown that PI3K is an endogenous suppressor of TLR-mediated IL-12 production and limits excessive Th1 polarization. Thus PI3K was suggested to be a negative regulator of innate immune responses in order to prevent prolonged activation of innate immunity harmful to the host. The knowledge that autoimmune responses in RA rely on Th1 cytokines, ultimately links PI3K with RA. Although no specific pathogen has been consistently linked to the development of RA, it is believed that RA develops in two stages in which an initial response induced by foreign antigens, subsequently develops into a self-sustaining autoimmune response (Klinman, D., Arthritis Rheum 48 (2003) 590-593).

### Urokinase-type plasminogen activator

Another epitope which was only found in non-erosive RA samples was derived from a urokinase-type plasminogen activator (uPA): the 16-mer uPA (328-343; SEQ. ID NO: 61) with the amino acid sequence YPEQLKMTVVKLISHR (Table 1). With regard to HLA-DRB1*0401 binding, the sequence reveals a moderate binding motif: 332L, 335T and 337V are putative P1, P4 and P6 anchors, respectively (TEPITOPE binding score: 2%).

Plasminogen activators (PAs) are highly specific serine proteases generating plasmin from plasminogen. Two types of PAs, a urokinase-type (uPA) and a tissue-type (tPA) have been identified in mammals. The activities of PAs are controlled by natural inhibitors (PAIs). Plasmin is involved in inflammatory reactions by inducing cytokines such as TGFβ, and in cartilage and fibrin degradation. Evidences for ongoing coagulation within the rheumatoid joint together with an enhancement of uPA synthesis and activation in arthritic joints have led to the hypothesis that the PA/plasmin system is associated with the clinical severity of arthritis (reviewed by Busso, N. & Hamilton, J.A., Arthritis Rheum 46 (2002) 2268-2279). Based on *in vitro* studies, there are several cell types present in the inflamed joints of RA patients, in particular monocytes and synovial fibroblasts which can express PAs and PAIs and which therefore could contribute to the *in vivo* levels. The activity of PAs (i.e. uPA) is stimulated by cytokines, such as IL-1 and TNFα, which are known to be highly upregulated in serum as well as in synovial fluid of RA patients. These lines of evidence render uPA (328-343) a putative peptide marker for RA.

### Immunoglobulin heavy chain V-III region (V_{H}26)

One of the identified epitopes that display a very strong binding motif with regard to *HLA-DRB1*0401* binding, is derived from the V_{H}26 gene segment of the immunglobulin heavy chain: the 16-mer V_{H}26 (95-110; SEQ. ID NO: 62) with the amino acid sequence KNTLYLQMNSLRAEDT (Table 1). The high TEPITOPE binding score (1%) is substantiated by 99Y functioning as a very potent P1 anchor, 102M as a moderate P4 anchor and 104S as a typical HLA-DR4 P6 anchor.

Immunglobulins (Ig) are responsible for antigen binding and stimulate further immune reactions, e.g. by binding to isotope-specific Fc receptors. Interestingly, the human V_{H}26 gene segment appears to encode a high-avidity synovial rheumatoid factor and thus seems to have an impact on RA progression (Wong, A. et al., Autoimmunity 20 (1995) 191-199). The fact that the identified epitope V_{H}26 (95-110) was found in the serum of two seropositive RA patients (Table 1) correlates with the above observation.

### DJ-1 protein

In two non-erosive RA samples our continued studies revealed the presence of an epitope which is derived from a protein termed DJ-1: the 16-mer DJ-1 (135-150; SEQ. ID NO: 63) with the amino acid sequence NGGHYTYSENRVEKDG (Table 1). According to TEPITOPE scoring the peptide contains a rather weak *HLA-DRB1 *0401* binding motif with 139Y serving as a P1 anchor, 142S as a P4 anchor and 144N as a possible P6 anchor (binding score: 8%).

DJ-1 belongs to the ThiJ/PfpI protein family whose members are evolutionary distributed from Archaea to Eukarya. ThiJ/PfpI proteins share a conserved ThiJ domain that is structurally related to the type I glutamine amidotransferase domain (Lee, S.J. et al., J Biol Chem 25 (2003) Epub ahead of print).

DJ-1, which is preferentially expressed in testis and moderately in other tissues, was first identified as a novel candidate of the oncogene product that transformed mouse NIH3T3 cells in cooperation with ras. In the mean time additional physiological roles of DJ-1 were revealed including a strong correlation to Parkinson disease and sperm fertilization. DJ-1 seems to have multiple functions - here we provide the first indication for an association of DJ-1 with RA.

### Example 2

In this example, the same technology was used that has been described in detail in example 1. Serum (6 samples) and synovial fluid (2 samples) of patients with diagnosed erosive RA were utilized in this case to identify candidate markers specific for erosive RA.

The peptide sequences found in the erosive RA samples were compared with the sequences identified in unpulsed DCs (control 1) and in DCs pulsed with the serum of healthy test persons (control 2). Among the RA-specific sequences, only those peptides were selected for further evaluation that re-occurred in at least three of six erosive RA samples.

In this study one epitope was discovered which occurred, apart from one exception, only in erosive RA samples.

### Apolipoprotein B-100

The epitope which was mainly found in erosive RA sera (4 out of 8 erosive RA samples) is derived from apolipoprotein B-100: the 16-mer ApoB (2877-2892) with the amino acid sequence of SEQ ID NO: 4 (Table 2). In addition a length variant of the same epitope was identified (Table 2): the 17-mer ApoB (2877-2893; SEQ ID NO: 5). The following *DRB1*0401* binding motif can be predicted: 2881L as a P1 anchor, 2884D as a P4 anchor and 2886N as a P6 anchor (binding score 3%).

In an earlier study on EBV-B cells, the epitope ApoB (2885-2900), which partly overlaps with the epitope described here, has been found in the context of HLA-DR4 (Chicz, R.M. et al., J Exp Med 178 (1993) 27-47).

Apolipoprotein B-100 is a constituent of very low-density lipoproteins (VLDL) and low-density lipoproteins (LDL) and functions as a recognition signal for the cellular binding and internalization of LDL particles by the ApoB/E receptor (Yang, C.Y. et al., Nature 323 (1986) 738-742). Interestingly, an increased ratio of LDL cholesterol to HDL cholesterol was observed among newly diagnosed RA patients (Park, Y.B. et al., J Rheumatol 26 (1999) 1701-1704). The adverse lipid profile in active RA could be improved by treating RA patients with DMARDs without the use of lipid-lowering agents (Park, Y.B. et al., Am J Med 113 (2002) 188-193). Since an increased cardiovascular mortality among patients with chronic inflammatory diseases, such as RA, is well documented (Symmons, D.P. et al., J Rheumatol 25 (1998) 1072-1077) it was suggested that local inflammation in RA leads to altered blood lipid levels, thereby increasing the risk of atherosclerosis. The question whether components of the lipoprotein metabolism are causal for pathogenesis or merely affected by ongoing immune reactions during RA development cannot be answered yet. However, the observation of adverse lipid profiles in RA patients supports the validity of the presented ApoB epitope as a serum-derived RA candidate marker.

The length variant ApoB (2877-2892), but not ApoB (2877-2893), has been identified in samples of two healthy controls (Table 2). Since Apolipoprotein B constitutes 1% of all plasma proteins, the presence of ApoB epitopes in healthy control samples is not surprising. The results suggest that only the length variant ApoB (2877-2893; SEQ ID NO: 5) is specific for erosive RA.

### 26S proteasome non-ATPase regulatory subunit 8

An epitope which was quite frequently identified in mostly erosive RA samples both from serum and synovia, is derived from the regulatory subunit 8 of the 26S proteasome (PSMD8): the 15-mer PSMD8 (218-232; SEQ. ID NO: 64) with the amino acid sequence GPNNYYSFASQQQKP (Table 2). Three additional length variants were identified: the 16-mer PSMD8 (218-233; SEQ. ID NO: 65), the 17-mer PSMD8 (218-234; SEQ. ID NO: 66) and the 18-mer PSMD8 (218-234; SEQ. ID NO: 67) (Table 2). The presence of length variants supports the validity of the identified epitope as a class II MHC-derived antigenic peptide. The peptide displays a moderate binding motif with respect to *DRB1*0401* binding (TEPITOPE binding score: 3%). Binding to HLA-DR4 could be confirmed in an *in vitro* binding assay using synthetic PSMD8 (218-233) peptide and purified HLA-DR4 molecules (Fig. 3): According to its IC₅₀ value against the reporter peptide HA(308-319), PSMD8 (218-233; SEQ. ID NO: 65) binds to HLA-DR4 with a moderate affinity, confirming the TEPITOPE prediction. The 15-mer PSMD8 (218-232; SEQ. ID NO: 64) was identified once in an unpulsed control sample.

The proteasome accouts for about 1% of the cytoplasmic protein pool and is involved in ATP-dependent degradation of ubiquitinated proteins. Moreover, the proteasome is responsible for the processing of several transcription factors (i.e. nuclear factor-κB), for cell cycle control and the generation of MHC class I restricted antigens. The regulatory subunits of the proteasome are important for the selectivity of protein degradation. The non-ATPase regulatory subunit 8, in particular, is known to be necessary for the activation of the cell division control protein 28 (Cdc28), an essential cell cycle regulator in yeast.

Importantly, strongly increased levels of circulating proteasomes (cProteasomes) were detected in serum samples obtained from patients with different systemic autoimmune diseases, including RA (Egerer, K. et al., J Reumatol 29 (2002) 2045-2052). There appears to be a dose correlation between the levels of cProteasome and disease activity and concentrations of C-reactive protein in patients with severe RA. cProteasome is discussed to trigger subsequent immune responses, which would indicate an antigen-driven mechanism. The concentration of released Proteasome antigen seems to reflect the magnitude of cellular damage in autoimmune diseases. From their findings Egerer and coworkers concluded that cProteasomes could represent a novel marker for disease severity in autoimmune processes. Since the epitope PSMD8 (218-232; SEQ. ID NO: 64) was mainly identified in erosive RA samples our analysis supports this conclusion.

### Interleukin-1 receptor

Another candidate marker for erosive RA is the Interleukin-1 receptor (IL-1R) which was identified in two erosive serum samples via its peptide IL-1R (79-94; SEQ. ID NO: 68) with the amino acid sequence EKLWFVPAKVEDSGHY (Table 2). IL-1R (79-94; SEQ. ID NO: 68), with 83F as a P1 anchor, 86A as a P4 anchor and 88V as a P6 anchor, contains a strong binding motif with regard to binding to the RA susceptibility allele *DRB1*0401* (TEPITOPE binding score: 1%). In an *in vitro* binding assay the synthetic peptide was shown to bind to HLA-DR4 molecules with high affinity (Fig. 3), similar to HA(309-319) peptide.

Interleukin-1 (IL-1) is a proinflammatory cytokine and implicated in a variety of infectious immune responses as well as in RA and other inflammatory diseases (Dinarello, C., Blood 87 (1996) 2095-2147). It binds to its respective receptor which functions as a signal transducer to trigger cell proliferation or to stimulate protein synthesis. Increased IL-1 production has been observed in RA patients and plays a pivotal role in its clinical manifestations (Dayer, J.M., Rheumatology 42 (2003) ii3-ii19). IL-1 is viewed as a key mediator in RA through activation of macrophages and T- and B- lymphocytes. In addition, IL-1 contributes to inflammation by inducing the expression of cell-adhesion molecules, other cytokines and chemokines. Furthermore IL-1 is also pivotal in the destruction of bone and cartilage in RA by stimulating the production of matrix metalloproteinases. Thus, the IL-1/IL-1R complex is a first class target for anti-inflammatory therapeutics. The use of a recombinant human IL-1 receptor antagonist (IL-1RA) has been approved for the treatment of RA patients.

### Fibromodulin

Three erosive RA samples gave rise to an epitope derived from the secreted matrix protein fibromodulin (FM): the 13-mer FM (178-190; SEQ. ID NO: 70) with the amino acid sequence LRELHLDHNQISR (Table 2). Furthermore, the 14-mer FM (177-190; SEQ. ID NO: 69) was identified, which was also found once in an unpulsed control experiment. The epitope depicts a strong *DRB1*0401* binding motif with 181L serving as a P1 anchor, 184D as a P4 anchor and 186N as a P6 anchor (TEPITOPE binding score: 1%).

Fibromodulin belongs to a family of small leucine-rich proteoglycans (SLRPs) that bind to TGFβs and collagens and other extracellular matrix molecules. *In vitro,* SLRPs were shown to regulate collagen fibrillogenesis, a process essential in development, tissue repair and metastasis. To better understand the function of SLRPs *in vivo,* SLRP-deficient mice were generated and shown to develop a wide array of diseases (e.g. osteoporosis and osteoarthritis), most of them resulting primarily from an abnormal collagen fibrillogenesis (Ameye, L. & Young, M.F., Glycobiology 12 (2002) 107R-116R). Since collagen formation and degradation is highly enhanced in the inflamed joints of RA patients, an increased level of fibromodulin can be envisaged. In accord with this consideration is the fact that the identified FM epitope was primarily found in RA samples of synovial origin (3 out of 4 synovia samples).

### GM-CSF/IL-3/IL-5 receptor

In 5 out of 8 samples from erosive RA patients an epitope from the β-chain of the multiple cytokine receptor CYRB could be identified: the 15-mer CYRB (359-373; SEQ. ID NO: 71) with the amino acid sequence ETMKMRYEHIDHTFE and its length variant, the 17-mer CYRB (359-375; SEQ. ID NO: 72) (Table 2). In an *in vitro* binding assay the synthetic CYRB (359-375) peptide was shown to bind to HLA-DR4 molecules with moderate affinity (Fig. 3). This is in agreement with TEPITOPE which predicted a moderate peptide binding motif with respect to DRB1*0401 binding (TEPITOPE binding score: 3%). The epitope was clearly overrepresented in the RA samples since it was identified in the serum of only one healthy test person.

The GM-CSF/IL-3/IL-5 receptor is a type I membrane protein and differentially expressed throughout the hematopoietic system (reviewed by Geijsen, N. et al., Cytokine Growth Factor Rev 12 (2001) 19-25). Its ligands, IL-3 and GM-CSF, are secreted by CD4+ T cells and important stimuli for the formation of dendritic cells originating from progenitor cells in the bone marrow. The critical role of dendritic cells in the initiation and development of an immune response suggests that they play a key role in the development of autoimmune inflammatory disorders, such as RA, by transporting autoantigen to the draining lymph node where DCs encounter and prime naive T cells. The activity of GM-CSF has been linked to proinflammatory effects in RA supporting the identified receptor epitope CYRB (359-373; SEQ. ID NO: 71) as a putative candidate peptide marker for the diagnosis of RA.

### Sorting nexin 3

Another epitope which appears to be indicative for erosive RA is derived from a protein termed sorting nexin 3 (SNX3): the 16-mer SNX3 (142-157; SEQ. ID NO: 73) having the amino acid sequence HMFLQDEIIDKSYTPS (Table 2). In this epitope 144F, 147D and 1491 could serve as P1, P4 and P6 anchors, respectively, in the peptide-binding groove of the RA susceptibility allele *DRB1*0401* (TEPITOPE binding score: 2%).

Sorting nexins are a diverse group of cellular trafficking proteins that share as common phospholipid-binding motif (reviewed by Worby, C.A. & Dixon, J.E., Nat Rev Mol Cell Biol 3 (2002) 919-31). The ability of these proteins to bind specific phospholipids, as well as their propensity to form protein-protein complexes, points to the involvement of these proteins in membrane trafficking and protein sorting. Sorting nexin 3 in particular is present in the cytosol and in endosomes and appears to be involved in membrane trafficking from early to recycling endosomes. Whether sorting nexin 3 plays a role in RA, e.g. by influencing antigen presentation routes, remains unknown at the moment. The epitope SNX3 (142-157; SEQ. ID NO: 73) identified in this study suggests a link between sorting nexins and autoimmunity.

### Example 3

All peptide sequences identified in examples 1 and 2 from non-erosive and erosive RA samples were used in this example to search for common markers relevant for both RA types. The RA-specific sequences were again compared with peptide sequences of the control samples (unpulsed DCs and DCs pulsed with the serum of two healthy test persons) and only those peptides were selected for further evaluation that re-occurred in at least three of altogether fifteen RA samples (erosive and non-erosive RA).

### Inter-alpha-trypsin inhibitor

Ten out of eleven serum samples (erosive & non-erosive RA) gave rise to an epitope derived from the heavy chain H4 of the inter-alpha-trypsin inhibitor: ITIH4 (271-287) with the amino acid sequence of SEQ ID NO: 8 (Table 3). Apart from this major length variant of the ITIH4 epitope, six length variants of the same ITIH4 epitope could be identified (Table 3): the 19-mer ITIH4 (271-289; SEQ ID NO: 6), the 18-mer ITIH4 (271-288; SEQ ID NO: 7), the 16-mer ITIH4 (274-289; SEQ ID NO: 12), the 15-mer ITIH4 (273-287; SEQ ID NO: 10), the 15-mer (274-288; SEQ ID NO: 11) and the 14-mer ITIH4 (274-287; SEQ ID NO: 9).

As judged from the shortest length variant, ITIH4 (274-287), the epitope contains a very strong binding motif, with regard to binding to the RA susceptibility allele *DRB1*0401:* 277F serves as a P1 anchor, 280D as a P4 anchor and 282S as a P6 anchor (binding score: 1%).

ITIH4 belongs to the Inter-alpha-inhibitor (IαI) family which is a group of serum protease inhibitors that bind to hyaluronic acid (HA) and appear to be involved in acute-phase reactions (Salier, J.P. et al., Biochemical Journal 315 (1996) 1-9).

HA is a polysaccharide found in all tissues of the body, in particular, in loose connective tissue, e.g. joint fluid (Evered, D. & Whelan, J. eds., The Biology of Hyaluronan, John Wiley & Sons (1989)). HA has an important structural function in cartilage and other tissues where it stabilizes the extracellular matrix by forming aggregates with proteoglycans. It has also been assigned important biological functions by regulating cellular activities via binding to cell surface proteins, such as CD44 and ICAM-1 (Knudson, C.B. & Knudson, W., FASEB J 7 (1993) 1233-1241; Hall, C.L. et al., J Cell Biol 126 (1994) 575-588). RA is accompanied by a large increase in total HA in the joint fluid as well as in the serum, suggesting that circulating HA originates from rheumatoid joints (Engström-Laurent, A. et al., Scand J Clin Lab Invest 45 (1985) 497-504).

Complexes of HA and some IαI family members were observed in large amounts in the synovial fluid of RA patients (Jessen, T.E. et al., Biological Chemistry Hoppe-Seyler 375 (1994) 521-526). The role of the IαI-HA complex in inflammatory reactions might be to modify the CD44-HA interaction that mediates leukocyte activation and invasion (Isacke, C.M. & Yarwood, H., Int J Biochem Cell Biol 34 (2002) 718-721). Additionally, synovial fluid of RA patients contains elevated levels of TSG-6, an anti-inflammatory glycoprotein and a member of the hyaladherin family of HA-binding proteins (Wisniewski, H.G. et al., J Immunol 151 (1993) 6593-6601). It has been shown that a complex of TSG-6 with IαI family members inhibits the activity of plasmin, a central molecule in the activation of inflammation-associated enzymes (Wisniewski, H.G. et al., J Immunol 156 (1996) 1609-1615). A regulation of plasmin activity by several acute-phase plasma proteins, namely TSG-6 and IαI family members, may prove to be important in RA, given the high contents of HA, TSG-6 and IαI family members in synovial fluid of inflamed joints.

This evidence, together with the identification of multiple length variants of the same epitope and a strong HLA-DR4 binding motif, convincingly support the validity of the presented ITIH4 epitope as a serum-derived RA candidate marker.

### Complement C4

In eight out of eleven RA sera (erosive and non-erosive) tested, another dominant epitope was identified which is derived from complement C4: the 15-mer C4 (1697-1711) with the amino acid sequence of SEQ ID NO: 13 (Table 3). Five additional length variants of the same epitope could be found (Table 3): the 12-mer C4 (1697-1708; SEQ ID NO: 18), the 13-mer C4 (1698-1710; SEQ ID NO: 17), the 14-mer C4 (1697-1710; SEQ ID NO: 15), the 16-mer C4 (1697-1712; SEQ ID NO: 14) and the 18-mer C4 (1697-1714; SEQ ID NO: 16). Moreover, the presented epitope displays a very strong *DRB1*0401* binding motif: 1700Y as P1 anchor, 1704D as P2 anchor and 1706N as P6 anchor (binding score: 1%).

C4 which constitutes approximately 0.5% of plasma protein mass plays a critical role in the triggering of the central pathway of the complement system. The protein is synthesized as a single-chain precursor and, prior to secretion, is enzymatically cleaved to form a trimer of non-identical α-, β-, and γ-chains. The identified epitope C4 (1697-1711) is located at the very C-terminus of the C4 γ-chain. The C4 α-chain is further proteolytically degraded by activated C1 to form the C4a anaphylatoxin, which is a mediator of local inflammatory processes (Moon, K.E. et al., J Biol Chem 256 (1981) 8685-8692).

In general, the complement cascade is involved in the induction and progression of inflammatory reactions and is a major defense system against various pathogenic agents, including bacteria, viruses and other antigens (Morgan, B.P., Methods Mol Biol 150 (2000) 1-13). Inappropriate activation, however, can lead to tissue damage and manifestation of disease (Speth, C. et al., Wien Klin Wochenschr 111 (1999) 378-391).

Activation of the complement system has been repeatedly implicated in the pathogenesis of RA, based on studies showing increased levels of complement metabolites, including C4 and C4a, in plasma, synovial fluid and synovial tissue of RA patients (Neumann, E. et al., Arthritis Rheum 46 (2002) 934-945). In addition collagen-induced arthritis (CIA) in mice is characterized by the presence of complement activation products (Linton, S.M. & Morgan, B.P., Mol Immunol 36 (1999) 905-914). CIA is prevented after treatment with anti-C5 monoclonal antibodies (Wang, Y. et al., PNAS 92 (1995) 8955-8959) or with soluble CR1, an inhibitor of the complement system, delivered by gene therapy (Dreja, H. et al., Arthritis Rheum 43 (2000) 1698-1709). Activation of complement factors in joints is possibly induced by the presence of various immune complexes and it was hypothesized that stimulation of the innate immune system by infectious agents and cytokines may contribute to the initiation of RA (Friese, M.A. et al., Clin Exp Immunol 121 (2000) 406-414).

Two of the six presented C4 epitopes, the 15- and the 18-mer, were also identified in healthy control samples (Table 3) indicating that only some length variants of this C4 epitope are RA-specific, namely the antigenic peptides of SEQ ID NOs: 14,15,17, and 18.

### Complement C3

Another epitope that was found in erosive and non-erosive RA samples is derived from complement C3 (alpha-chain): the 14-mer C3 (1431-1444) with the amino acid sequence of SEQ ID NO: 21 (Table 3). Six additional length variants of the same epitope were identified in serum (Table 3): the 13-mer C3 (1431-1443; SEQ ID NO: 23), the 14-mer C3 (1429-1442; SEQ ID NO: 74), the 15-mer C3 (1431-1445; SEQ ID NO: 22), the 15-mer C3 (1429-1443; SEQ ID NO: 20), the 17-mer C3 (1427-1443; SEQ ID NO:75) and the 19-mer C3 (1426-1444; SEQ ID NO: 19). As judged from the shortest length variant, C3 (1431-1443), a DRB1*0401 binding motif can be postulated: 1434Y serves as a P1 anchor, 1437D as a P4 anchor and 1439A as a P6 anchor.

In erovise and non-erosive RA samples a further epitope was found, which is derived from complement C3 (beta-chain): the 19 mer C3 (157-175) with the amino acid sequence of SEQ. ID NO: 76 (Table 3). One additional length variant of the same epitope was identified in serum (Table 3): 20-mer C3 (157-176; SEQ. ID NO: 77)

Complement C3 which constitutes about 1-2% of plasma protein mass plays a central role in the activation of the complement system and belongs to the family of the acute-phase proteins. Its processing by C3 convertase to C3a anaphylatoxin and C3b is the central step in both the classical and alternative complement pathways (Barrington, R. et al., Immunol Rev 180 (2001) 5-15). After activation, C3b can bind covalently, via a reactive thiolester, to cell surface carbohydrates or immune aggregates (Isaac, L. & Isenman, D.E., J Biol Chem 267 (1992) 10062-10069). The identified epitope C3 (1431-1444) is located at the C-terminus of C3b.

As already discussed in the context of complement epitope C4 (1697-1711), there is increasing evidence for an important role of components of the complement cascade in the pathophysiology of RA. The result of this study, in which two major epitopes derived from complement C3 and C4 were identified in serum of RA patients, underlines the close link between the activated complement system and pathogenesis of RA. This coincidence makes a strong argument for the validity of the presented C3 / C4 epitopes as serum-derived candidate RA markers.

### SH3 domain-binding glutamic acid-rich-like protein 3

Another epitope which was elucidated quite frequently in serum of RA patients (six out of eleven erosive and non-erosive RA samples), is derived from the SH3 domain-binding glutamic acid-rich-like protein 3 (SH3BGRL3): SH3BGRL3 (15-26) with the amino acid sequence of SEQ ID NO: 25 (Table 3). Three length variants of the same epitope were identified (Table 3): the 14-mer SH3BGRL3 (13-26; SEQ ID NO: 26), the 14-mer SH3BGRL3 (15-28; SEQ ID NO: 27) and the 16-mer SH3BGRL3 (13-28; SEQ ID NO: 24). The *Drub1*0401* binding motif is: 171 as P1 anchor, 20Q as P4 anchor and 22S as P6 anchor (binding score 4%).

SH3BGRL3 is a small 10 kD protein that belongs to the SH3BGR family. The precise function of the protein is unknown but a role as a modulator of glutaredoxin biological activity is postulated (Mazzocco, M. et al., Biochem Biophys Res Commun 285 (2001) 540-545). So far, SH3BGRL3 has not been described in the context of RA.

Interestingly, the analysis elucidated a second epitope of the same protein, which was highly abundant in all RA and control samples: the 16-mer SH3BGRL3 (29-44) with the amino acid sequence DGKRIQYQLVDISQDN. In addition multiple length variants of the same epitope were found in most samples as well. As judged from the shortest length variant, SH3BGRL3 (31-42), the epitope contains almost similar *DRB1*0401* anchor residues compared with SH3BGRL3 (15-26): 331 serves as a P1 anchor, 36Q as a P4 anchor and 38V as a P6 anchor (binding score -2). This similarity is reflected by comparable binding scores.

The presence of this second SH3BGRL3 epitope supports the validity of the SH3BGRL3 (15-26) epitope because both peptides are derived from the same protein, however, only one of them, epitope SH3BGRL3 (15-26), appears to be generated in a RA-specific manner. A similar observation has been described already for GILT in example 1.

Among the four SH3BGRL3 length variants the longest variant, SH3BGRL3 (13-28), was also identified in a healthy control sample (Table 3). However, this particular length variant was found only one time, which indicates a significant enrichment of the SH3BGRL3 epitope in the context of RA.

### Interleukin-4 (IL-4) induced protein 1

In all the investigated synovial fluids (erosive & non-erosive RA) and in eight out of eleven sera (erosive & non-erosive RA), one highly dominant epitope was identified which is derived from the human homolog of the IL-4 induced protein 1 (Fig1): Fig1 (293-309) with the amino acid sequence of SEQ ID NO: 28 (Table 3). The validity of the epitope was further supported by the presence of additional length variants in several samples (Table 3): the 16-mer Fig1 (293-308; SEQ ID NO: 30) and the 19-mer Fig1 (293-311; SEQ ID NO: 29). Moreover, the amino acid sequence displays a typical *DRB1*0401* binding motif. 299V serves as P1 anchor, 302E as P4 anchor and 304S as P6 anchor (binding score 1%).

Two length variants of the same epitope, Fig1 (293-308) and Fig1 (293-309), were identified in one unpulsed sample and in one healthy control sample as well (Table 3). However, the presence of the Fig1 epitope in almost all RA samples but not in all of the control samples tested strongly indicates an enrichment in the context of RA.

The human *fig1* gene was first identified in IL-4-stimulated B cell cultures (Chu, C.C. & Paul, W.E., PNAS 94 (1997) 2507-2512). The human *fig1* resides on chromosome 19q13.3-19q13.4, a region previously identified to be involved in susceptibility to autoimmune diseases, including SLE, arthritis, multiple sclerosis, and insulin-dependent diabetes mellitus (Becker K.G. et al., PNAS 95 (1998) 9979-9984). Since its expression is largely limited to immune tissues and its regulation is dependent on IL-4, a key modulator of the immune response, *fig1* is thus an attractive candidate gene for autoimmune disease susceptibility (Chavan, S.S. et al., Biochim Biophys Acta 1576 (2002) 70-80). The HLA-DR4-restricted presentation of a Fig1 epitope provides the first indication that Fig1 protein is produced and possibly involved in the disease development of RA. The Fig1 polypeptide has not been known as a marker for RA until now, and is considered as an important candidate marker for RA.

### Hemopexin

Another RA candidate marker which was frequently identified in serum samples (ten out of eleven samples) and in synovia samples (two out of four samples) (erosive & non-erosive RA) is derived from hemopexin (HPX): HPX (351-367) with the amino acid sequence of SEQ ID NO: 32 (Table 3). Several length variants were found which support the validity of this epitope (Table 3): the 13-mer HPX (351-363; SEQ ID NO: 33), the 14-mer HPX (350-363; SEQ ID NO: 34), the 15-mer HPX (351-365; SEQ ID NO: 35), the 18-mer HPX (351-368; SEQ ID NO: 31) and the 18-mer HPX (350-367; SEQ ID NO: 78). Furthermore, the epitope contains a very strong *DRB1*0401* binding motif. 355I serves as a P1 anchor, 358D as a P4 anchor and 360V as a P6 anchor (binding score: 1%).

Two length variants of the same epitope, HPX (351-367; SEQ ID NO: 32) and HPX (351-365; SEQ ID NO: 35), could also be identified in healthy control samples (Table 3) indicating that only some length variants are specific for RA, namely the antigenic peptides of SEQ ID NOs. 31, 33, 34 and 78.

HPX is a 60 kD plasma glycoprotein with a high binding affinity to heme (Muller-Eberhard, U., Methods Enzymol 163 (1988) 536-565). It is mainly expressed in the liver, and belongs to the acute-phase proteins the synthesis of which is induced in an inflammatory situation. RA is a chronic inflammatory autoimmune disease and elevated levels of several acute-phase proteins, including C-reactive protein and serum amyloid A, have been reported (Nakamura, R., J Clin Lab Anal 14 (2000) 305-313). HPX is responsive to the cytokines IL-1 and IL-6, which are upregulated in patients suffering from RA (Feldmann, M. & Maini, R.N., Rheumatology 38, Suppl 2 (1999) 3-7).

HPX is the major vehicle for the transportation of heme in the plasma and its principal role is to prevent heme-mediated oxidative stress and loss of heme-bound iron (Tolosano, E. & Altruda, F., DNA Cell Biol 21 (2002) 297-306). It can protect cells against oxidative stress by inducing the expression of intracellular antioxidants such as heme oxygenase, metallothioneins and ferritin. Metallothioneins are cytosolic proteins that are expressed particularly in synovial fibroblasts (Backman, J.T. et al., Virchows Arch 433 (1998) 153-160). There is significant experimental evidence for the presence of oxidative stress in the synovial tissue of RA patients (reviewed in: Schett, G. et al., Arthritis Res 3 (2000) 80-86). Furthermore HPX was reported to promote proliferation of human T lymphocytes (Smith, A. et al., Exp Cell Res 232 (1997) 246-254). These studies render it likely that HPX belongs to the up-regulated proteins in serum and synovia of RA patients, thereby providing a rationale for the relevance of HPX (351-367) as a RA-specific candidate marker.

### Hsc70-interacting protein

An epitope which was mostly identified in serum samples (4 out of eleven erosive and non-erosive RA samples) and which is also related to stress responses is derived from the Hsc70-interacting protein Hip: Hip (83-98) with the amino acid sequence of SEQ ID NO: 38 (Table 3). Two length variants of this epitope were identified (Table 3): the 18-mer Hip (83-100; SEQ ID NO: 36) and the 15-mer Hip (84-98; SEQ ID NO: 39). An additional length variant was discovered in one erosive synovia sample (Table 3): the 15-mer Hip (85-99; SEQ ID NO: 37). As judged from the shortest length variant Hip (84-98) a *DRB1*0401* binding motif attaining a moderate score of 8%, can be postulated: 891 as P1 anchor, 92D as P4 anchor, 94D as P6 anchor.

In the cytosol of eukaryotic cells, Hip and Hop proteins associate with Hsc70 in order to participate in the regulation of Hsc70 chaperone activity (Frydman, J. & Höhfeld, J., Trends Biochem Sci 22 (1997) 87-92). The 42 kD Hip protein binds to the ATPase domain of Hsc70. It was postulated that Hip might increase the half-life of the chaperone-substrate complex providing the molecular basis for an efficient cooperation of Hsc70 with downstream chaperone systems. Hsc70 and Hsp90 have been shown to cooperate during protein *folding in vitro* (Jakob, U. & Buchner, J., Trends Biochem Sci 19 (1994) 205-211; Freeman, B.C. & Morimoto, R.I., EMBO J 15 (1996) 2969-2979) and to play a role in thermal denaturation (Schneider, C. et al., PNAS 93 (1996) 14536-14541). The Hsc70 and . Hsp90 association with stress-adaptation ultimately links Hip to stress responses, including the induction of heat shock proteins, in the synovial tissue of RA patients (reviewed in: Schett, G. et al., Arthritis Res 3 (2001) 80-86).

### Binding properties of ITIH4, C4, C3, SH3BGRL3, Fig1, HPX and Hip

In order to further investigate the binding properties of the above described antigenic peptides ofITIH4, C4, C3, SH3BGRL3, Fig1, HPX and Hip to HLA-DR4 molecules, *in vitro* binding assays were performed (Fig. 3): According to their IC₅₀ values against the reporter peptide HA (309-317), the synthetic peptides ITTH4 (274-287), SH3BGRL3 (13-26), and Fig1 (293-309) bind to HLA-DR4 with high affinity (Fig. 3). Moderate binding to HLA-DR4 was measured for the synthetic peptides C4 (1696-1709) and HPX (351-365). The synthetic peptide C3 (1431-1444) bound only weakly to HLA-DR4 which is in agreement with TEPITOPE scoring (9%). No binding was measured for the Hip (84-98) peptide (data not shown).

### Invariant chain (Ii)

Among the candidate marker peptides that appear to be indicative for erosive and non-erosive RA is an epitope which is derived from the HLA-DR-associated invariant chain (Ii): the 15-mer Ii (110-124; SEQ. ID NO: 83) with the amino acid sequence ATPLLMQALPMGALP (Table 3). Moreover, four length variants were identified: the 16-mer Ii (109-124; SEQ. ID NO: 81), the 17-mer Ii (109-125; SEQ. ID NO: 80), the 18-mer Ii (109-126; SEQ. ID NO: 79) and the 21-mer Ii (109-129; SEQ. ID NO: 82) (Table 3). The presence of length variants gives confidence in the identified peptide sequence which displays a moderate *DRB1*0401* binding motif (TEPITOPE binding score: 5%) with 114L serving as a P1 anchor, 117A as a P4 anchor and 119P as a P6 anchor. The epitope was identified in two erosive and three non-erosive RA samples. Two Ii length variants were found in one unpulsed sample and two samples of healthy test persons.

Peptide loading of MHC class II molecules is regulated by two accessory molecules in the class II pathway, invariant chain Ii and HLA-DM (reviewed by Bakke, O. & Nordeng T.W., Immunol Rev 172 (1999) 171-187 and Kropshofer, H. et al., Immunol Today 18 (1997) 77-82). Ii trimers bind to nascent MHC class II molecules in the rough endoplasmic reticulum (ER) and block the peptide-binding groove, stabilizing the class II molecule and preventing the binding of ligands available in the ER. Class II/Ii complexes are transported via the Golgi apparatus to endosomes, where Ii is degraded to a nested set of class II-associated Ii peptides (CLIP). The release of CLIP enables endosomal peptides to bind to the MHC class II molecules which are presented to T cell receptors on the cell surface. The identified epitope Ii (110-124) overlaps with the C-terminal part of CLIP. Interestingly, a reduced interaction of CLIP with RA-associated HLA-DR alleles was described (Patil, N.S. et al., J Immunol 167 (2001) 7157-7168), indicating that a reduced interaction may contribute to the pathophysiology of autoimmunity in RA.

### Retinoic acid receptor responder protein 2

In 4 out of 11 serum samples (erosive & non-erosive RA) an epitope was identified which is derived from retinoic acid receptor responder protein 2 (RARRES2): the 22-mer RARRES2 (40-61; SEQ. ID- NO: 86) with the amino acid sequence HPPVQWAFQETSVESAVDTPFP (Table 3). In the C-terminal part of the epitope two length variants could have been identified: the 23-mer RARRES2 (40-62; SEQ. ID NO: 84) and the 24-mer RARRES2 (40-63; SEQ. ID NO: 85) (Table 3). With 45W as P1 anchor, 48Q as P4 anchor and 50T as P6 anchor, the epitope displays a moderate binding motif in the context of *DRB1*0401* (TEPITOPE binding score: 3%).

RARRES2 is a small 18.6 kD protein and mostly expressed in the endothelium and epidermis. The expression appears to be hormone dependent and a response to retinoic acid in skin and some osteotrophic hormones in marrow-derived stromal cells was identified (Nagpal, S. et al., J Invest Dermatol 109 (1997) 91-95 and Adams, A.E. et al., J Cell Biochem 74 (1999) 587-595). The function of RARRES2 is largely unknown and an association with RA, for instance through impaired osteoclastogenesis, is imaginable.

### Fibronectin

Another HLA-DR4 associated peptide which was exclusively found in 3 out of 4 synovia samples (erosive & non-erosive RA) is derived from fibronectin (Fn), a major glycoprotein in blood plasma and in the extracellular matrix: the 15-mer Fn (1881-1895; SEQ. ID NO: 90) with the amino acid sequence IYLYTLNDNARSSPV (Table 3). The epitope appears to be a very good *DRB1*0401* binder with 1885Y serving as a hydrophobic P1 anchor, 1888N as a P4 anchor and 1890N as a P6 anchor (TEPITOPE binding score: 1%). Four length variants were additionally identified: the 16-mer Fn (1881-1896; SEQ. ID NO: 91), the 16-mer Fn (1880-1895; SEQ. ID NO: 88), the 17-mer Fn (1881-1897; SEQ. ID NO: 89) and the 17-mer Fn (1880-1896; SEQ. ID NO: 87), strongly supporting the validity of the identified Fn epitope (Table 3).

Fibronectin plays an important role in cell adhesion, cell motility and in opsonization. It binds collagen and fibrin and mediates adhesion of fibroblasts to collagen fibrils. Fibronectin is strongly expressed in the synovial lining layer of both RA and osteoarthritis patients and correlates with hyperplasia of diseased joints. The identification of epitope Fn (1881-1895) in only samples of synovial origin is in line with these findings and points to a putative role of highly abundant fibronectin in autoimmunity.

### Cathepsin B

Three out of fifteen RA samples (erosive & non-erosive RA) gave rise to an epitope which originates from Cathepsin B (CatB): the 15-mer CatB (227-241; SEQ. ID NO: 92) with the amino acid sequence YNSYSVSNSEKDIMA (Table 3). The peptide displays a moderate *DRB1*0401* binding motif with 230Y as a hydrophobic P1 anchor and the serine residues at positions 233 and 235 as putative P4 and P6 anchors (TEPITOPE binding score: 6%).

Cathepsin B is a thiol peptidase and believed to participate in intracellular degradation and turnover of proteins, e.g. collagen. It is located in lysosomes of different cell types including leukocytes. In the context of inflammation and disease it was shown that Cathepsin B contributes to cartilage destruction in osteoarthritis and pathological proteolysis in RA and cancer (Cunnane, G. et al., Arthritis Rheum 44 (2001) 1744-1753). Enzyme activities of Cathepsin B and other lysosomal peptidases correlate with RA progression which supports the validity of epitope CatB (227-241) as a putative RA marker peptide (Sohar, N. et al., Biol Chem 383 (2002) 865-869).

### Tripeptidyl-peptidase II

Another peptidase-derived epitope which was found in three out of fifteen RA samples (erosive & non-erosive RA) is derived from tripeptidyl-peptidase II (TPP2): the 15-mer TPP2 (970-984; SEQ. ID NO: 93) having the amino acid sequence AGSLTLSKTELGKKA (Table 3). Additionally, the length variant TPP2 (970-985; SEQ. ID NO: 94) was identified. The peptidase epitope contains a typical *DRB1*0401* binding motif with 973L, 976S and 978T as P1, P4 and P6 anchors respectively (TEPITOPE binding score: 3%).

Similar to Cathepsin B, tripeptidyl-peptidase II is involved in lysosomal protein degradation. The identification of epitope TPP2 (970-984) in the context of RA provides the first indication that TPP2 might be implicated in impaired protein degradation in the context of inflammation and RA.

### Legumain

Legumain (LGMN) completes the set of three peptidases which were found in this investigation. The identified epitope LGMN (99-112; SEQ. ID NO: 95) with the amino acid sequence VPKDYTGEDVTPQN (Table 3) displays a typical binding motif with respect to the HLA allele *DRB1*0401* in which 103Y could serve as a P1 anchor, 106E as a P4 anchor and 108V as a P6 anchor (TEPITOPE binding score: 1%).

Legumain occurs in endosomal and lysosomal fractions of antigen-presenting cells such as dendritic cells (Schwarz, G. et al., Biol Chem 383 (2002) 1813-1816). It has a strict specificity for the hydrolysis of asparaginyl bonds and was shown to play an important role in the processing of bacterial antigens for MHC class II presentation (Manoury, B. et al., Nature 396 (1998) 695-699). Whether Legumain is involved in autoimmune diseases, such as RA, remains unknown but the fact that three lysosomal peptidases together with GILT and the 26S proteasome were identified in this investigation suggests that the protein degradation machinery facilitating antigen processing might be significantly altered in the context of RA.

### Platelet activating factor receptor

An epitope which was identified in three out of eleven serum RA samples (erosive & non-erosive RA) is derived from a receptor for platelet activating factor (PAFR): the 13-mer PAFR (264-276; SEQ. ID NO: 96) with the amino acid sequence DSKFHQAINDAHQ (Table 3). According to TEPITOPE scoring (3%) the epitope contains a moderated *DRB1*0401* binding motif with 267F as P1 anchor, 270A as P4 anchor and 272N as P6 anchor (Table 3).

Platelet activating factor (PAF) is a pro-inflammatory lipid mediator which binds to a G-protein-coupled seven transmembrane receptor on the surface of a broad range of cell types. By receptor binding, PAF transduces pleiotrophic functions which include cell motility, smooth muscle contraction, and synthesis and release of cytokines (reviewed by Honda, Z. et al., J Biochem 131 (2002) 773-779). Pharmacological studies and the establishment of PAFR (-/-) mice have suggested that PAF functions in a variety of settings including allergy, inflammation, neural functions, reproduction, and atherosclerosis. Interestingly, PAF was found in elevated levels in the synovial fluid of RA patients and shown to induce neoangiogenesis, which is frequently observed in rheumatoid synovitis (Lupia, E. et al., Eur J Immunol 26 (1996) 1690-1694). This type of evidence gives further confidence in epitope PAFR (264-276) being a RA-associated marker peptide.

### Poly-alpha-2,8-sialyltransferase

Another epitope which was found in three out of eleven serum samples (erosive & non-erosive RA) originates from Polysialyltransferase (PST): the 15-mer PST (333-347; SEQ. ID NO: 97), MPLEFKTLNVLHNRG (Table 3), displays a moderate binding motif with respect to *DRB1***0401* binding (TEPITOPE scoring: 2%). 337F could serve as a P1 anchor, 340L as a P4 anchor and 342V as a P6 anchor.

Polysialic acid is a carbohydrate composed of a linear homopolymer of α-2,8-linked sialic acid residues. The glycan is mainly attached to the neural cell adhesion molecule (N-CAM) and implicated in many morphogenic processes of the neural cells by modulating the adhesive property of N-CAM. The membrane protein polysialyltransferase catalyzes the polycondensation of sialic acid residues and is highly expressed in fetal brain, lung and kidney, in adult heart, spleen and thymus, and to a lesser extent in peripheral blood leukocytes (Nakayama, J. et al., Proc Natl Acad Sci 92 (1995) 7031-7035). Elevated PST levels were observed in serum of patients with metastatic tumors and an increased enzyme activity was also associated with rheumatoid arthritis (Berge, P.G. et al., Klin Wochenschr 60 (1982) 445-449). The identification of epitope PST (333-347) in several RA samples is in line with these observations and supports the putative role of PST in RA.

### Ras-related protein Rab-11B

The last epitope presented in this analysis of RA samples is derived from the Ras-related protein Rab-11B: the 13-mer Rab-11B (51-63; SEQ. ID NO: 102) with the amino acid sequence RSIQVDGKTIKAQ (Table 3, the peptide sequence were validated with regard to binding to the RA susceptibility allele Drub1*0301 by using the TEPITOPE software (Hammer, J. et al., Adv Immunol 66 (1997) 67-100)). This epitope and four additional lenght variants, the 14-mer Rab-11B (50-63; SEQ. ID NO: 100), the 15-mer Rab-11B (49-63; SEQ. ID NO: 98), the 17-mer Rab-11B (49-65; SEQ. ID NO: 101) and the 18-mer Rab-11B (49-66; SEQ. ID NO: 99) were found in three erosive and two non-erosive RA samples (Table 3). The 15-mer Rab-11B (49-63) was identified in 1 out of 12 control samples.

.Rab proteins are small GTPases that play an important role in membrane trafficking along the endo- and exocytic pathway. Rab-11B is assumed to be essential for the transport of internalized transferrin from the recycling compartment to the plasma membrane. Furthermore Rab-11B was identified in rat osteoclasts and might play an additional role in bone resorption. The epitope Rab-11B (51-63) provides the first indication of a role of Rab-11B in RA development.

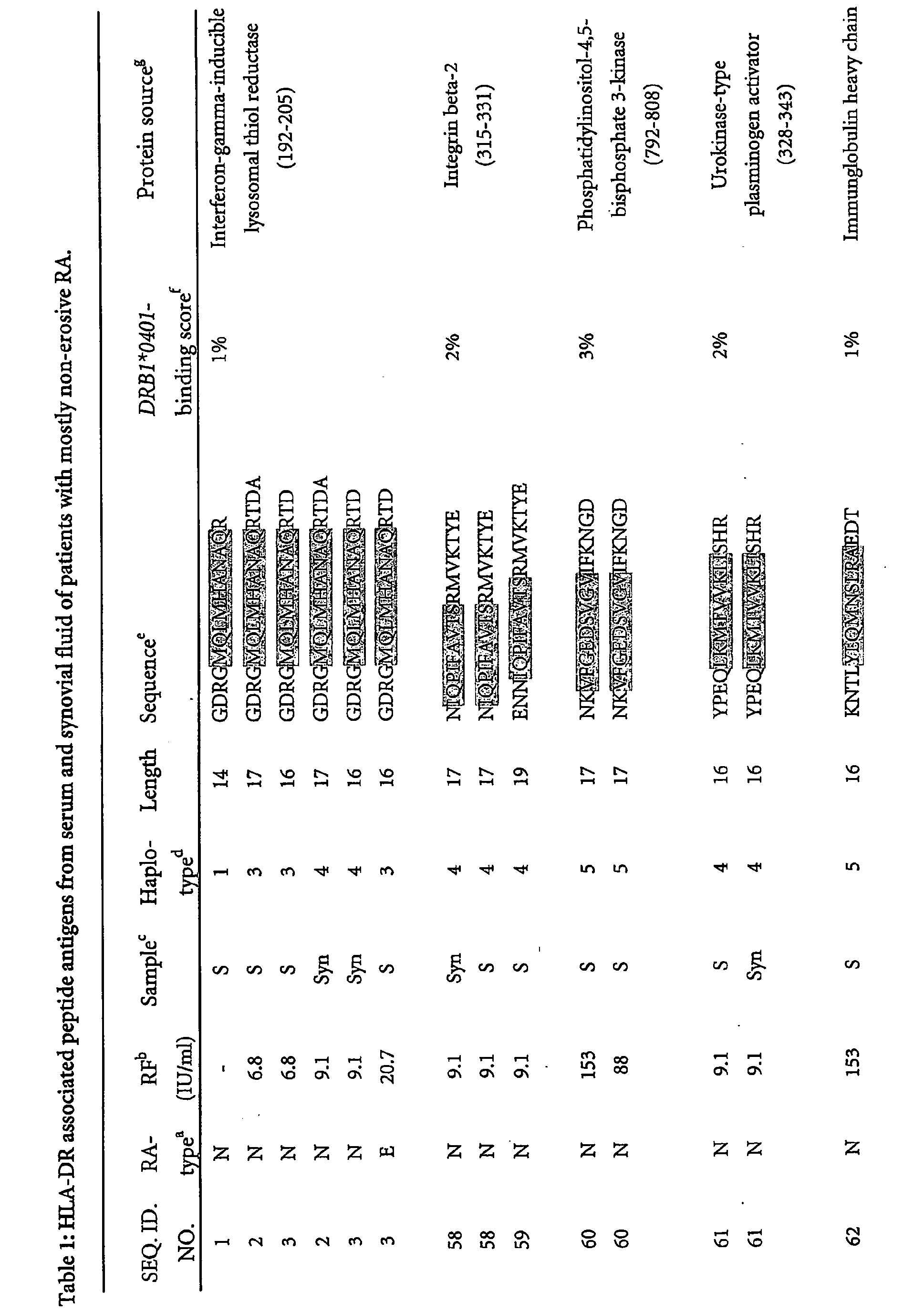

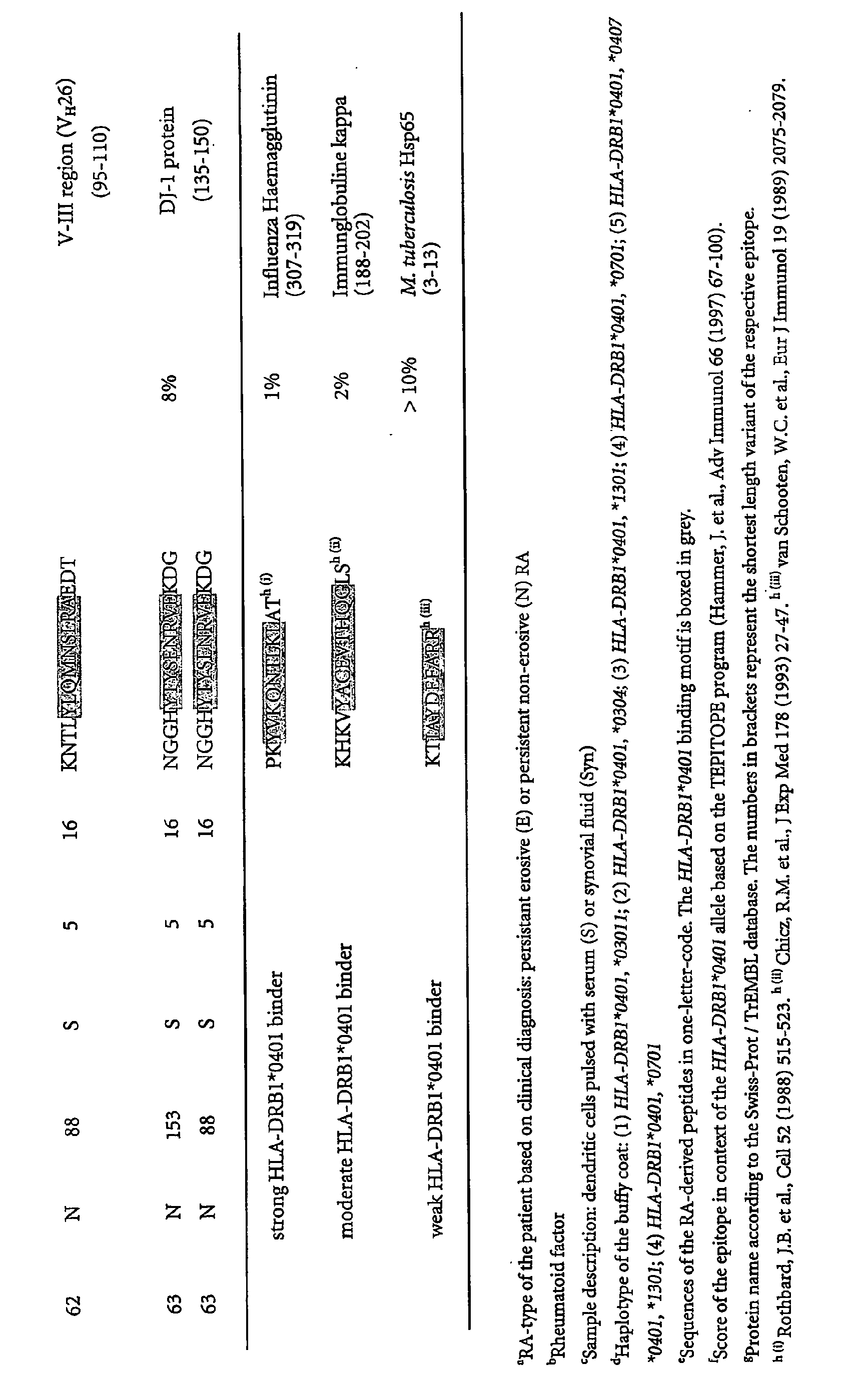

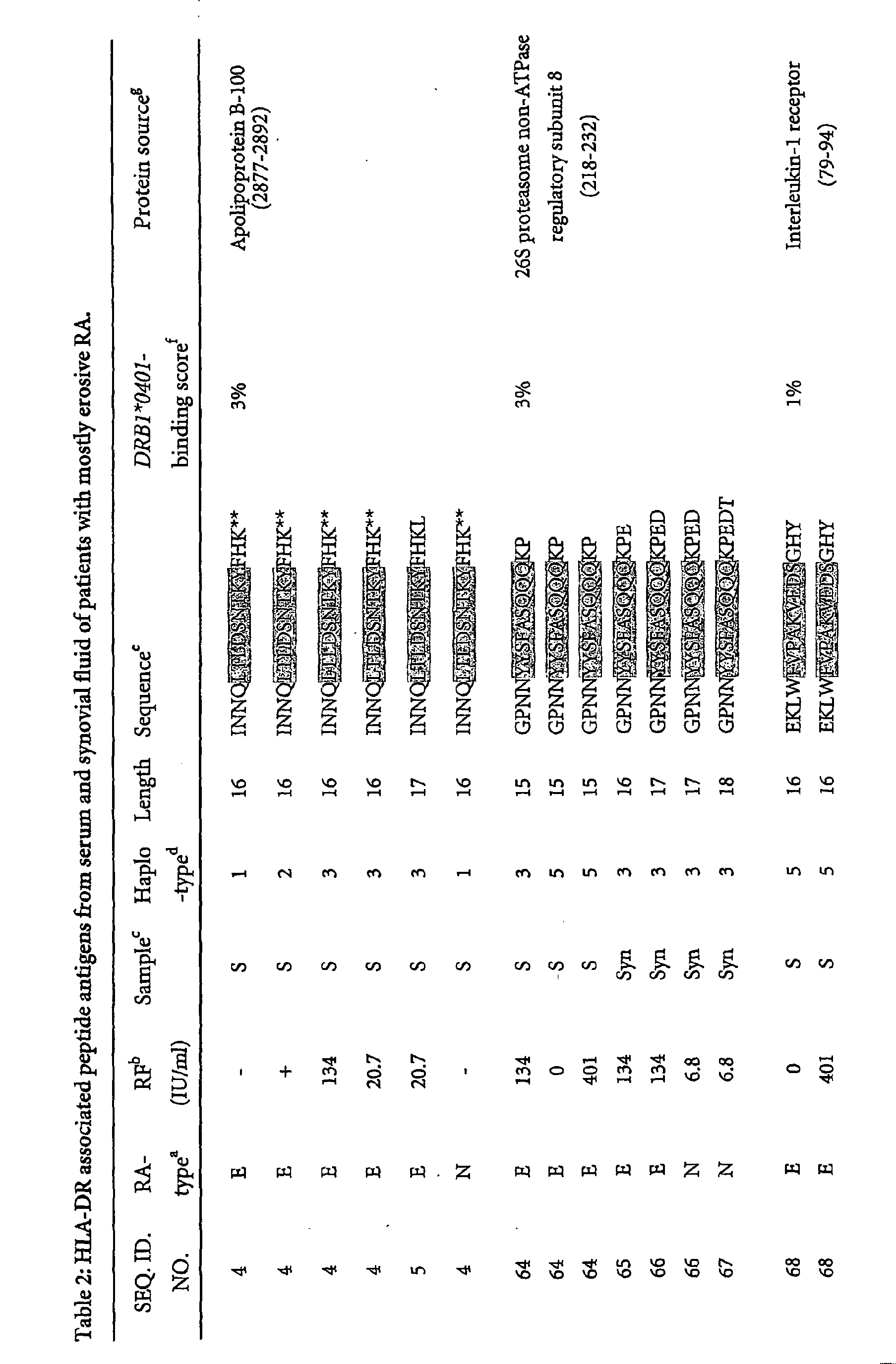

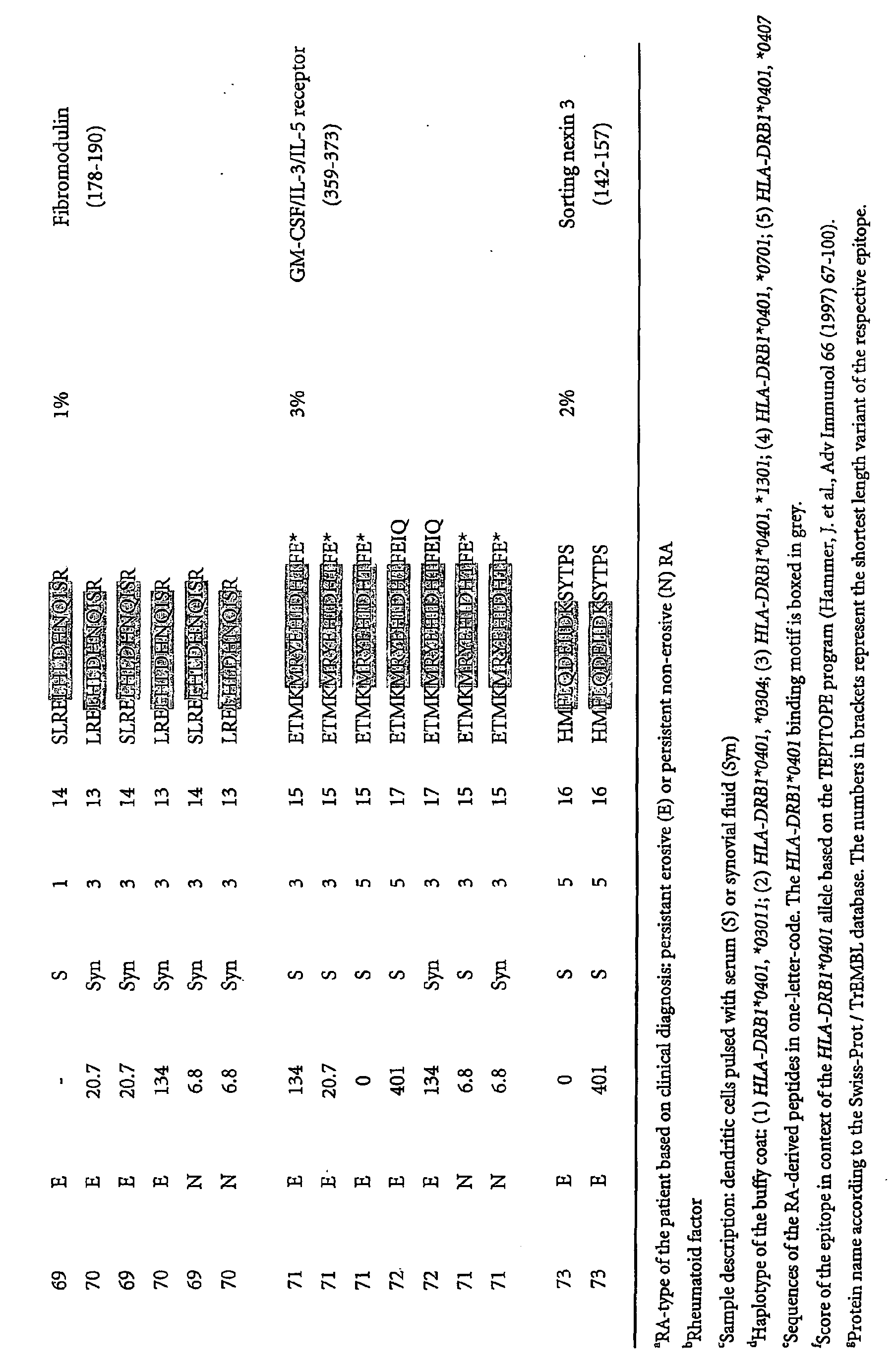

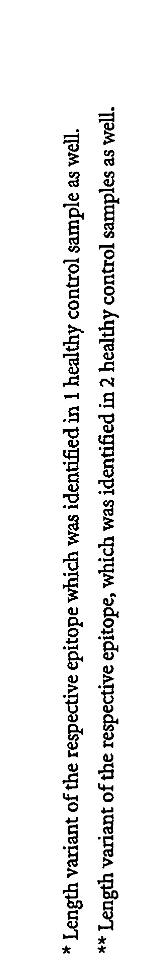

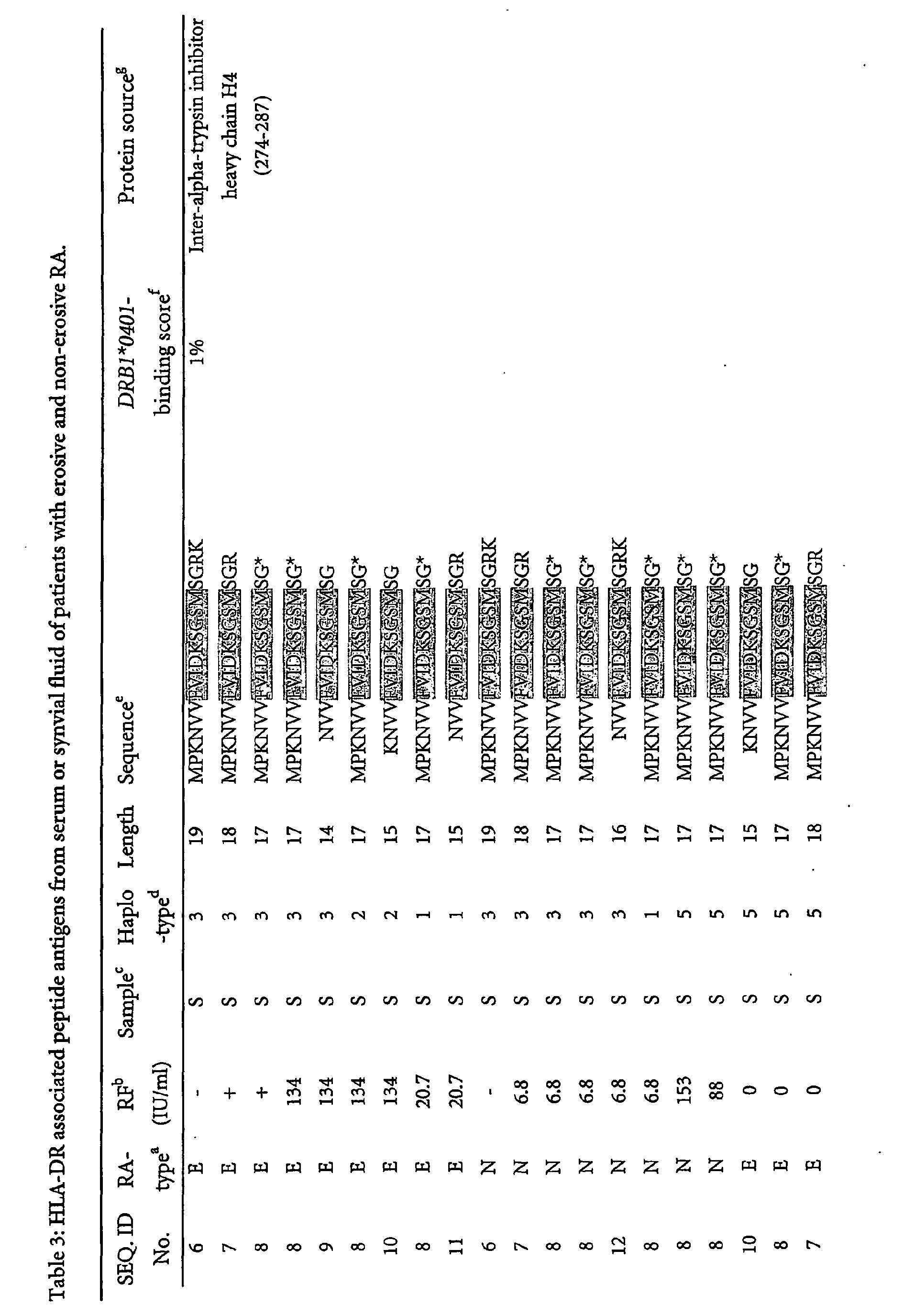

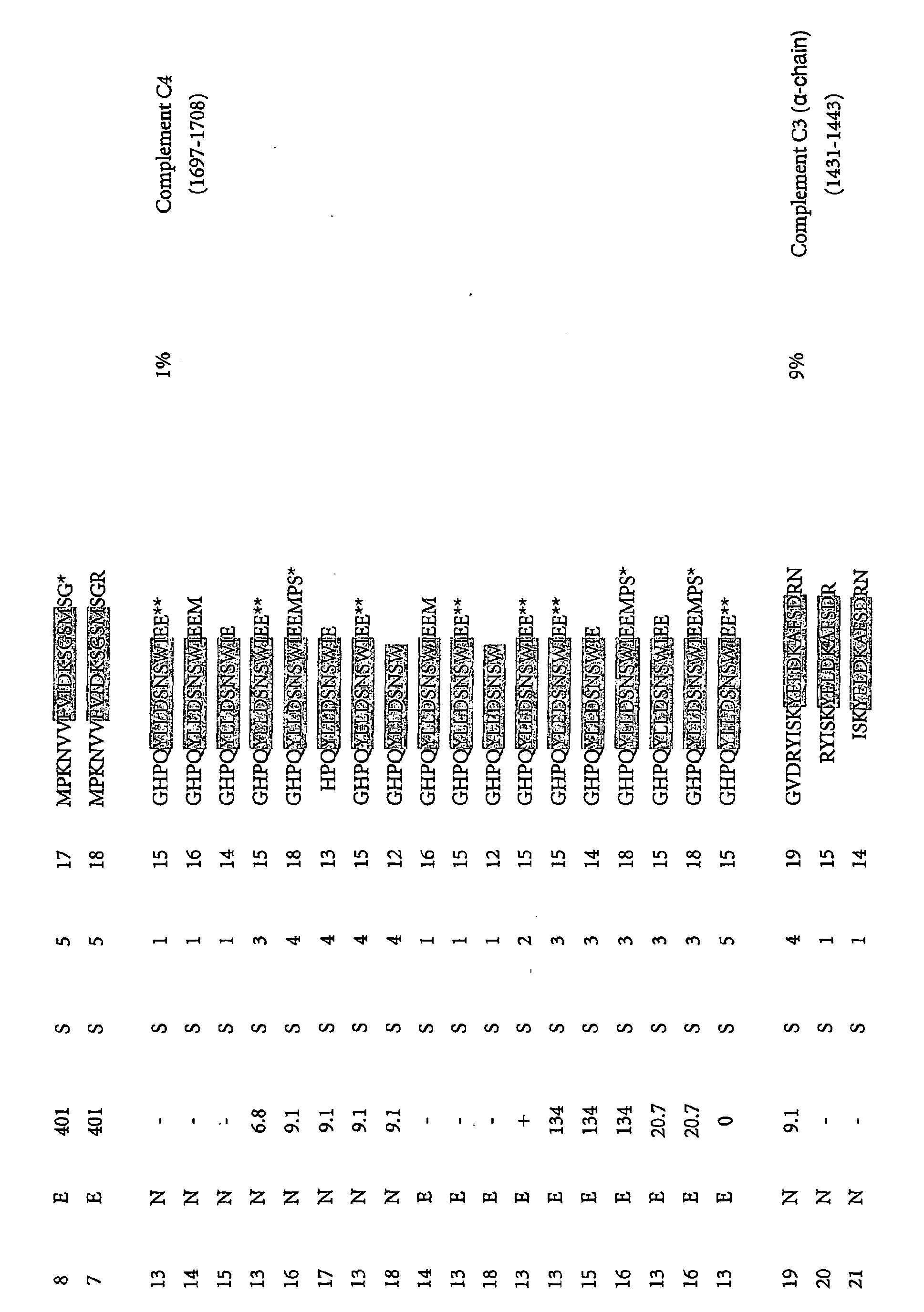

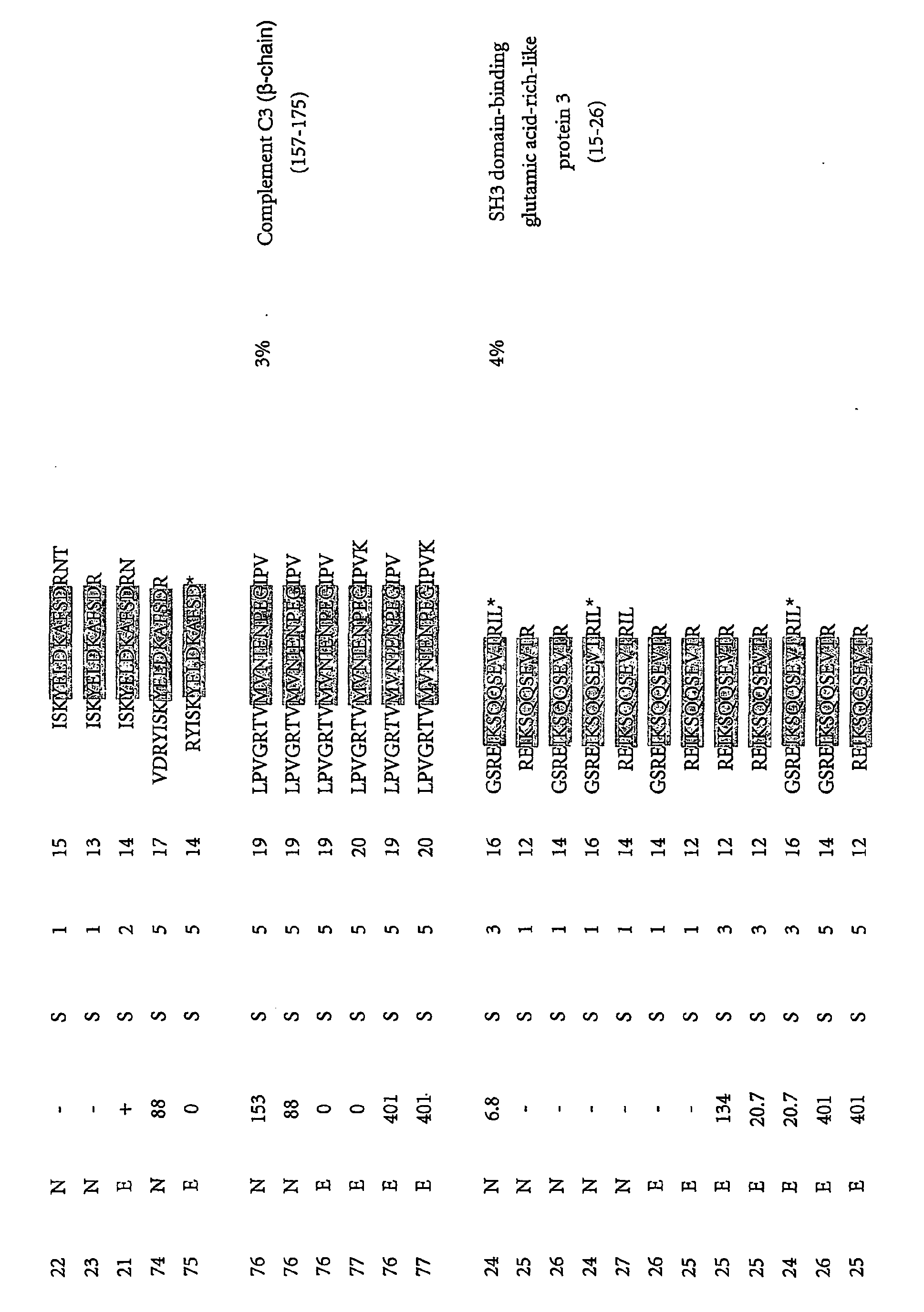

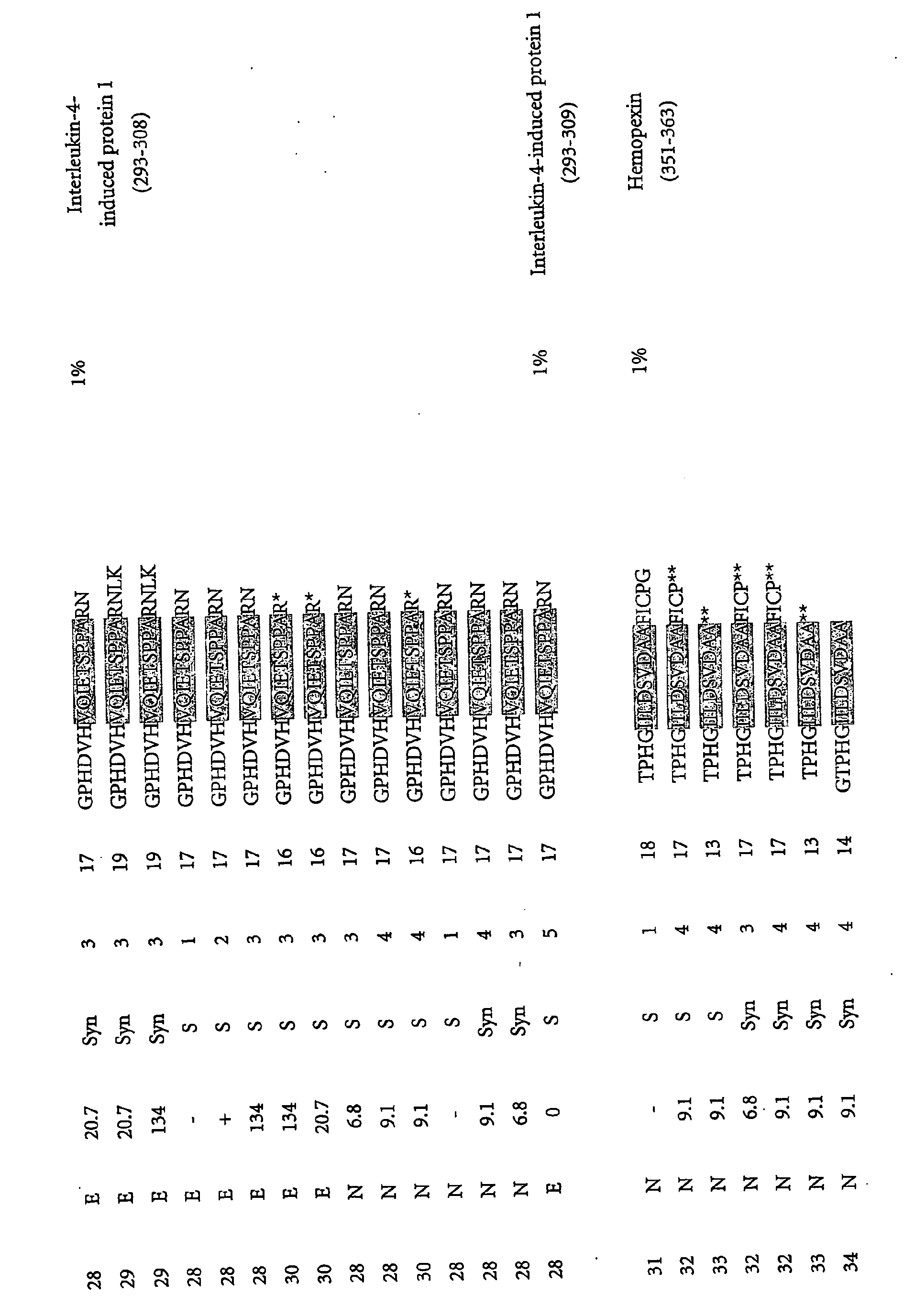

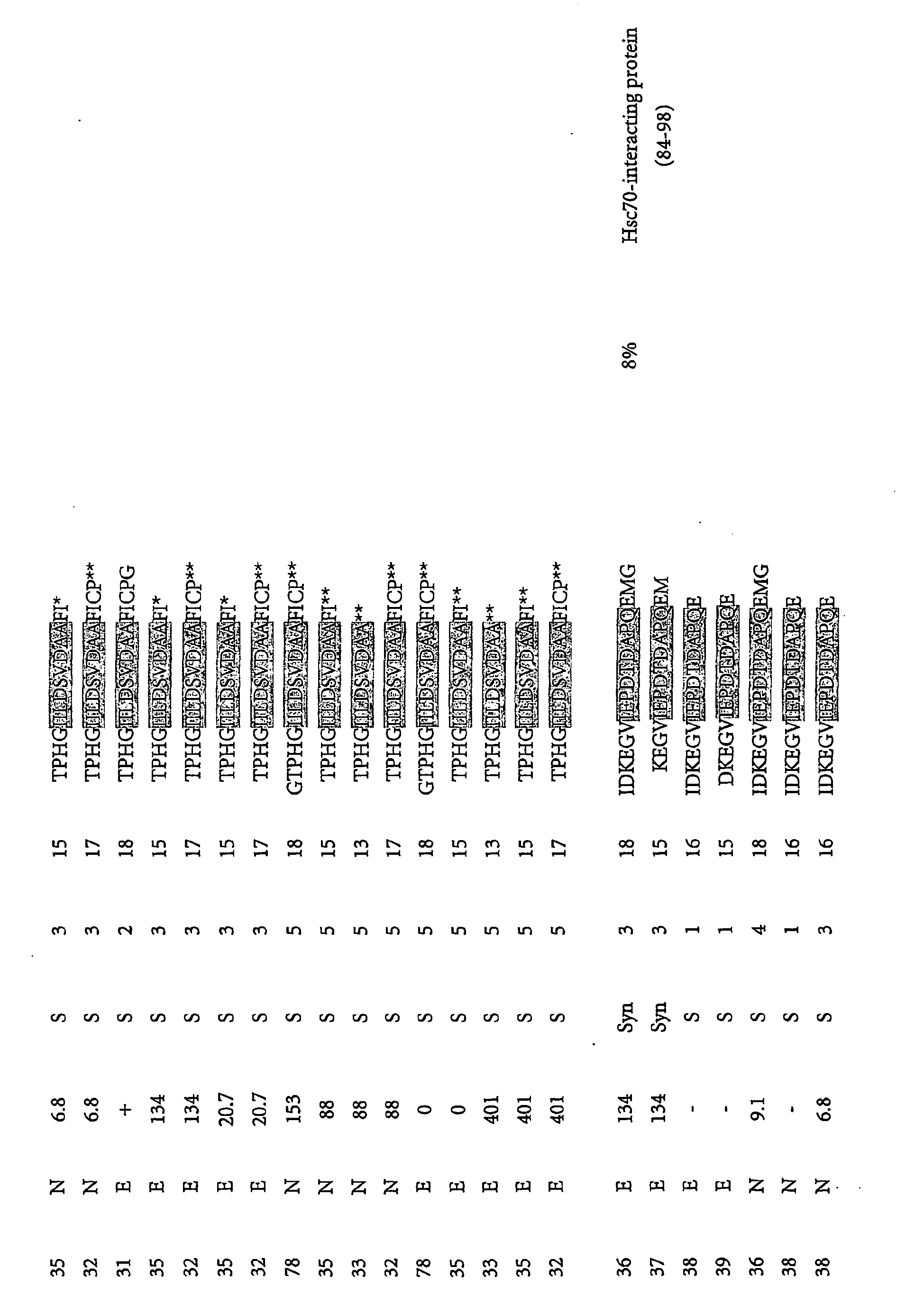

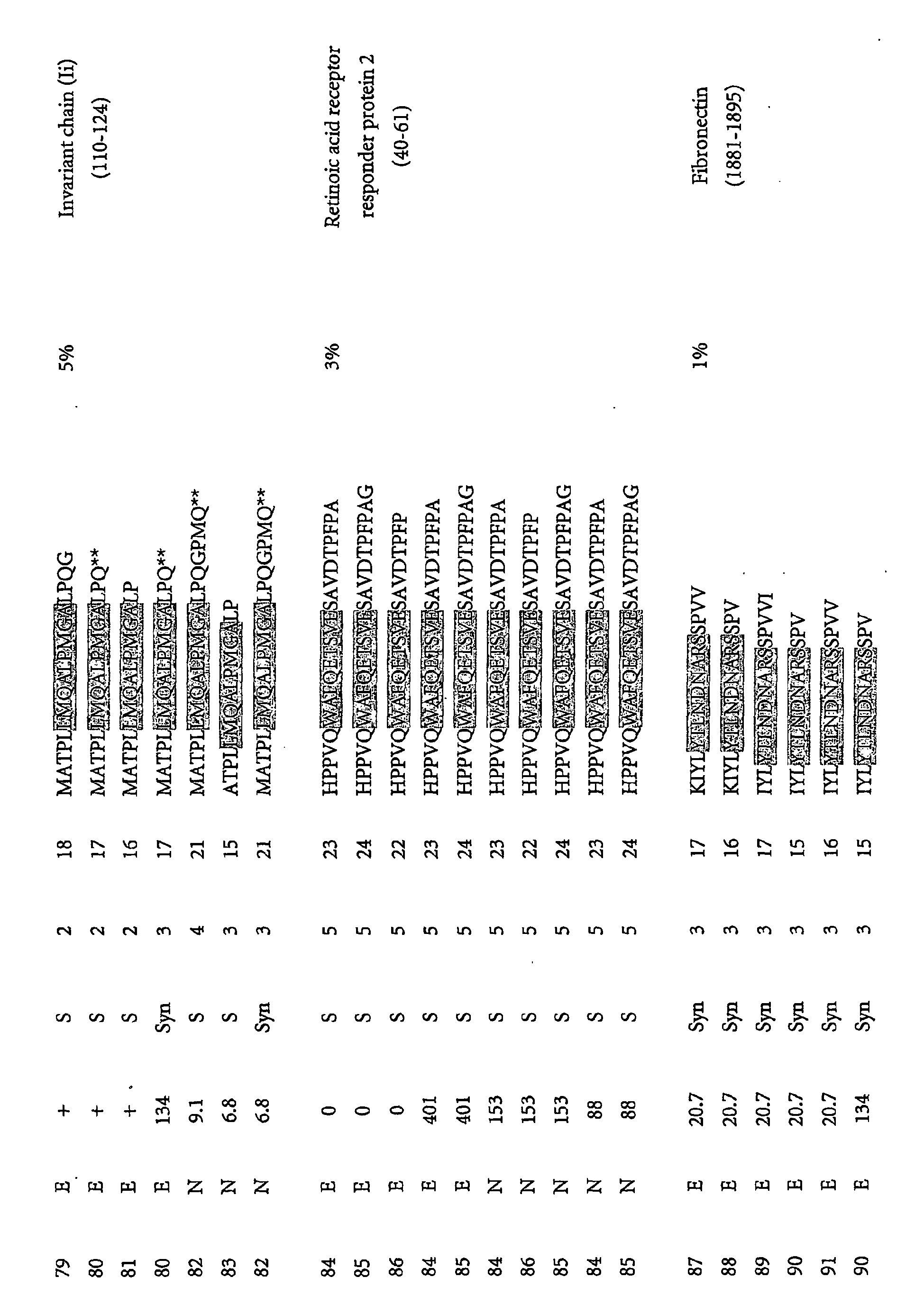

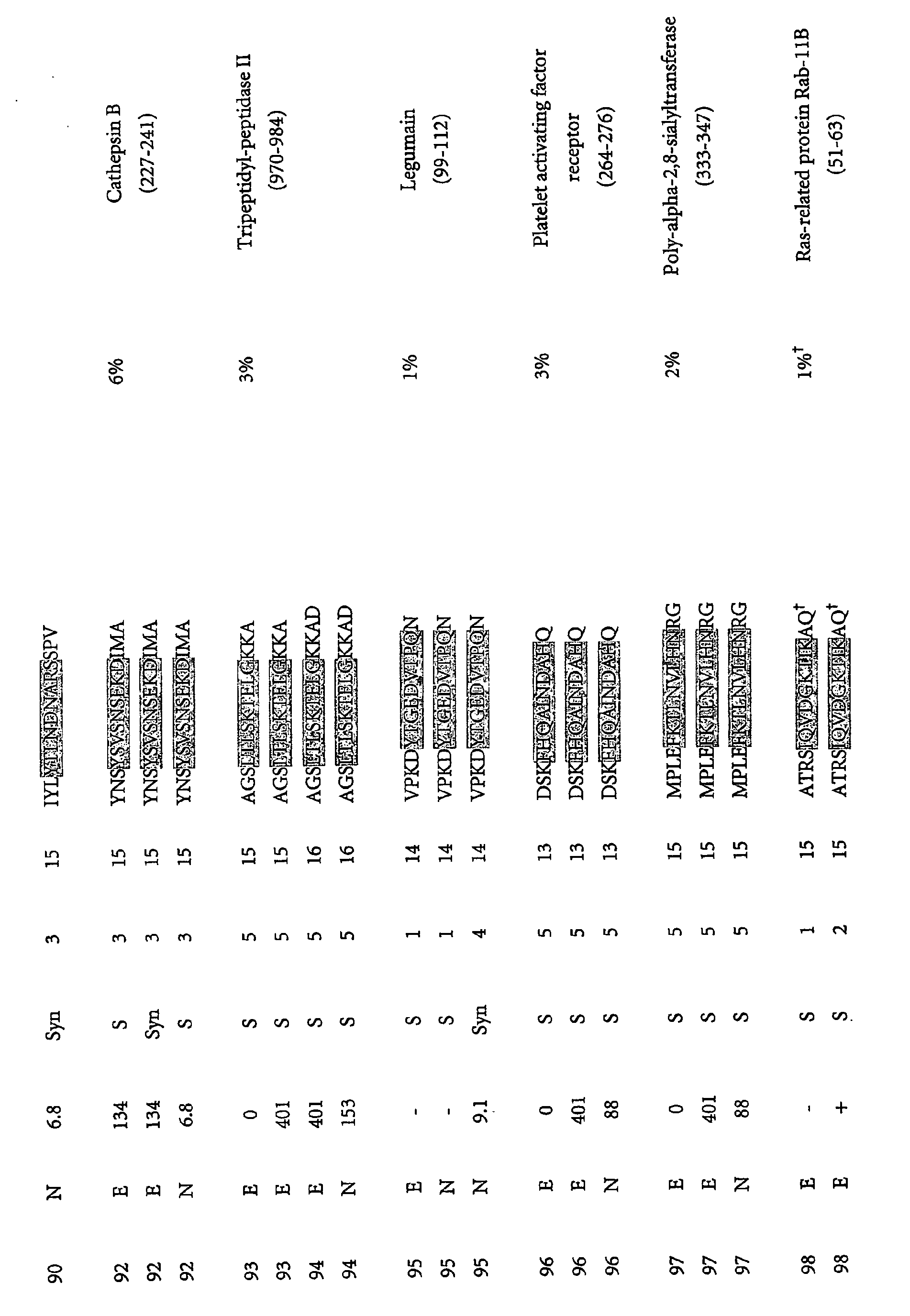

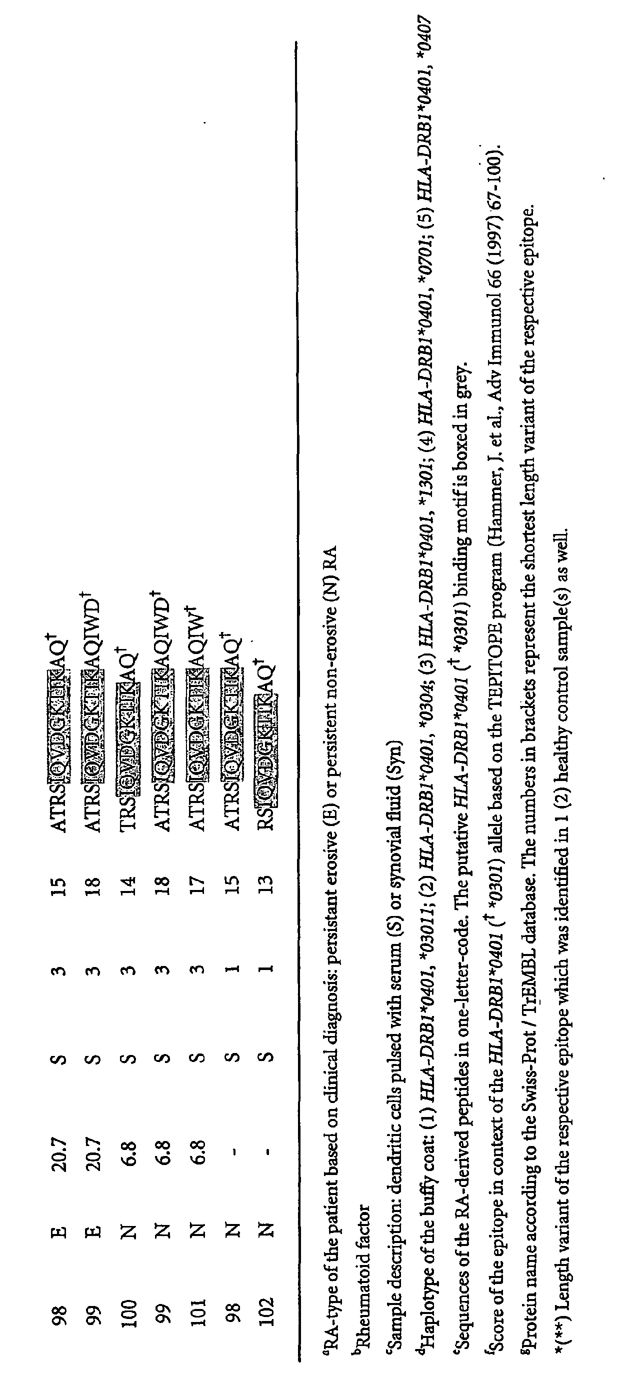

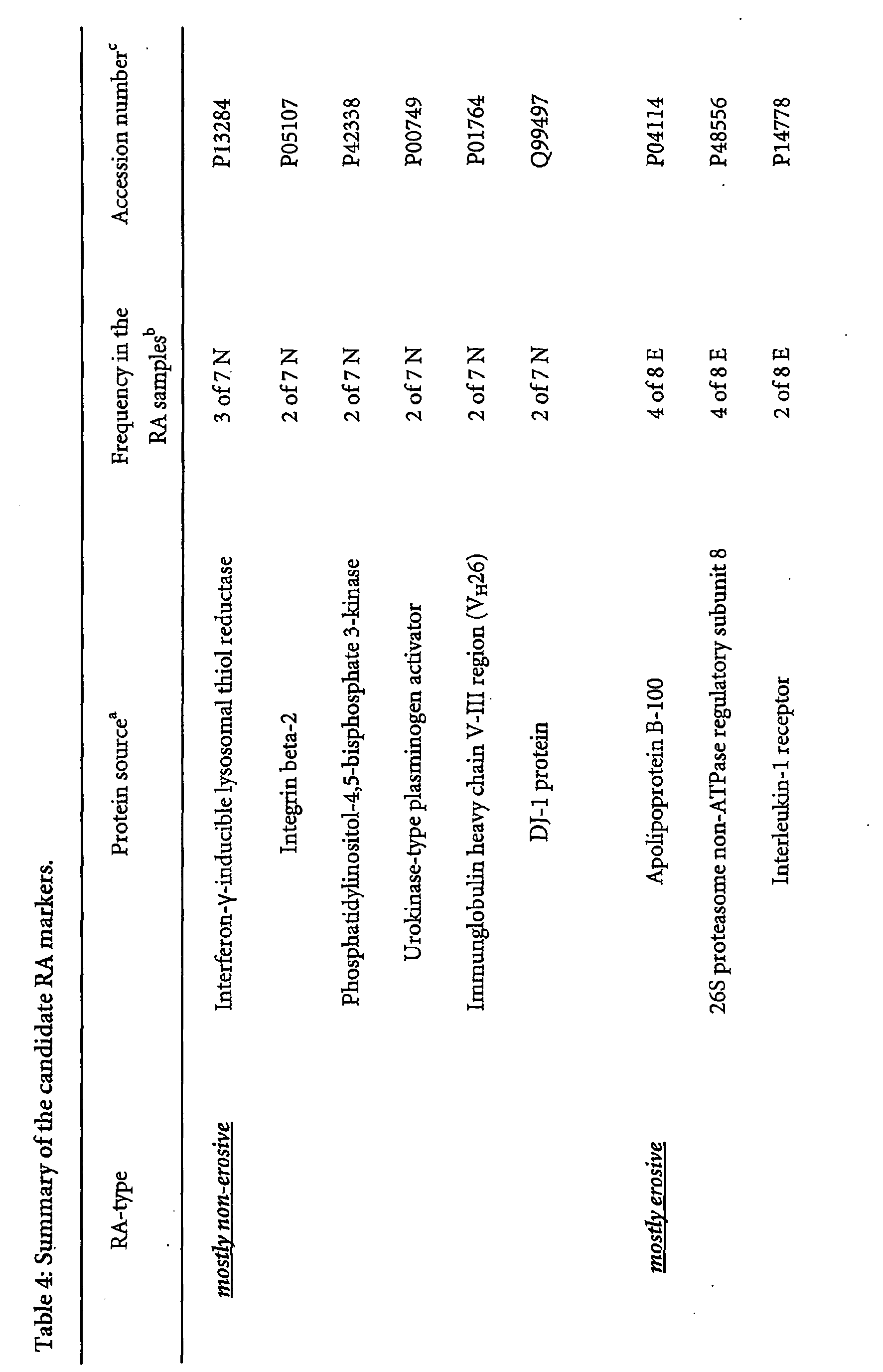

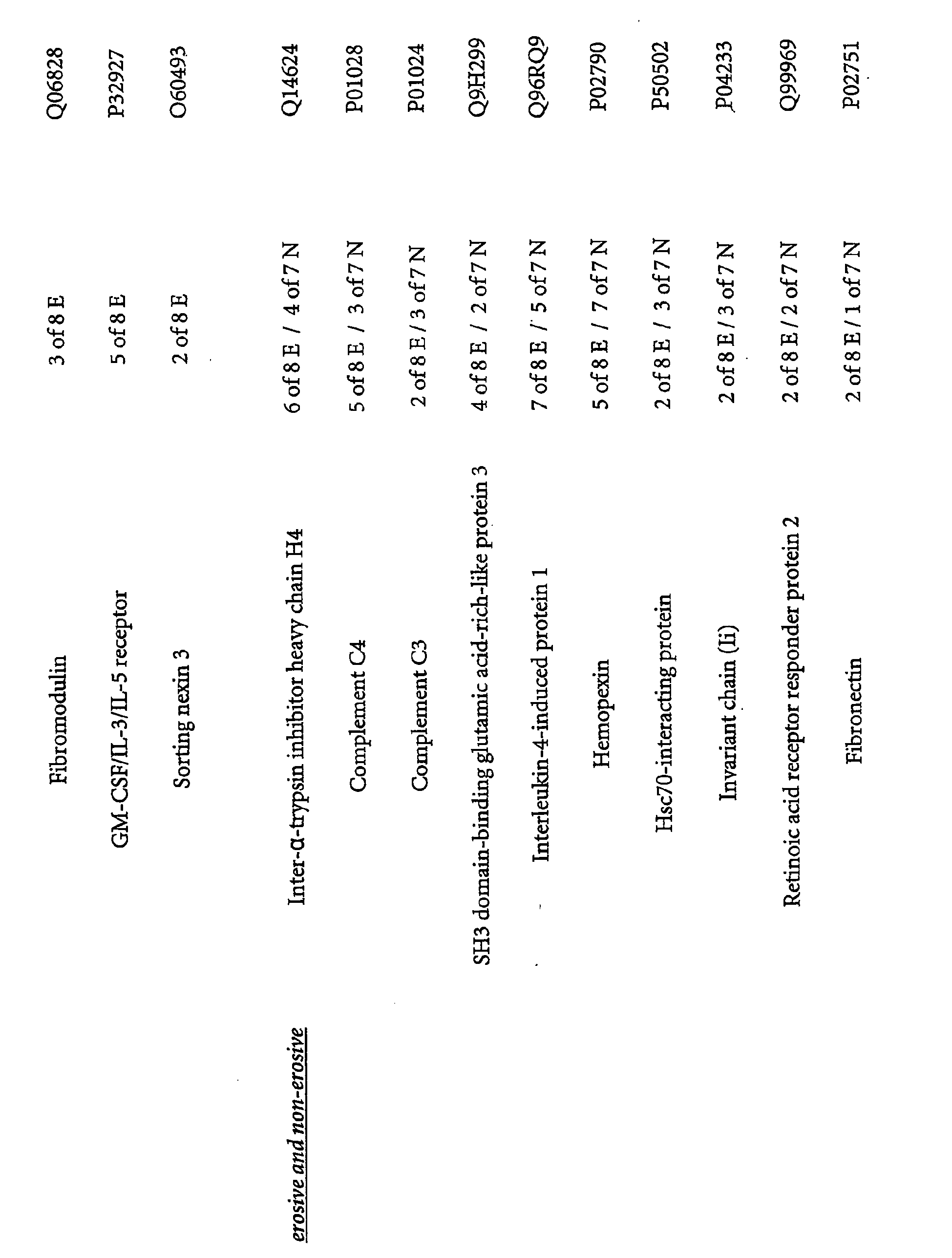

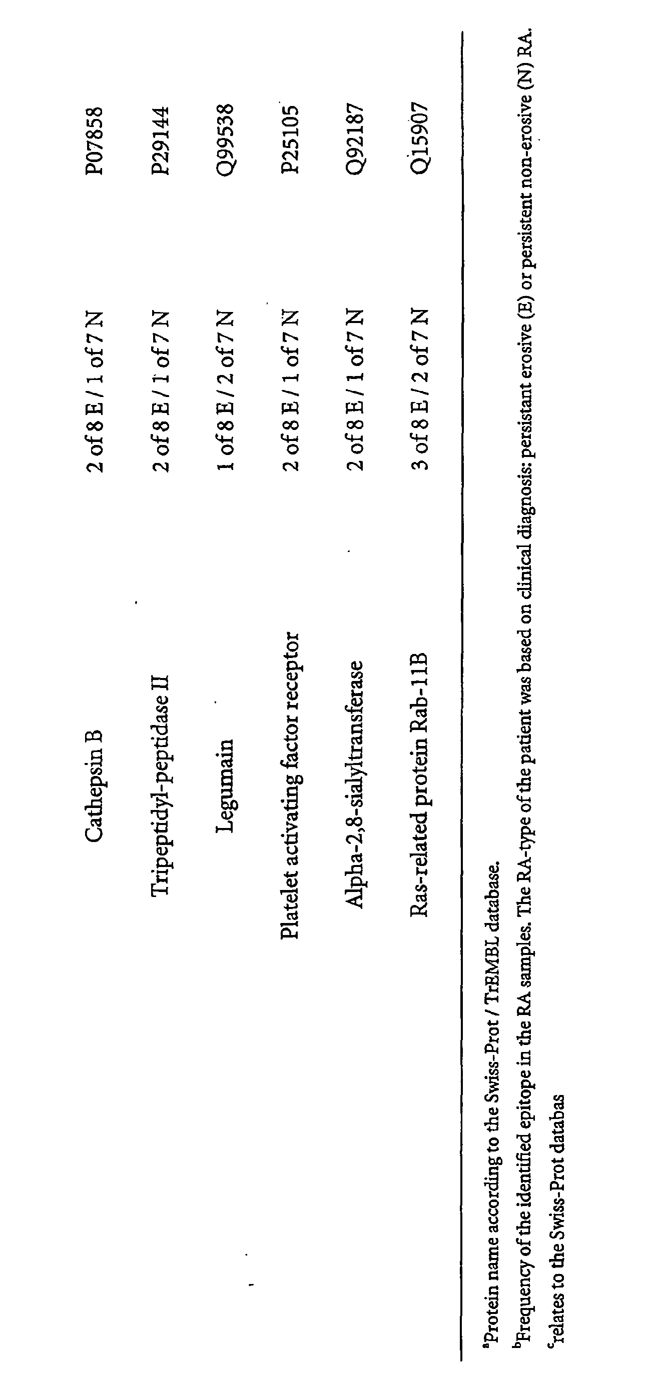

## Claims

1. A MHC class II antigenic peptide comprising
(a) at least the amino acid sequence of the peptide binding motif of SEQ ID NO. 49, or
(b) at least the amino acid sequence of the peptide binding motif of SEQ ID NO. 49 with additional N-and C-termirlal flanking sequences of a corresponding sequence selected from the group consisting of SEQ ID NOs. 1 to 3.

2. The MHC class II antigenic peptide according to claim 1 linked to a MHC class II molecule.

3. An antibody reactive with a MHC class II antigenic peptide according to claim 1.

4. A nucleic acid molecule encoding a peptide or polypeptide according to claim 1.

5. A recombinant nucleic acid construct comprising the nucleic acid molecule according to claim 4 operably linked to an expression vector.

6. A host cell containing the nucleic acid construct according to claim 5.

7. A method for producing a MHC class II antigenic peptide according to claim 1 comprising the steps of culturing the host cell of claim 6 under conditions allowing expression of said peptide and recovering the peptide from the cells or the culture medium.

8. A pharmaceutical composition comprising a MHC class II antigenic peptide according to claim 1 or an antibody according to claim 3, and optionally a pharmaceutically acceptable carrier.

9. A diagnostic composition comprising the antibody according to claim 3.

10. The use of the MHC class II antigenic peptide according to claim 1 as a marker for erosive and/or non-erosive RA.

## Patentansprüche

1. MHC-Klasse II-antigenes Peptid, das umfasst
(a) mindestens die Aminosäuresequenz des Peptidbindemotivs von SEQ ID NO. 49, oder
(b) mindestens die Aminosäuresequenz des Peptidbindemotivs von SEQ ID NO. 49 mit zusätzlichen N- und C-terminal-flankierenden Sequenzen einer entsprechenden Sequenz ausgewählt aus der Gruppe bestehend aus SEQ ID NOs. 1 bis 3.

2. MHC-Klasse II-antigenes Peptid gemäß Anspruch 1, das an ein MHC-Klasse II-Molekül geknüpft ist.

3. Antikörper, der mit einem MHC-Klasse II-antigenen Peptid gemäß Anspruch 1 reaktiv ist.

4. Nucleinsäuremolekül, das ein Peptid oder Polypeptid gemäß Anspruch 1 codiert.

5. Rekombinantes Nucleinsäurekonstrukt, das das Nucleinsäuremolekül gemäß Anspruch 4, funktionell an einen Expressionsvektor geknüpft, umfasst.

6. Wirtszelle, die das Nucleinsäurekonstrukt gemäß Anspruch 5 enthält.

7. Verfahren zum Herstellen eines MHC-Klasse II-antigenen Peptids gemäß Anspruch 1, umfassend die Schritte des Züchtens der Wirtszelle gemäß Anspruch 6 unter Bedingungen, die die Expression des Peptids erlauben, und des Gewinnens der Peptide aus den Zellen oder dem Kulturmedium.

8. Arzneimittel, das das MHC-Klasse II-antigene Peptid gemäß Anspruch 1 oder den Antikörper gemäß Anspruch 3 und gegebenenfalls einen pharmazeutisch verträglichen Träger umfasst.

9. Diagnostische Zusammensetzung, die den Antikörper gemäß Anspruch 3 umfasst.

10. Verwendung des MHC-Klasse II-antigenen Peptids gemäß Anspruch 1 als einen Marker für erosive und/oder nicht-erosive RA.

## Revendications

1. Peptide antigénique de complexe majeur d'histocompatibilité (MHC) de classe II, comprenant
(a) au moins la séquence d'aminoacides du motif de liaison de peptide de la SEQ ID N° 49, ou
(b) au moins la séquence d'aminoacides du motif de liaison de peptide de la SEQ ID N° 49 avec des séquences adjacentes N- et C-terminales supplémentaires d'une séquence correspondante choisie dans le groupe consistant en les SEQ ID N° 1 à 3.

2. Peptide antigénique de MHC de classe II suivant la revendication 1, lié à une molécule de MHC de classe II.

3. Anticorps réactif avec un peptide antigénique de MHC de classe II suivant la revendication 1.

4. Molécule d'acide nucléique codant pour un peptide ou polypeptide suivant la revendication 1.

5. Produit d'assemblage d'acide nucléique recombinant comprenant la molécule d'acide nucléique suivant la revendication 4 liée de manière fonctionnelle à un vecteur d'expression.

6. Cellule hôte contenant le produit d'assemblage d'acide nucléique suivant la revendication 5.

7. Méthode pour la production d'un peptide antigénique de MHC de classe II suivant la revendication 1, comprenant les étapes consistant à cultiver la cellule hôte de la revendication 6 dans les conditions permettant l'expression dudit peptide et à recueillir le peptide à partir de cellules ou du milieu de culture.

8. Composition pharmaceutique comprenant un peptide antigénique de MHC de classe II suivant la revendication 1 ou un anticorps suivant la revendication 3, et facultativement un support pharmaceutiquement acceptable.

9. Composition de diagnostic comprenant l'anticorps suivant la revendication 3.

10. Utilisation du peptide antigénique de MHC de classe II suivant la revendication 1 comme marqueur pour la polyarthrite rhumatoïde (RA) érosive et/ou non érosive.


### SEQUENCE LISTING

<110> F. Hoffmann-La Roche AG
<120> RA antigenic peptides
<130> 21796
<160> 141
<170> PatentIn version 3.2
<210> 1
   <211> 14
   <212> PRT
   <213> Homo sapiens
<400> 1
   Gly Asp Arg Gly Met Gln Leu Met His Ala Asn Ala Gln Arg
   1               5                   10
<210> 2
   <211> 17
   <212> PRT
   <213> Homo sapiens
<400> 2
   Gly Asp Arg Gly Met Gln Leu Met His Ala Asn Ala Gln Arg Thr Asp
   1               5                   10                  15
   Ala
<210> 3
   <211> 16
   <212> PRT
   <213> Homo sapiens
<400> 3
   Gly Asp Arg Gly Met Gln Leu Met His Ala Asn Ala Gln Arg Thr Asp
   1               5                   10                  15
<210> 4
   <211> 16
   <212> PRT
   <213> Homo sapiens
<400> 4
   Ile Asn Asn Gln Leu Thr Leu Asp Ser Asn Thr Lys Tyr Phe His Lys
   1               5                   10                  15
<210> 5
   <211> 17
   <212> PRT
   <213> Homo sapiens
<400> 5
   Ile Asn Asn Gln Leu Thr Leu Asp Ser Asn Thr Lys Tyr Phe His Lys
   1               5                   10                  15
   Leu
<210> 6
   <211> 19
   <212> PRT
   <213> Homo sapiens
<400> 6
   Met Pro Lys Asn Val Val Phe Val Ile Asp Lys Ser Gly Ser Met Ser
   1               5                   10                  15
   Gly Arg Lys
<210> 7
   <211> 18
   <212> PRT
   <213> Homo sapiens
<400> 7
   Met Pro Lys Asn Val Val Phe Val Ile Asp Lys Ser Gly Ser Met Ser
   1               5                   10                  15
   Gly Arg
<210> 8
   <211> 17
   <212> PRT
   <213> Homo sapiens
<400> 8
   Met Pro Lys Asn Val Val Phe Val Ile Asp Lys Ser Gly Ser Met Ser
   1               5                   10                  15
   Gly
<210> 9
   <211> 14
   <212> PRT
   <213> Homo sapiens
<400> 9
   Asn Val Val Phe Val Ile Asp Lys Ser Gly Ser Met Ser Gly
   1               5                   10
<210> 10
   <211> 15
   <212> PRT
   <213> Homo sapiens
<400> 10
   Lys Asn Val Val Phe Val Ile Asp Lys Ser Gly Ser Met Ser Gly
   1               5                   10                  15
<210> 11
   <211> 15
   <212> PRT
   <213> Homo sapiens
<400> 11
   Asn Val Val Phe Val Ile Asp Lys Ser Gly Ser Met Ser Gly Arg
   1               5                   10                  15
<210> 12
   <211> 16
   <212> PRT
   <213> Homo sapiens
<400> 12
   Asn Val Val Phe Val Ile Asp Lys Ser Gly Ser Met Ser Gly Arg Lys
   1               5                   10                  15
<210> 13
   <211> 15
   <212> PRT
   <213> Homo sapiens
<400> 13
   Gly His Pro Gln Tyr Leu Leu Asp Ser Asn Ser Trp Ile Glu Glu
   1               5                   10                  15
<210> 14
   <211> 16
   <212> PRT
   <213> Homo sapiens
<400> 14
   Gly His Pro Gln Tyr Leu Leu Asp Ser Asn Ser Trp Ile Glu Glu Met
   1               5                   10                  15
<210> 15
   <211> 14
   <212> PRT
   <213> Homo sapiens
<400> 15
   Gly His Pro Gln Tyr Leu Leu Asp Ser Asn Ser Trp Ile Glu
   1               5                   10
<210> 16
   <211> 18
   <212> PRT
   <213> Homo sapiens
<400> 16
   Gly His Pro Gln Tyr Leu Leu Asp Ser Asn Ser Trp Ile Glu Glu Met
   1               5                   10                  15
   Pro Ser
<210> 17
   <211> 13
   <212> PRT
   <213> Homo sapiens
<400> 17
   His Pro Gln Tyr Leu Leu Asp Ser Asn Ser Trp Ile Glu
   1               5                   10
<210> 18
   <211> 12
   <212> PRT
   <213> Homo sapiens
<400> 18
   Gly His Pro Gln Tyr Leu Leu Asp Ser Asn Ser Trp
   1               5                   10
<210> 19
   <211> 19
   <212> PRT
   <213> Homo sapiens
<400> 19
   Gly Val Asp Arg Tyr Ile Ser Lys Tyr Glu Leu Asp Lys Ala Phe Ser
   1               5                   10                  15
   Asp Arg Asn
<210> 20
   <211> 15
   <212> PRT
   <213> Homo sapiens
<400> 20
   Arg Tyr Ile Ser Lys Tyr Glu Leu Asp Lys Ala Phe Ser Asp Arg
   1               5                   10                  15
<210> 21
   <211> 14
   <212> PRT
   <213> Homo sapiens
<400> 21
   Ile Ser Lys Tyr Glu Leu Asp Lys Ala Phe Ser Asp Arg Asn
   1               5                   10
<210> 22
   <211> 15
   <212> PRT
   <213> Homo sapiens
<400> 22
   Ile Ser Lys Tyr Glu Leu Asp Lys Ala Phe Ser Asp Arg Asn Thr
   1 5 10 15
<210> 23
   <211> 13
   <212> PRT
   <213> Homo sapiens
<400> 23
   Ile Ser Lys Tyr Glu Leu Asp Lys Ala Phe Ser Asp Arg
   1 5 10
<210> 24
   <211> 16
   <212> PRT
   <213> Homo sapiens
<400> 24
   Gly Ser Arg Glu Ile Lys Ser Gln Gln Ser Glu Val Thr Arg Ile Leu
   1 5 10 15
<210> 25
   <211> 12
   <212> PRT
   <213> Homo sapiens
<400> 25
   Arg Glu Ile Lys Ser Gln Gln Ser Glu Val Thr Arg
   1 5 10
<210> 26
   <211> 14
   <212> PRT
   <213> Homo sapiens
<400> 26
   Gly Ser Arg Glu Ile Lys Ser Gln Gln Ser Glu Val Thr Arg
   1 5 10
<210> 27
   <211> 14
   <212> PRT
   <213> Homo sapiens
<400> 27
   Arg Glu Ile Lys Ser Gln Gln Ser Glu Val Thr Arg Ile Leu
   1               5                   10
<210> 28
   <211> 17
   <212> PRT
   <213> Homo sapiens
<400> 28
   Gly Pro His Asp Val His Val Gln Ile Glu Thr Ser Pro Pro Ala Arg
   1               5                   10                  15
   Asn
<210> 29
   <211> 19
   <212> PRT
   <213> Homo sapiens
<400> 29
   Gly Pro His Asp Val His Val Gln Ile Glu Thr Ser Pro Pro Ala Arg
   1               5                   10                  15
   Asn Leu Lys
<210> 30
   <211> 16
   <212> PRT
   <213> Homo sapiens
<400> 30
   Gly Pro His Asp Val His Val Gln Ile Glu Thr Ser Pro Pro Ala Arg
   1               5                   10                  15
<210> 31
   <211> 18
   <212> PRT
   <213> Homo sapiens
<400> 31
   Thr Pro His Gly Ile Ile Leu Asp Ser Val Asp Ala Ala Phe Ile Cys
   1               5                   10                  15
   Pro Gly
<210> 32
   <211> 17
   <212> PRT
   <213> Homo sapiens
<400> 32
   Thr Pro His Gly Ile Ile Leu Asp Ser Val Asp Ala Ala Phe Ile Cys
   1               5                   10                  15
   Pro
<210> 33
   <211> 13 .
   <212> PRT
   <213> Homo sapiens
<400> 33
   Thr Pro His Gly Ile Ile Leu Asp Ser Val Asp Ala Ala
   1               5                   10
<210> 34
   <211> 14
   <212> PRT
   <213> Homo sapiens
<400> 34
   Gly Thr Pro His Gly Ile Ile Leu Asp Ser Val Asp Ala Ala
   1               5                   10
<210> 35
   <211> 15
   <212> PRT
   <213> Homo sapiens
<400> 35
   Thr Pro His Gly Ile Ile Leu Asp Ser Val Asp Ala Ala Phe Ile
   1               5                   10                  15
<210> 36
   <211> 18
   <212> PRT
   <213> Homo sapiens
<400> 36
   Ile Asp Lys Glu Gly Val Ile Glu Pro Asp Thr Asp Ala Pro Gln Glu
   1               5                   10                  15
   Met Gly
<210> 37
   <211> 15
   <212> PRT
   <213> Homo sapiens
<400> 37
   Lys Glu Gly Val Ile Glu Pro Asp Thr Asp Ala Pro Gln Glu Met
   1               5                   10                  15
<210> 38
   <211> 16
   <212> PRT
   <213> Homo sapiens
<400> 38
   Ile Asp Lys Glu Gly Val Ile Glu Pro Asp Thr Asp Ala Pro Gln Glu
   1               5                   10                  15
<210> 39
   <211> 15
   <212> PRT
   <213> Homo sapiens
<400> 39
   Asp Lys Glu Gly Val Ile Glu Pro Asp Thr Asp Ala Pro Gln Glu
   1               5                   10                  15
<210> 40
   <211> 261
   <212> PRT
   <213> Homo sapiens
<300>
   <308> Swiss-Prot/P13284
   <309> 1990-01-01
   <313> (1)..(261)
<400> 40
<210> 41
   <211> 4563
   <212> PRT
   <213> Homo sapiens
<300>
   <308> Swiss-Prot/P04114
   <309> 1986-11-01
   <313> (1)..(4563)
<400> 41
   Met Asp Pro Pro Arg Pro Ala Leu Leu Ala Leu Leu Ala Leu Pro Ala
   1               5                   10                  15
   Leu Leu Leu Leu Leu Leu Ala Gly Ala Arg Ala Glu Glu Glu Met Leu
               20                  25                  30
   Glu Asn Val Ser Leu Val Cys Pro Lys Asp Ala Thr Arg Phe Lys His
           35                  40                  45
   Leu Arg Lys Tyr Thr Tyr Asn Tyr Glu Ala Glu Ser Ser Ser Gly Val
       50                  55                  60
   Pro Gly Thr Ala Asp Ser Arg Ser Ala Thr Arg Ile Asn Cys Lys Val
   65                  70                  75                  80
   Glu Leu Glu Val Pro Gln Leu Cys Ser Phe Ile Leu Lys Thr Ser Gln
                   85                  90                  95
   Cys Thr Leu Lys Glu Val Tyr Gly Phe Asn Pro Glu Gly Lys Ala Leu
               100                 105                 110
   Leu Lys Lys Thr Lys Asn Ser Glu Glu Phe Ala Ala Ala Met Ser Arg
           115                 120                 125
   Tyr Glu Leu Lys Leu Ala Ile Pro Glu Gly Lys Gln Val Phe Leu Tyr
       130                 135                 140
   Pro Glu Lys Asp Glu Pro Thr Tyr Ile Leu Asn Ile Lys Arg Gly Ile
   145                 150                 155                 160
   Ile Ser Ala Leu Leu Val Pro Pro Glu Thr Glu Glu Ala Lys Gln Val
                   165                 170                 175
   Leu Phe Leu Asp Thr Val Tyr Gly Asn Cys Ser Thr His Phe Thr Val
               180                 185                 190
   Lys Thr Arg Lys Gly Asn Val Ala Thr Glu Ile Ser Thr Glu Arg Asp
   195 200 205
   Leu Gly Gln Cys Asp Arg Phe Lys Pro Ile Arg Thr Gly Ile Ser Pro
   210 215 220
   Leu Ala Leu Ile Lys Gly Met Thr Arg Pro Leu Ser Thr Leu Ile Ser
   225 230 235 240
   Ser Ser Gln Ser Cys Gln Tyr Thr Leu Asp Ala Lys Arg Lys His Val
   245 250 255
   Ala Glu Ala Ile Cys Lys Glu Gln His Leu Phe Leu Pro Phe Ser Tyr
   260 265 270
   Asn Asn Lys Tyr Gly Met Val Ala Gln Val Thr Gln Thr Leu Lys Leu
   275 280 285
   Glu Asp Thr Pro Lys Ile Asn Ser Arg Phe Phe Gly Glu Gly Thr Lys
   290 295 300
   Lys Met Gly Leu Ala Phe Glu Ser Thr Lys Ser Thr Ser Pro Pro Lys
   305 310 315 320
   Gln Ala Glu Ala Val Leu Lys Thr Leu Gln Glu Leu Lys Lys Leu Thr
   325 330 335
   Ile Ser Glu Gln Asn Ile Gln Arg Ala Asn Leu Phe Asn Lys Leu Val
   340 345 350
   Thr Glu Leu Arg Gly Leu Ser Asp Glu Ala Val Thr Ser Leu Leu Pro
   355 360 365
   Gln Leu Ile Glu Val Ser Ser Pro Ile Thr Leu Gln Ala Leu Val Gln
   370 375 380
   Cys Gly Gln Pro Gln Cys Ser Thr His Ile Leu Gln Trp Leu Lys Arg
   385 390 395 400
   Val His Ala Asn Pro Leu Leu Ile Asp Val Val Thr Tyr Leu Val Ala
   405 410 415
   Leu Ile Pro Glu Pro Ser Ala Gln Gln Leu Arg Glu Ile Phe Asn Met
   420 425 430
   Ala Arg Asp Gln Arg Ser Arg Ala Thr Leu Tyr Ala Leu Ser His Ala
   435 440 445
   Val Asn Asn Tyr His Lys Thr Asn Pro Thr Gly Thr Gln Glu Leu Leu
   450 455 460
   Asp Ile Ala Asn Tyr Leu Met Glu Gln Ile Gln Asp Asp Cys Thr Gly
   465 470 475 480
   Asp Glu Asp Tyr Thr Tyr Leu Ile Leu Arg Val Ile Gly Asn Met Gly
   485 490 495
   Gln Thr Met Glu Gln Leu Thr Pro Glu Leu Lys Ser Ser Ile Leu Lys
   500 505 510
   Cys Val Gln Ser Thr Lys Pro Ser Leu Met Ile Gln Lys Ala Ala Ile
   515 520 525
   Gln Ala Leu Arg Lys Met Glu Pro Lys Asp Lys Asp Gln Glu Val Leu
   530 535 540
   Leu Gln Thr Phe Leu Asp Asp Ala Ser Pro Gly Asp Lys Arg Leu Ala
   545 550 555 560
   Ala Tyr Leu Met Leu Met Arg Ser Pro Ser Gln Ala Asp Ile Asn Lys
   565 570 575
   Ile Val Gln Ile Leu Pro Trp Glu Gln Asn Glu Gln Val Lys Asn Phe
   580 585 590
   Val Ala Ser His Ile Ala Asn Ile Leu Asn Ser Glu Glu Leu Asp Ile
   595 600 605
   Gln Asp Leu Lys Lys Leu Val Lys Glu Ala Leu Lys Glu Ser Gln Leu
   610 615 620
   Pro Thr Val Met Asp Phe Arg Lys Phe Ser Arg Asn Tyr Gln Leu Tyr
   625 630 635 640
   Lys Ser Val Ser Leu Pro Ser Leu Asp Pro Ala Ser Ala Lys Ile Glu
   645 650 655
   Gly Asn Leu Ile Phe Asp Pro Asn Asn Tyr Leu Pro Lys Glu Ser Met
   660 665 670
   Leu Lys Thr Thr Leu Thr Ala Phe Gly Phe Ala Ser Ala Asp Leu Ile
   675 680 685
   Glu Ile Gly Leu Glu Gly Lys Gly Phe Glu Pro Thr Leu Glu Ala Leu
   690 695 700
   Phe Gly Lys Gln Gly Phe Phe Pro Asp Ser Val Asn Lys Ala Leu Tyr
   705 710 715 720
   Trp Val Asn Gly Gln Val Pro Asp Gly Val Ser Lys Val Leu Val Asp
   725 730 735
   His Phe Gly Tyr Thr Lys Asp Asp Lys His Glu Gln Asp Met Val Asn
   740 745 750
   Gly Ile Met Leu Ser Val Glu Lys Leu Ile Lys Asp Leu Lys Ser Lys
   755 760 765
   Glu Val Pro Glu Ala Arg Ala Tyr Leu Arg Ile Leu Gly Glu Glu Leu
   770 775 780
   Gly Phe Ala Ser Leu His Asp Leu Gln Leu Leu Gly Lys Leu Leu Leu
   785 790 795 800
   Met Gly Ala Arg Thr Leu Gln Gly Ile Pro Gln Met Ile Gly Glu Val
   805 810 815
   Ile Arg Lys Gly Ser Lys Asn Asp Phe Phe Leu His Tyr Ile Phe Met
   820 825 830
   Glu Asn Ala Phe Glu Leu Pro Thr Gly Ala Gly Leu Gln Leu Gln Ile
   835 840 845
   Ser Ser Ser Gly Val Ile Ala Pro Gly Ala Lys Ala Gly Val Lys Leu
   850 855 860
   Glu Val Ala Asn Met Gln Ala Glu Leu Val Ala Lys Pro Ser Val Ser
   865 870 875 880
   Val Glu Phe Val Thr Asn Met Gly Ile Ile Ile Pro Asp Phe Ala Arg
   885 890 895
   Ser Gly Val Gln Met Asn Thr Asn Phe Phe His Glu Ser Gly Leu Glu
   900 905 910
   Ala His Val Ala Leu Lys Ala Gly Lys Leu Lys Phe Ile Ile Pro Ser
   915 920 925
   Pro Lys Arg Pro Val Lys Leu Leu Ser Gly Gly Asn Thr Leu His Leu
   930 935 940
   Val Ser Thr Thr Lys Thr Glu Val Ile Pro Pro Leu Ile Glu Asn Arg
   945 950 955 960
   Gln Ser Trp Ser Val Cys Lys Gln Val Phe Pro Gly Leu Asn Tyr Cys
   965 970 975
   Thr Ser Gly Ala Tyr Ser Asn Ala Ser Ser Thr Asp Ser Ala Ser Tyr
   980 985 990
   Tyr Pro Leu Thr Gly Asp Thr Arg Leu Glu Leu Glu Leu Arg Pro Thr
   995 1000 1005
   Gly Glu Ile Glu Gln Tyr Ser Val Ser Ala Thr Tyr Glu Leu Gln
   1010 1015 1020
   Arg Glu Asp Arg Ala Leu Val Asp Thr Leu Lys Phe Val Thr Gln
   1025 1030 1035
   Ala Glu Gly Ala Lys Gln Thr Glu Ala Thr Met Thr Phe Lys Tyr
   1040 1045 1050
   Asn Arg Gln Ser Met Thr Leu Ser Ser Glu Val Gln Ile Pro Asp
   1055 1060 1065
   Phe Asp Val Asp Leu Gly Thr Ile Leu Arg Val Asn Asp Glu Ser
   1070 1075 1080
   Thr Glu Gly Lys Thr Ser Tyr Arg Leu Thr Leu Asp Ile Gln Asn
   1085 1090 1095
   Lys Lys Ile Thr Glu Val Ala Leu Met Gly His Leu Ser Cys Asp
   1100 1105 1110
   Thr Lys Glu Glu Arg Lys Ile Lys Gly Val Ile Ser Ile Pro Arg
   1115 1120 1125
   Leu Gln Ala Glu Ala Arg Ser Glu Ile Leu Ala His Trp Ser Pro
   1130 1135 1140
   Ala Lys Leu Leu Leu Gln Met Asp Ser Ser Ala Thr Ala Tyr Gly
   1145 1150 1155
   Ser Thr Val Ser Lys Arg Val Ala Trp His Tyr Asp Glu Glu Lys
   1160 1165 1170
   Ile Glu Phe Glu Trp Asn Thr Gly Thr Asn Val Asp Thr Lys Lys
   1175 1180 1185
   Met Thr Ser Asn Phe Pro Val Asp Leu Ser Asp Tyr Pro Lys Ser
   1190 1195 1200
   Leu His Met Tyr Ala Asn Arg Leu Leu Asp His Arg Val Pro Glu
   1205 1210 1215
   Thr Asp Met Thr Phe Arg His Val Gly Ser Lys Leu Ile Val Ala
   1220 1225 1230
   Met Ser Ser Trp Leu Gln Lys Ala Ser Gly Ser Leu Pro Tyr Thr
   1235 1240 1245
   Gln Thr Leu Gln Asp His Leu Asn Ser Leu Lys Glu Phe Asn Leu
   1250 1255 1260
   Gln Asn Met Gly Leu Pro Asp Phe His Ile Pro Glu Asn Leu Phe
   1265 1270 1275
   Leu Lys Ser Asp Gly Arg Val Lys Tyr Thr Leu Asn Lys Asn Ser
   1280 1285 1290
   Leu Lys Ile Glu Ile Pro Leu Pro Phe Gly Gly Lys Ser Ser Arg
   1295 1300 1305
   Asp Leu Lys Met Leu Glu Thr Val Arg Thr Pro Ala Leu His Phe
   1310 1315 1320
   Lys Ser Val Gly Phe His Leu Pro Ser Arg Glu Phe Gln Val Pro
   1325 1330 1335
   Thr Phe Thr Ile Pro Lys Leu Tyr Gln Leu Gln Val Pro Leu Leu
   1340 1345 1350
   Gly Val Leu Asp Leu Ser Thr Asn Val Tyr Ser Asn Leu Tyr Asn
   1355 1360 1365
   Trp Ser Ala Ser Tyr Ser Gly Gly Asn Thr Ser Thr Asp His Phe
   1370 1375 1380
   Ser Leu Arg Ala Arg Tyr His Met Lys Ala Asp Ser Val Val Asp
   1385 1390 1395
   Leu Leu Ser Tyr Asn Val Gln Gly Ser Gly Glu Thr Thr Tyr Asp
   1400 1405 1410
   His Lys Asn Thr Phe Thr Leu Ser Cys Asp Gly Ser Leu Arg His
   1415 1420 1425
   Lys Phe Leu Asp Ser Asn Ile Lys Phe Ser His Val Glu Lys Leu
   1430 1435 1440
   Gly Asn Asn Pro Val Ser Lys Gly Leu Leu Ile Phe Asp Ala Ser
   1445 1450 1455
   Ser Ser Trp Gly Pro Gln Met Ser Ala Ser Val His Leu Asp Ser
   1460 1465 1470
   Lys Lys Lys Gln His Leu Phe Val Lys Glu Val Lys Ile Asp Gly
   1475 1480 1485
   Gln Phe Arg Val Ser Ser Phe Tyr Ala Lys Gly Thr Tyr Gly Leu
   1490 1495 1500
   Ser Cys Gln Arg Asp Pro Asn Thr Gly Arg Leu Asn Gly Glu Ser
   1505 1510 1515
   Asn Leu Arg Phe Asn Ser Ser Tyr Leu Gln Gly Thr Asn Gln Ile
   1520 1525 1530
   Thr Gly Arg Tyr Glu Asp Gly Thr Leu Ser Leu Thr Ser Thr Ser
   1535 1540 1545
   Asp Leu Gln Ser Gly Ile Ile Lys Asn Thr Ala Ser Leu Lys Tyr
   1550 1555 1560
   Glu Asn Tyr Glu Leu Thr Leu Lys Ser Asp Thr Asn Gly Lys Tyr
   1565 1570 1575
   Lys Asn Phe Ala Thr Ser Asn Lys Met Asp Met Thr Phe Ser Lys
   1580 1585 1590
   Gln Asn Ala Leu Leu Arg Ser Glu Tyr Gln Ala Asp Tyr Glu Ser
   1595 1600 1605
   Leu Arg Phe Phe Ser Leu Leu Ser Gly Ser Leu Asn Ser His Gly
   1610 1615 1620
   Leu Glu Leu Asn Ala Asp Ile Leu Gly Thr Asp Lys Ile Asn Ser
   1625 1630 1635
   Gly Ala His Lys Ala Thr Leu Arg Ile Gly Gln Asp Gly Ile Ser
   1640 1645 1650
   Thr Ser Ala Thr Thr Asn Leu Lys Cys Ser Leu Leu Val Leu Glu
   1655 1660 1665
   Asn Glu Leu Asn Ala Glu Leu Gly Leu Ser Gly Ala Ser Met Lys
   1670 1675 1680
   Leu Thr Thr Asn Gly Arg Phe Arg Glu His Asn Ala Lys Phe Ser
   1685 1690 1695
   Leu Asp Gly Lys Ala Ala Leu Thr Glu Leu Ser Leu Gly Ser Ala
   1700 1705 1710
   Tyr Gln Ala Met Ile Leu Gly Val Asp Ser Lys Asn Ile Phe Asn
   1715 1720 1725
   Phe Lys Val Ser Gln Glu Gly Leu Lys Leu Ser Asn Asp Met Met
   1730 1735 1740
   Gly Ser Tyr Ala Glu Met Lys Phe Asp His Thr Asn Ser Leu Asn
   1745 1750 1755
   Ile Ala Gly Leu Ser Leu Asp Phe Ser Ser Lys Leu Asp Asn Ile
   1760 1765 1770
   Tyr Ser Ser Asp Lys Phe Tyr Lys Gln Thr Val Asn Leu Gln Leu
   1775 1780 1785
   Gln Pro Tyr Ser Leu Val Thr Thr Leu Asn Ser Asp Leu Lys Tyr
   1790 1795 1800
   Asn Ala Leu Asp Leu Thr Asn Asn Gly Lys Leu Arg Leu Glu Pro
   1805 1810 1815
   Leu Lys Leu His Val Ala Gly Asn Leu Lys Gly Ala Tyr Gln Asn
   1820 1825 1830
   Asn Glu Ile Lys His Ile Tyr Ala Ile Ser Ser Ala Ala Leu Ser
   1835 1840 1845
   Ala Ser Tyr Lys Ala Asp Thr Val Ala Lys Val Gln Gly Val Glu
   1850 1855 1860
   Phe Ser His Arg Leu Asn Thr Asp Ile Ala Gly Leu Ala Ser Ala
   1865 1870 1875
   Ile Asp Met Ser Thr Asn Tyr Asn Ser Asp Ser Leu His Phe Ser
   1880 1885 1890
   Asn Val Phe Arg Ser Val Met Ala Pro Phe Thr Met Thr Ile Asp
   1895 1900 1905
   Ala His Thr Asn Gly Asn Gly Lys Leu Ala Leu Trp Gly Glu His
   1910 1915 1920
   Thr Gly Gln Leu Tyr Ser Lys Phe Leu Leu Lys Ala Glu Pro Leu
   1925 1930 1935
   Ala Phe Thr Phe Ser His Asp Tyr Lys Gly Ser Thr Ser His His
   1940 1945 1950
   Leu Val Ser Arg Lys Ser Ile Ser Ala Ala Leu Glu His Lys Val
   1955 1960 1965
   Ser Ala Leu Leu Thr Pro Ala Glu Gln Thr Gly Thr Trp Lys Leu
   1970 1975 1980
   Lys Thr Gln Phe Asn Asn Asn Glu Tyr Ser Gln Asp Leu Asp Ala
   1985 1990 1995
   Tyr Asn Thr Lys Asp Lys Ile Gly Val Glu Leu Thr Gly Arg Thr
   2000 2005 2010
   Leu Ala Asp Leu Thr Leu Leu Asp Ser Pro Ile Lys Val Pro Leu
   2015 2020 2025
   Leu Leu Ser Glu Pro Ile Asn Ile Ile Asp Ala Leu Glu Met Arg
   2030 2035 2040
   Asp Ala Val Glu Lys Pro Gln Glu Phe Thr Ile Val Ala Phe Val
   2045 2050 2055
   Lys Tyr Asp Lys Asn Gln Asp Val His Ser Ile Asn Leu Pro Phe
   2060 2065 2070
   Phe Glu Thr Leu Gln Glu Tyr Phe Glu Arg Asn Arg Gln Thr Ile
   2075 2080 2085
   Ile Val Val Val Glu Asn Val Gln Arg Asn Leu Lys His Ile Asn
   2090 2095 2100
   Ile Asp Gln Phe Val Arg Lys Tyr Arg Ala Ala Leu Gly Lys Leu
   2105 2110 2115
   Pro Gln Gln Ala Asn Asp Tyr Leu Asn Ser Phe Asn Trp Glu Arg
   2120 2125 2130
   Gln Val Ser His Ala Lys Glu Lys Leu Thr Ala Leu Thr Lys Lys
   2135 2140 2145
   Tyr Arg Ile Thr Glu Asn Asp Ile Gln Ile Ala Leu Asp Asp Ala
   2150 2155 2160
   Lys Ile Asn Phe Asn Glu Lys Leu Ser Gln Leu Gln Thr Tyr Met
   2165 2170 2175
   Ile Gln Phe Asp Gln Tyr Ile Lys Asp Ser Tyr Asp Leu His Asp
   2180 2185 2190
   Leu Lys Ile Ala Ile Ala Asn Ile Ile Asp Glu Ile Ile Glu Lys
   2195 2200 2205
   Leu Lys Ser Leu Asp Glu His Tyr His Ile Arg Val Asn Leu Val
   2210 2215 2220
   Lys Thr Ile His Asp Leu His Leu Phe Ile Glu Asn Ile Asp Phe
   2225 2230 2235
   Asn Lys Ser Gly Ser Ser Thr Ala Ser Trp Ile Gln Asn Val Asp
   2240 2245 2250
   Thr Lys Tyr Gln Ile Arg Ile Gln Ile Gln Glu Lys Leu Gln Gln
   2255 2260 2265
   Leu Lys Arg His Ile Gln Asn Ile Asp Ile Gln His Leu Ala Gly
   2270 2275 2280
   Lys Leu Lys Gln His Ile Glu Ala Ile Asp Val Arg Val Leu Leu
   2285 2290 2295
   Asp Gln Leu Gly Thr Thr Ile Ser Phe Glu Arg Ile Asn Asp Val
   2300 2305 2310
   Leu Glu His Val Lys His Phe Val Ile Asn Leu Ile Gly Asp Phe
   2315 2320 2325
   Glu Val Ala Glu Lys Ile Asn Ala Phe Arg Ala Lys Val His Glu
   2330 2335 2340
   Leu Ile Glu Arg Tyr Glu Val Asp Gln Gln Ile Gln Val Leu Met
   2345 2350 2355
   Asp Lys Leu Val Glu Leu Thr His Gln Tyr Lys Leu Lys Glu Thr
   2360 2365 2370
   Ile Gln Lys Leu Ser Asn Val Leu Gln Gln Val Lys Ile Lys Asp
   2375 2380 2385
   Tyr Phe Glu Lys Leu Val Gly Phe Ile Asp Asp Ala Val Lys Lys
   2390 2395 2400
   Leu Asn Glu Leu Ser Phe Lys Thr Phe Ile Glu Asp Val Asn Lys
   2405 2410 2415
   Phe Leu Asp Met Leu Ile Lys Lys Leu Lys Ser Phe Asp Tyr His
   2420 2425 2430
   Gln Phe Val Asp Glu Thr Asn Asp Lys Ile Arg Glu Val Thr Gln
   2435 2440 2445
   Arg Leu Asn Gly Glu Ile Gln Ala Leu Glu Leu Pro Gln Lys Ala
   2450 2455 2460
   Glu Ala Leu Lys Leu Phe Leu Glu Glu Thr Lys Ala Thr Val Ala
   2465 2470 2475
   Val Tyr Leu Glu Ser Leu Gln Asp Thr Lys Ile Thr Leu Ile Ile
   2480 2485 2490
   Asn Trp Leu Gln Glu Ala Leu Ser Ser Ala Ser Leu Ala His Met
   2495 2500 2505
   Lys Ala Lys Phe Arg Glu Thr Leu Glu Asp Thr Arg Asp Arg Met
   2510 2515 2520
   Tyr Gln Met Asp Ile Gln Gln Glu Leu Gln Arg Tyr Leu Ser Leu
   2525 2530 2535
   Val Gly Gln Val Tyr Ser Thr Leu Val Thr Tyr Ile Ser Asp Trp
   2540 2545 2550
   Trp Thr Leu Ala Ala Lys Asn Leu Thr Asp Phe Ala Glu Gln Tyr
   2555 2560 2565
   Ser Ile Gln Asp Trp Ala Lys Arg Met Lys Ala Leu Val Glu Gln
   2570 2575 2580
   Gly Phe Thr Val Pro Glu Ile Lys Thr Ile Leu Gly Thr Met Pro
   2585 2590 2595
   Ala Phe Glu Val Ser Leu Gln Ala Leu Gln Lys Ala Thr Phe Gln
   2600 2605 2610
   Thr Pro  Asp Phe Ile Val Pro  Leu Thr Asp Leu Arg  Ile Pro Ser
       2615                 2620                 2625
   Val Gln  Ile Asn Phe Lys Asp  Leu Lys Asn Ile Lys  Ile Pro Ser
       2630                 2635                 2640
   Arg Phe  Ser Thr Pro Glu Phe  Thr Ile Leu Asn Thr  Phe His Ile
       2645                 2650                 2655
   Pro Ser  Phe Thr Ile Asp Phe  Val Glu Met Lys Val  Lys Ile Ile
       2660                 2665                 2670
   Arg Thr  Ile Asp Gln Met Gln  Asn Ser Glu Leu Gln  Trp Pro Val
       2675                 2680                 2685
   Pro Asp  Ile Tyr Leu Arg Asp  Leu Lys Val Glu Asp  Ile Pro Leu
       2690                 2695                 2700
   Ala Arg  Ile Thr Leu Pro Asp  Phe Arg Leu Pro Glu  Ile Ala Ile
       2705                 2710                 2715
   Pro Glu  Phe Ile Ile Pro Thr  Leu Asn Leu Asn Asp  Phe Gln Val
       2720                 2725                 2730
   Pro Asp  Leu His Ile Pro Glu  Phe Gln Leu Pro His  Ile Ser His
       2735                 2740                 2745
   Thr Ile  Glu Val Pro Thr Phe  Gly Lys Leu Tyr Ser  Ile Leu Lys
       2750                 2755                 2760
   Ile Gln  Ser Pro Leu Phe Thr  Leu Asp Ala Asn Ala  Asp Ile Gly
       2765                 2770                 2775
   Asn Gly  Thr Thr Ser Ala Asn  Glu Ala Gly Ile Ala  Ala Ser Ile
       2780                 2785                 2790
   Thr Ala  Lys Gly Glu Ser Lys  Leu Glu Val Leu Asn  Phe Asp Phe
       2795                 2800                 2805
   Gln Ala  Asn Ala Gln Leu Ser  Asn Pro Lys Ile Asn  Pro Leu Ala
       2810                 2815                 2820
   Leu Lys  Glu Ser Val Lys Phe  Ser Ser Lys Tyr Leu  Arg Thr Glu
       2825                 2830                 2835
   His Gly  Ser Glu Met Leu Phe  Phe Gly Asn Ala Ile  Glu Gly Lys
       2840                 2845                 2850
   Ser Asn  Thr Val Ala Ser Leu  His Thr Glu Lys Asn  Thr Leu Glu
       2855                 2860                 2865
   Leu Ser  Asn Gly Val Ile Val  Lys Ile Asn Asn Gln  Leu Thr Leu
   2870 2875 2880
   Asp Ser Asn Thr Lys Tyr Phe His Lys Leu Asn Ile Pro Lys Leu
   2885 2890 2895
   Asp Phe Ser Ser Gln Ala Asp Leu Arg Asn Glu Ile Lys Thr Leu
   2900 2905 2910
   Leu Lys Ala Gly His Ile Ala Trp Thr Ser Ser Gly Lys Gly Ser
   2915 2920 2925
   Trp Lys Trp Ala Cys Pro Arg Phe Ser Asp Glu Gly Thr His Glu
   2930 2935 2940
   Ser Gln Ile Ser Phe Thr Ile Glu Gly Pro Leu Thr Ser Phe Gly
   2945 2950 2955
   Leu Ser Asn Lys Ile Asn Ser Lys His Leu Arg Val Asn Gln Asn
   2960 2965 2970
   Leu Val Tyr Glu Ser Gly Ser Leu Asn Phe Ser Lys Leu Glu Ile
   2975 2980 2985
   Gln Ser Gln Val Asp Ser Gln His Val Gly His Ser Val Leu Thr
   2990 2995 3000
   Ala Lys Gly Met Ala Leu Phe Gly Glu Gly Lys Ala Glu Phe Thr
   3005 3010 3015
   Gly Arg His Asp Ala His Leu Asn Gly Lys Val Ile Gly Thr Leu
   3020 3025 3030
   Lys Asn Ser Leu Phe Phe Ser Ala Gln Pro Phe Glu Ile Thr Ala
   3035 3040 3045
   Ser Thr Asn Asn Glu Gly Asn Leu Lys Val Arg Phe Pro Leu Arg
   3050 3055 3060
   Leu Thr Gly Lys Ile Asp Phe Leu Asn Asn Tyr Ala Leu Phe Leu
   3065 3070 3075
   Ser Pro Ser Ala Gln Gln Ala Ser Trp Gln Val Ser Ala Arg Phe
   3080 3085 3090
   Asn Gln Tyr Lys Tyr Asn Gln Asn Phe Ser Ala Gly Asn Asn Glu
   3095 3100 3105
   Asn Ile Met Glu Ala His Val Gly Ile Asn Gly Glu Ala Asn Leu
   3110 3115 3120
   Asp Phe Leu Asn Ile Pro Leu Thr Ile Pro Glu Met Arg Leu Pro
   3125 3130 3135
   Tyr Thr Ile Ile Thr Thr Pro Pro Leu Lys Asp Phe Ser Leu Trp
   3140 3145 3150
   Glu Lys Thr Gly Leu Lys Glu Phe Leu Lys Thr Thr Lys Gln Ser
   3155 3160 3165
   Phe Asp Leu Ser Val Lys Ala Gln Tyr Lys Lys Asn Lys His Arg
   3170 3175 3180
   His Ser Ile Thr Asn Pro Leu Ala Val Leu Cys Glu Phe Ile Ser
   3185 3190 3195
   Gln Ser Ile Lys Ser Phe Asp Arg His Phe Glu Lys Asn Arg Asn
   3200 3205 3210
   Asn Ala Leu Asp Phe Val Thr Lys Ser Tyr Asn Glu Thr Lys Ile
   3215 3220 3225
   Lys Phe Asp Lys Tyr Lys Ala Glu Lys Ser His Asp Glu Leu Pro
   3230 3235 3240
   Arg Thr Phe Gln Ile Pro Gly Tyr Thr Val Pro Val Val Asn Val
   3245 3250 3255
   Glu Val Ser Pro Phe Thr Ile Glu Met Ser Ala Phe Gly Tyr Val
   3260 3265 3270
   Phe Pro Lys Ala Val Ser Met Pro Ser Phe Ser Ile Leu Gly Ser
   3275 3280 3285
   Asp Val Arg Val Pro Ser Tyr Thr Leu Ile Leu Pro Ser Leu Glu
   3290 3295 3300
   Leu Pro Val Leu His Val Pro Arg Asn Leu Lys Leu Ser Leu Pro
   3305 3310 3315
   His Phe Lys Glu Leu Cys Thr Ile Ser His Ile Phe Ile Pro Ala
   3320 3325 3330
   Met Gly Asn Ile Thr Tyr Asp Phe Ser Phe Lys Ser Ser Val Ile
   3335 3340 3345
   Thr Leu Asn Thr Asn Ala Glu Leu Phe Asn Gln Ser Asp Ile Val
   3350 3355 3360
   Ala His Leu Leu Ser Ser Ser Ser Ser Val Ile Asp Ala Leu Gln
   3365 3370 3375
   Tyr Lys Leu Glu Gly Thr Thr Arg Leu Thr Arg Lys Arg Gly Leu
   3380 3385 3390
   Lys Leu Ala Thr Ala Leu Ser Leu Ser Asn Lys Phe Val Glu Gly
   3395 3400 3405
   Ser His Asn Ser Thr Val Ser Leu Thr Thr Lys Asn Met Glu Val
   3410 3415 3420
   Ser Val Ala Lys Thr Thr Lys Ala Glu Ile Pro Ile Leu Arg Met
   3425 3430 3435
   Asn Phe Lys Gln Glu Leu Asn Gly Asn Thr Lys Ser Lys Pro Thr
   3440 3445 3450
   Val Ser Ser Ser Met Glu Phe Lys Tyr Asp Phe Asn Ser Ser Met
   3455 3460 3465
   Leu Tyr Ser Thr Ala Lys Gly Ala Val Asp His Lys Leu Ser Leu
   3470 3475 3480
   Glu Ser Leu Thr Ser Tyr Phe Ser Ile Glu Ser Ser Thr Lys Gly
   3485 3490 3495
   Asp Val Lys Gly Ser Val Leu Ser Arg Glu Tyr Ser Gly Thr Ile
   3500 3505 3510
   Ala Ser Glu Ala Asn Thr Tyr Leu Asn Ser Lys Ser Thr Arg Ser
   3515 3520 3525
   Ser Val Lys Leu Gln Gly Thr Ser Lys Ile Asp Asp Ile Trp Asn
   3530 3535 3540
   Leu Glu Val Lys Glu Asn Phe Ala Gly Glu Ala Thr Leu Gln Arg
   3545 3550 3555
   Ile Tyr Ser Leu Trp Glu His Ser Thr Lys Asn His Leu Gln Leu
   3560 3565 3570
   Glu Gly Leu Phe Phe Thr Asn Gly Glu His Thr Ser Lys Ala Thr
   3575 3580 3585
   Leu Glu Leu Ser Pro Trp Gln Met Ser Ala Leu Val Gln Val His
   3590 3595 3600
   Ala Ser Gln Pro Ser Ser Phe His Asp Phe Pro Asp Leu Gly Gln
   3605 3610 3615
   Glu Val Ala Leu Asn Ala Asn Thr Lys Asn Gln Lys Ile Arg Trp
   3620 3625 3630
   Lys Asn Glu Val Arg Ile His Ser Gly Ser Phe Gln Ser Gln Val
   3635 3640 3645
   Glu Leu Ser Asn Asp Gln Glu Lys Ala His Leu Asp Ile Ala Gly
   3650 3655 3660
   Ser Leu Glu Gly His Leu Arg Phe Leu Lys Asn Ile Ile Leu Pro
   3665 3670 3675
   Val Tyr Asp Lys Ser Leu Trp Asp Phe Leu Lys Leu Asp Val Thr
   3680 3685 3690
   Thr Ser Ile Gly Arg Arg Gln His Leu Arg Val Ser Thr Ala Phe
   3695 3700 3705
   Val Tyr Thr Lys Asn Pro Asn Gly Tyr Ser Phe Ser Ile Pro Val
   3710 3715 3720
   Lys Val Leu Ala Asp Lys Phe Ile Thr Pro Gly Leu Lys Leu Asn
   3725 3730 3735
   Asp Leu Asn Ser Val Leu Val Met Pro Thr Phe His Val Pro Phe
   3740 3745 3750
   Thr Asp Leu Gln Val Pro Ser Cys Lys Leu Asp Phe Arg Glu Ile
   3755 3760 3765
   Gln Ile Tyr Lys Lys Leu Arg Thr Ser Ser Phe Ala Leu Asn Leu
   3770 3775 3780
   Pro Thr Leu Pro Glu Val Lys Phe Pro Glu Val Asp Val Leu Thr
   3785 3790 3795
   Lys Tyr Ser Gln Pro Glu Asp Ser Leu Ile Pro Phe Phe Glu Ile
   3800 3805 3810
   Thr Val Pro Glu Ser Gln Leu Thr Val Ser Gln Phe Thr Leu Pro
   3815 3820 3825
   Lys Ser Val Ser Asp Gly Ile Ala Ala Leu Asp Leu Asn Ala Val
   3830 3835 3840
   Ala Asn Lys Ile Ala Asp Phe Glu Leu Pro Thr Ile Ile Val Pro
   3845 3850 3855
   Glu Gln Thr Ile Glu Ile Pro Ser Ile Lys Phe Ser Val Pro Ala
   3860 3865 3870
   Gly Ile Val Ile Pro Ser Phe Gln Ala Leu Thr Ala Arg Phe Glu
   3875 3880 3885
   Val Asp Ser Pro Val Tyr Asn Ala Thr Trp Ser Ala Ser Leu Lys
   3890 3895 3900
   Asn Lys Ala Asp Tyr Val Glu Thr Val Leu Asp Ser Thr Cys Ser
   3905 3910 3915
   Ser Thr Val Gln Phe Leu Glu Tyr Glu Leu Asn Val Leu Gly Thr
   3920 3925 3930
   His Lys Ile Glu Asp Gly Thr Leu Ala Ser Lys Thr Lys Gly Thr
   3935 3940 3945
   Leu Ala His Arg Asp Phe Ser Ala Glu Tyr Glu Glu Asp Gly Lys
   3950 3955 3960
   Phe Glu Gly Leu Gln Glu Trp Glu Gly Lys Ala His Leu Asn Ile
   3965 3970 3975
   Lys Ser Pro Ala Phe Thr Asp Leu His Leu Arg Tyr Gln Lys Asp
   3980 3985 3990
   Lys Lys Gly Ile Ser Thr Ser Ala Ala Ser Pro Ala Val Gly Thr
   3995 4000 4005
   Val Gly Met Asp Met Asp Glu Asp Asp Asp Phe Ser Lys Trp Asn
   4010 4015 4020
   Phe Tyr Tyr Ser Pro Gln Ser Ser Pro Asp Lys Lys Leu Thr Ile
   4025 4030 4035
   Phe Lys Thr Glu Leu Arg Val Arg Glu Ser Asp Glu Glu Thr Gln
   4040 4045 4050
   Ile Lys Val Asn Trp Glu Glu Glu Ala Ala Ser Gly Leu Leu Thr
   4055 4060 4065
   Ser Leu Lys Asp Asn Val Pro Lys Ala Thr Gly Val Leu Tyr Asp
   4070 4075 4080
   Tyr Val Asn Lys Tyr His Trp Glu His Thr Gly Leu Thr Leu Arg
   4085 4090 4095
   Glu Val Ser Ser Lys Leu Arg Arg Asn Leu Gln Asn Asn Ala Glu
   4100 4105 4110
   Trp Val Tyr Gln Gly Ala Ile Arg Gln Ile Asp Asp Ile Asp Val
   4115 4120 4125
   Arg Phe Gln Lys Ala Ala Ser Gly Thr Thr Gly Thr Tyr Gln Glu
   4130 4135 4140
   Trp Lys Asp Lys Ala Gln Asn Leu Tyr Gln Glu Leu Leu Thr Gln
   4145 4150 4155
   Glu Gly Gln Ala Ser Phe Gln Gly Leu Lys Asp Asn Val Phe Asp
   4160 4165 4170
   Gly Leu Val Arg Val Thr Gln Lys Phe His Met Lys Val Lys His
   4175 4180 4185
   Leu Ile Asp Ser Leu Ile Asp Phe Leu Asn Phe Pro Arg Phe Gln
   4190 4195 4200
   Phe Pro Gly Lys Pro Gly Ile Tyr Thr Arg Glu Glu Leu Cys Thr
   4205 4210 4215
   Met Phe Ile Arg Glu Val Gly Thr Val Leu Ser Gln Val Tyr Ser
   4220 4225 4230
   Lys Val His Asn Gly Ser Glu Ile Leu Phe Ser Tyr Phe Gln Asp
   4235 4240 4245
   Leu Val Ile Thr Leu Pro Phe Glu Leu Arg Lys His Lys Leu Ile
   4250 4255 4260
   Asp Val Ile Ser Met Tyr Arg Glu Leu Leu Lys Asp Leu Ser Lys
   4265 4270 4275
   Glu Ala Gln Glu Val Phe Lys Ala Ile Gln Ser Leu Lys Thr Thr
   4280 4285 4290
   Glu Val Leu Arg Asn Leu Gln Asp Leu Leu Gln Phe Ile Phe Gln
   4295 4300 4305
   Leu Ile Glu Asp Asn Ile Lys Gln Leu Lys Glu Met Lys Phe Thr
   4310 4315 4320
   Tyr Leu Ile Asn Tyr Ile Gln Asp Glu Ile Asn Thr Ile Phe Asn
   4325 4330 4335
   Asp Tyr Ile Pro Tyr Val Phe Lys Leu Leu Lys Glu Asn Leu Cys
   4340 4345 4350
   Leu Asn Leu His Lys Phe Asn Glu Phe Ile Gln Asn Glu Leu Gln
   4355 4360 4365
   Glu Ala Ser Gln Glu Leu Gln Gln Ile His Gln Tyr Ile Met Ala
   4370 4375 4380
   Leu Arg Glu Glu Tyr Phe Asp Pro Ser Ile Val Gly Trp Thr Val
   4385 4390 4395
   Lys Tyr Tyr Glu Leu Glu Glu Lys Ile Val Ser Leu Ile Lys Asn
   4400 4405 4410
   Leu Leu Val Ala Leu Lys Asp Phe His Ser Glu Tyr Ile Val Ser
   4415 4420 4425
   Ala Ser Asn Phe Thr Ser Gln Leu Ser Ser Gln Val Glu Gln Phe
   4430 4435 4440
   Leu His Arg Asn Ile Gln Glu Tyr Leu Ser Ile Leu Thr Asp Pro
   4445 4450 4455
   Asp Gly Lys Gly Lys Glu Lys Ile Ala Glu Leu Ser Ala Thr Ala
   4460 4465 4470
   Gln Glu Ile Ile Lys Ser Gln Ala Ile Ala Thr Lys Lys Ile Ile
   4475 4480 4485
   Ser Asp Tyr His Gln Gln Phe Arg Tyr Lys Leu Gln Asp Phe Ser
   4490 4495 4500
   Asp Gln Leu Ser Asp Tyr Tyr Glu Lys Phe Ile Ala Glu Ser Lys
   4505 4510 4515
   Arg Leu Ile Asp Leu Ser Ile Gln Asn Tyr His Thr Phe Leu Ile
   4520 4525 4530
   Tyr Ile Thr Glu Leu Leu Lys Lys Leu Gln Ser Thr Thr Val Met
   4535 4540 4545
   Asn Pro Tyr Met Lys Leu Ala Pro Gly Glu Leu Thr Ile Ile Leu
   4550 4555 4560
<210> 42
   <211> 930
   <212> PRT
   <213> Homo sapiens
<300>
   <308> Swiss-Prot/Q14624
   <309> 1998-07-15
   <313> (1)..(930)
<400> 42
<210> 43
   <211> 1744
   <212> PRT
   <213> Homo sapiens
<300>
   <308> Swiss-Prot/P01028
   <309> 1986-07-21
   <313> (1)..(1744)
<400> 43
   Met Arg Leu Leu Trp Gly Leu Ile Trp Ala Ser Ser Phe Phe Thr Leu
   1 5 10 15
   Ser Leu Gln Lys Pro Arg Leu Leu Leu Phe Ser Pro Ser Val Val His
   20 25 30
   Leu Gly Val Pro Leu Ser Val Gly Val Gln Leu Gln Asp Val Pro Arg
   35 40 45
   Gly Gln Val Val Lys Gly Ser Val Phe Leu Arg Asn Pro Ser Arg Asn
   50 55 60
   Asn Val Pro Cys Ser Pro Lys Val Asp Phe Thr Leu Ser Ser Glu Arg
   65 70 75 80
   Asp Phe Ala Leu Leu Ser Leu Gln Val Pro Leu Lys Asp Ala Lys Ser
   85 90 95
   Cys Gly Leu His Gln Leu Leu Arg Gly Pro Glu Val Gln Leu Val Ala
   100 105 110
   His Ser Pro Trp Leu Lys Asp Ser Leu Ser Arg Thr Thr Asn Ile Gln
   115 120 125
   Gly Ile Asn Leu Leu Phe Ser Ser Arg Arg Gly His Leu Phe Leu Gln
   130 135 140
   Thr Asp Gln Pro Ile Tyr Asn Pro Gly Gln Arg Val Arg Tyr Arg Val
   145 150 155 160
   Phe Ala Leu Asp Gln Lys Met Arg Pro Ser Thr Asp Thr Ile Thr Val
   165 170 175
   Met Val Glu Asn Ser His Gly Leu Arg Val Arg Lys Lys Glu Val Tyr
   180 185 190
   Met Pro Ser Ser Ile Phe Gln Asp Asp Phe Val Ile Pro Asp Ile Ser
   195 200 205
   Glu Pro Gly Thr Trp Lys Ile Ser Ala Arg Phe Ser Asp Gly Leu Glu
   210 215 220
   Ser Asn Ser Ser Thr Gln Phe Glu Val Lys Lys Tyr Val Leu Pro Asn
   225 230 235 240
   Phe Glu Val Lys Ile Thr Pro Gly Lys Pro Tyr Ile Leu Thr Val Pro
                   245                 250                 255
   Gly His Leu Asp Glu Met Gln Leu Asp Ile Gln Ala Arg Tyr Ile Tyr
               260                 265                 270
   Gly Lys Pro Val Gln Gly Val Ala Tyr Val Arg Phe Gly Leu Leu Asp
           275                 280                 285
   Glu Asp Gly Lys Lys Thr Phe Phe Arg Gly Leu Glu Ser Gln Thr Lys
       290                 295                 300
   Leu Val Asn Gly Gln Ser His Ile Ser Leu Ser Lys Ala Glu Phe Gln
   305                 310                 315                 320
   Asp Ala Leu Glu Lys Leu Asn Met Gly Ile Thr Asp Leu Gln Gly Leu
                   325                 330                 335
   Arg Leu Tyr Val Ala Ala Ala Ile Ile Glu Ser Pro Gly Gly Glu Met
               340                 345                 350
   Glu Glu Ala Glu Leu Thr Ser Trp Tyr Phe Val Ser Ser Pro Phe Ser
           355                 360                 365
   Leu Asp Leu Ser Lys Thr Lys Arg His Leu Val Pro Gly Ala Pro Phe
       370                 375                 380
   Leu Leu Gln Ala Leu Val Arg Glu Met Ser Gly Ser Pro Ala Ser Gly
   385                 390                 395                 400
   Ile Pro Val Lys Val Ser Ala Thr Val Ser Ser Pro Gly Ser Val Pro
                   405                 410                 415
   Glu Val Gln Asp Ile Gln Gln Asn Thr Asp Gly Ser Gly Gln Val Ser
               420                 425                 430
   Ile Pro Ile Ile Ile Pro Gln Thr Ile Ser Glu Leu Gln Leu Ser Val
           435                 440                 445
   Ser Ala Gly Ser Pro His Pro Ala Ile Ala Arg Leu Thr Val Ala Ala
       450                 455                 460
   Pro Pro Ser Gly Gly Pro Gly Phe Leu Ser Ile Glu Arg Pro Asp Ser
   465                 470                 475                 480
   Arg Pro Pro Arg Val Gly Asp Thr Leu Asn Leu Asn Leu Arg Ala Val
                   485                 490                 495
   Gly Ser Gly Ala Thr Phe Ser His Tyr Tyr Tyr Met Ile Leu Ser Arg
               500                 505                 510
   Gly Gln Ile Val Phe Met Asn Arg Glu Pro Lys Arg Thr Leu Thr Ser
   515 520 525
   Val Ser Val Phe Val Asp His His Leu Ala Pro Ser Phe Tyr Phe Val
   530 535 540
   Ala Phe Tyr Tyr His Gly Asp His Pro Val Ala Asn Ser Leu Arg Val
   545 550 555 560
   Asp Val Gln Ala Gly Ala Cys Glu Gly Lys Leu Glu Leu Ser Val Asp
   565 570 575
   Gly Ala Lys Gln Tyr Arg Asn Gly Glu Ser Val Lys Leu His Leu Glu
   580 585 590
   Thr Asp Ser Leu Ala Leu Val Ala Leu Gly Ala Leu Asp Thr Ala Leu
   595 600 605
   Tyr Ala Ala Gly Ser Lys Ser His Lys Pro Leu Asn Met Gly Lys Val
   610 615 620
   Phe Glu Ala Met Asn Ser Tyr Asp Leu Gly Cys Gly Pro Gly Gly Gly
   625 630 635 640
   Asp Ser Ala Leu Gln Val Phe Gln Ala Ala Gly Leu Ala Phe Ser Asp
   645 650 655
   Gly Asp Gln Trp Thr Leu Ser Arg Lys Arg Leu Ser Cys Pro Lys Glu
   660 665 670
   Lys Thr Thr Arg Lys Lys Arg Asn Val Asn Phe Gln Lys Ala Ile Asn
   675 680 685
   Glu Lys Leu Gly Gln Tyr Ala Ser Pro Thr Ala Lys Arg Cys Cys Gln
   690 695 700
   Asp Gly Val Thr Arg Leu Pro Met Met Arg Ser Cys Glu Gln Arg Ala
   705 710 715 720
   Ala Arg Val Gln Gln Pro Asp Cys Arg Glu Pro Phe Leu Ser Cys Cys
   725 730 735
   Gln Phe Ala Glu Ser Leu Arg Lys Lys Ser Arg Asp Lys Gly Gln Ala
   740 745 750
   Gly Leu Gln Arg Ala Leu Glu Ile Leu Gln Glu Glu Asp Leu Ile Asp
   755 760 765
   Glu Asp Asp Ile Pro Val Arg Ser Phe Phe Pro Glu Asn Trp Leu Trp
   770 775 780
   Arg Val Glu Thr Val Asp Arg Phe Gln Ile Leu Thr Leu Trp Leu Pro
   785 790 795 800
   Asp Ser Leu Thr Thr Trp Glu Ile His Gly Leu Ser Leu Ser Lys Thr
   805 810 815
   Lys Gly Leu Cys Val Ala Thr Pro Val Gln Leu Arg Val Phe Arg Glu
   820 825 830
   Phe His Leu His Leu Arg Leu Pro Met Ser Val Arg Arg Phe Glu Gln
   835 840 845
   Leu Glu Leu Arg Pro Val Leu Tyr Asn Tyr Leu Asp Lys Asn Leu Thr
   850 855 860
   Val Ser Val His Val Ser Pro Val Glu Gly Leu Cys Leu Ala Gly Gly
   865 870 875 880
   Gly Gly Leu Ala Gln Gln Val Leu Val Pro Ala Gly Ser Ala Arg Pro
   885 890 895
   Val Ala Phe Ser Val Val Pro Thr Ala Ala Ala Ala Val Ser Leu Lys
   900 905 910
   Val Val Ala Arg Gly Ser Phe Glu Phe Pro Val Gly Asp Ala Val Ser
   915 920 925
   Lys Val Leu Gln Ile Glu Lys Glu Gly Ala Ile His Arg Glu Glu Leu
   930 935 940
   Val Tyr Glu Leu Asn Pro Leu Asp His Arg Gly Arg Thr Leu Glu Ile
   945 950 955 960
   Pro Gly Asn Ser Asp Pro Asn Met Ile Pro Asp Gly Asp Phe Asn Ser
   965 970 975
   Tyr Val Arg Val Thr Ala Ser Asp Pro Leu Asp Thr Leu Gly Ser Glu
   980 985 990
   Gly Ala Leu Ser Pro Gly Gly Val Ala Ser Leu Leu Arg Leu Pro Arg
   995 1000 1005
   Gly Cys Gly Glu Gln Thr Met Ile Tyr Leu Ala Pro Thr Leu Ala
   1010 1015 1020
   Ala Ser Arg Tyr Leu Asp Lys Thr Glu Gln Trp Ser Thr Leu Pro
   1025 1030 1035
   Pro Glu Thr Lys Asp His Ala Val Asp Leu Ile Gln Lys Gly Tyr
   1040 1045 1050
   Met Arg Ile Gln Gln Phe Arg Lys Ala Asp Gly Ser Tyr Ala Ala
   1055 1060 1065
   Trp Leu Ser Arg Asp Ser Ser Thr Trp Leu Thr Ala Phe Val Leu
   1070 1075 1080
   Lys Val Leu Ser Leu Ala Gln Glu Gln Val Gly Gly Ser Pro Glu
   1085 1090 1095
   Lys Leu Gln Glu Thr Ser Asn Trp Leu Leu Ser Gln Gln Gln Ala
   1100 1105 1110
   Asp Gly Ser Phe Gln Asp Pro Cys Pro Val Leu Asp Arg Ser Met
   1115 1120 1125
   Gln Gly Gly Leu Val Gly Asn Asp Glu Thr Val Ala Leu Thr Ala
   1130 1135 1140
   Phe Val Thr Ile Ala Leu His His Gly Leu Ala Val Phe Gln Asp
   1145 1150 1155
   Glu Gly Ala Glu Pro Leu Lys Gln Arg Val Glu Ala Ser Ile Ser
   1160 1165 1170
   Lys Ala Asn Ser Phe Leu Gly Glu Lys Ala Ser Ala Gly Leu Leu
   1175 1180 1185
   Gly Ala His Ala Ala Ala Ile Thr Ala Tyr Ala Leu Ser Leu Thr
   1190 1195 1200
   Lys Ala Pro Val Asp Leu Leu Gly Val Ala His Asn Asn Leu Met
   1205 1210 1215
   Ala Met Ala Gln Glu Thr Gly Asp Asn Leu Tyr Trp Gly Ser Val
   1220 1225 1230
   Thr Gly Ser Gln Ser Asn Ala Val Ser Pro Thr Pro Ala Pro Arg
   1235 1240 1245
   Asn Pro Ser Asp Pro Met Pro Gln Ala Pro Ala Leu Trp Ile Glu
   1250 1255 1260
   Thr Thr Ala Tyr Ala Leu Leu His Leu Leu Leu His Glu Gly Lys
   1265 1270 1275
   Ala Glu Met Ala Asp Gln Ala Ser Ala Trp Leu Thr Arg Gln Gly
   1280 1285 1290
   Ser Phe Gln Gly Gly Phe Arg Ser Thr Gln Asp Thr Val Ile Ala
   1295 1300 1305
   Leu Asp Ala Leu Ser Ala Tyr Trp Ile Ala Ser His Thr Thr Glu
   1310 1315 1320
   Glu Arg Gly Leu Asn Val Thr Leu Ser Ser Thr Gly Arg Asn Gly
   1325 1330 1335
   Phe Lys Ser His Ala Leu Gln Leu Asn Asn Arg Gln Ile Arg Gly
   1340 1345 1350
   Leu Glu Glu Glu Leu Gln Phe Ser Leu Gly Ser Lys Ile Asn Val
   1355 1360 1365
   Lys Val Gly Gly Asn Ser Lys Gly Thr Leu Lys Val Leu Arg Thr
   1370 1375 1380
   Tyr Asn Val Leu Asp Met Lys Asn Thr Thr Cys Gln Asp Leu Gln
   1385 1390 1395
   Ile Glu Val Thr Val Lys Gly His Val Glu Tyr Thr Met Glu Ala
   1400 1405 1410
   Asn Glu Asp Tyr Glu Asp Tyr Glu Tyr Asp Glu Leu Pro Ala Lys
   1415 1420 1425
   Asp Asp Pro Asp Ala Pro Leu Gln Pro Val Thr Pro Leu Gln Leu
   1430 1435 1440
   Phe Glu Gly Arg Arg Asn Arg Arg Arg Arg Glu Ala Pro Lys Val
   1445 1450 1455
   Val Glu Glu Gln Glu Ser Arg Val His Tyr Thr Val Cys Ile Trp
   1460 1465 1470
   Arg Asn Gly Lys Val Gly Leu Ser Gly Met Ala Ile Ala Asp Val
   1475 1480 1485
   Thr Leu Leu Ser Gly Phe His Ala Leu Arg Ala Asp Leu Glu Lys
   1490 1495 1500
   Leu Thr Ser Leu Ser Asp Arg Tyr Val Ser His Phe Glu Thr Glu
   1505 1510 1515
   Gly Pro His Val Leu Leu Tyr Phe Asp Ser Val Pro Thr Ser Arg
   1520 1525 1530
   Glu Cys Val Gly Phe Glu Ala Val Gln Glu Val Pro Val Gly Leu
   1535 1540 1545
   Val Gln Pro Ala Ser Ala Thr Leu Tyr Asp Tyr Tyr Asn Pro Glu
   1550 1555 1560
   Arg Arg Cys Ser Val Phe Tyr Gly Ala Pro Ser Lys Ser Arg Leu
   1565 1570 1575
   Leu Ala Thr Leu Cys Ser Ala Glu Val Cys Gln Cys Ala Glu Gly
   1580 1585 1590
   Lys Cys Pro Arg Gln Arg Arg Ala Leu Glu Arg Gly Leu Gln Asp
   1595 1600 1605
   Glu Asp Gly Tyr Arg Met Lys Phe Ala Cys Tyr Tyr Pro Arg Val
   1610 1615 1620
   Glu Tyr Gly Phe Gln Val Lys Val Leu Arg Glu Asp Ser Arg Ala
   1625 1630 1635
   Ala Phe Arg Leu Phe Glu Thr Lys Ile Thr Gln Val Leu His Phe
   1640 1645 1650
   Thr Lys Asp Val Lys Ala Ala Ala Asn Gln Met Arg Asn Phe Leu
   1655 1660 1665
   Val Arg Ala Ser Cys Arg Leu Arg Leu Glu Pro Gly Lys Glu Tyr
   1670 1675 1680
   Leu Ile Met Gly Leu Asp Gly Ala Thr Tyr Asp Leu Glu Gly His
   1685 1690 1695
   Pro Gln Tyr Leu Leu Asp Ser Asn Ser Trp Ile Glu Glu Met Pro
   1700 1705 1710
   Ser Glu Arg Leu Cys Arg Ser Thr Arg Gln Arg Ala Ala Cys Ala
   1715 1720 1725
   Gln Leu Asn Asp Phe Leu Gln Glu Tyr Gly Thr Gln Gly Cys Gln
   1730 1735 1740
   Val
<210> 44
   <211> 1663
   <212> PRT
   <213> Homo sapiens
<300>
   <308> Swiss-Prot/P01024
   <309> 1986-07-21
   <313> (1)..(1663)
<400> 44
   Met Gly Pro Thr Ser Gly Pro Ser Leu Leu Leu Leu Leu Leu Thr His
   1               5                   10                  15
   Leu Pro Leu Ala Leu Gly Ser Pro Met Tyr Ser Ile Ile Thr Pro Asn
               20                  25                  30
   Ile Leu Arg Leu Glu Ser Glu Glu Thr Met Val Leu Glu Ala His Asp
           35                  40                  45
   Ala Gln Gly Asp Val Pro Val Thr Val Thr Val His Asp Phe Pro Gly
       50                  55                  60
   Lys Lys Leu Val Leu Ser Ser Glu Lys Thr Val Leu Thr Pro Ala Thr
   65 70 75 80
   Asn His Met Gly Asn Val Thr Phe Thr Ile Pro Ala Asn Arg Glu Phe
   85 90 95
   Lys Ser Glu Lys Gly Arg Asn Lys Phe Val Thr Val Gln Ala Thr Phe
   100 105 110
   Gly Thr Gln Val Val Glu Lys Val Val Leu Val Ser Leu Gln Ser Gly
   115 120 125
   Tyr Leu Phe Ile Gln Thr Asp Lys Thr Ile Tyr Thr Pro Gly Ser Thr
   130 135 140
   Val Leu Tyr Arg Ile Phe Thr Val Asn His Lys Leu Leu Pro Val Gly
   145 150 155 160
   Arg Thr Val Met Val Asn Ile Glu Asn Pro Glu Gly Ile Pro Val Lys
   165 170 175
   Gln Asp Ser Leu Ser Ser Gln Asn Gln Leu Gly Val Leu Pro Leu Ser
   180 185 190
   Trp Asp Ile Pro Glu Leu Val Asn Met Gly Gln Trp Lys Ile Arg Ala
   195 200 205
   Tyr Tyr Glu Asn Ser Pro Gln Gln Val Phe Ser Thr Glu Phe Glu Val
   210 215 220
   Lys Glu Tyr Val Leu Pro Ser Phe Glu Val Ile Val Glu Pro Thr Glu
   225 230 235 240
   Lys Phe Tyr Tyr Ile Tyr Asn Glu Lys Gly Leu Glu Val Thr Ile Thr
   245 250 255
   Ala Arg Phe Leu Tyr Gly Lys Lys Val Glu Gly Thr Ala Phe Val Ile
   260 265 270
   Phe Gly Ile Gln Asp Gly Glu Gln Arg Ile Ser Leu Pro Glu Ser Leu
   275 280 285
   Lys Arg Ile Pro Ile Glu Asp Gly Ser Gly Glu Val Val Leu Ser Arg
   290 295 300
   Lys Val Leu Leu Asp Gly Val Gln Asn Leu Arg Ala Glu Asp Leu Val
   305 310 315 320
   Gly Lys Ser Leu Tyr Val Ser Ala Thr Val Ile Leu His Ser Gly Ser
   325 330 335
   Asp Met Val Gln Ala Glu Arg Ser Gly Ile Pro Ile Val Thr Ser Pro
   340 345 350
   Tyr Gln Ile His Phe Thr Lys Thr Pro Lys Tyr Phe Lys Pro Gly Met
   355 360 365
   Pro Phe Asp Leu Met Val Phe Val Thr Asn Pro Asp Gly Ser Pro Ala
   370 375 380
   Tyr Arg Val Pro Val Ala Val Gln Gly Glu Asp Thr Val Gln Ser Leu
   385 390 395 400
   Thr Gln Gly Asp Gly Val Ala Lys Leu Ser Ile Asn Thr His Pro Ser
   405 410 415
   Gln Lys Pro Leu Ser Ile Thr Val Arg Thr Lys Lys Gln Glu Leu Ser
   420 425 430
   Glu Ala Glu Gln Ala Thr Arg Thr Met Gln Ala Leu Pro Tyr Ser Thr
   435 440 445
   Val Gly Asn Ser Asn Asn Tyr Leu His Leu Ser Val Leu Arg Thr Glu
   450 455 460
   Leu Arg Pro Gly Glu Thr Leu Asn Val Asn Phe Leu Leu Arg Met Asp
   465 470 475 480
   Arg Ala His Glu Ala Lys Ile Arg Tyr Tyr Thr Tyr Leu Ile Met Asn
   485 490 495
   Lys Gly Arg Leu Leu Lys Ala Gly Arg Gln Val Arg Glu Pro Gly Gln
   500 505 510
   Asp Leu Val Val Leu Pro Leu Ser Ile Thr Thr Asp Phe Ile Pro Ser
   515 520 525
   Phe Arg Leu Val Ala Tyr Tyr Thr Leu Ile Gly Ala Ser Gly Gln Arg
   530 535 540
   Glu Val Val Ala Asp Ser Val Trp Val Asp Val Lys Asp Ser Cys Val
   545 550 555 560
   Gly Ser Leu Val Val Lys Ser Gly Gln Ser Glu Asp Arg Gln Pro Val
   565 570 575
   Pro Gly Gln Gln Met Thr Leu Lys Ile Glu Gly Asp His Gly Ala Arg
   580 585 590
   Val Val Leu Val Ala Val Asp Lys Gly Val Phe Val Leu Asn Lys Lys
   595 600 605
   Asn Lys Leu Thr Gln Ser Lys Ile Trp Asp Val Val Glu Lys Ala Asp
   610 615 620
   Ile Gly Cys Thr Pro Gly Ser Gly Lys Asp Tyr Ala Gly Val Phe Ser
   625 630 635 640
   Asp Ala Gly Leu Thr Phe Thr Ser Ser Ser Gly Gln Gln Thr Ala Gln
   645 650 655
   Arg Ala Glu Leu Gln Cys Pro Gln Pro Ala Ala Arg Arg Arg Arg Ser
   660 665 670
   Val Gln Leu Thr Glu Lys Arg Met Asp Lys Val Gly Lys Tyr Pro Lys
   675 680 685
   Glu Leu Arg Lys Cys Cys Glu Asp Gly Met Arg Glu Asn Pro Met Arg
   690 695 700
   Phe Ser Cys Gln Arg Arg Thr Arg Phe Ile Ser Leu Gly Glu Ala Cys
   705 710 715 720
   Lys Lys Val Phe Leu Asp Cys Cys Asn Tyr Ile Thr Glu Leu Arg Arg
   725 730 735
   Gln His Ala Arg Ala Ser His Leu Gly Leu Ala Arg Ser Asn Leu Asp
   740 745 750
   Glu Asp Ile Ile Ala Glu Glu Asn Ile Val Ser Arg Ser Glu Phe Pro
   755 760 765
   Glu Ser Trp Leu Trp Asn Val Glu Asp Leu Lys Glu Pro Pro Lys Asn
   770 775 780
   Gly Ile Ser Thr Lys Leu Met Asn Ile Phe Leu Lys Asp Ser Ile Thr
   785 790 795 800
   Thr Trp Glu Ile Leu Ala Val Ser Met Ser Asp Lys Lys Gly Ile Cys
   805 810 815
   Val Ala Asp Pro Phe Glu Val Thr Val Met Gln Asp Phe Phe Ile Asp
   820 825 830
   Leu Arg Leu Pro Tyr Ser Val Val Arg Asn Glu Gln Val Glu Ile Arg
   835 840 845
   Ala Val Leu Tyr Asn Tyr Arg Gln Asn Gln Glu Leu Lys Val Arg Val
   850 855 860
   Glu Leu Leu His Asn Pro Ala Phe Cys Ser Leu Ala Thr Thr Lys Arg
   865 870 875 880
   Arg His Gln Gln Thr Val Thr Ile Pro Pro Lys Ser Ser Leu Ser Val
   885 890 895
   Pro Tyr Val Ile Val Pro Leu Lys Thr Gly Leu Gln Glu Val Glu Val
   900 905 910
   Lys Ala Ala Val Tyr His His Phe Ile Ser Asp Gly Val Arg Lys Ser
   915 920 925
   Leu Lys Val Val Pro Glu Gly Ile Arg Met Asn Lys Thr Val Ala Val
   930 935 940
   Arg Thr Leu Asp Pro Glu Arg Leu Gly Arg Glu Gly Val Gln Lys Glu
   945 950 955 960
   Asp Ile Pro Pro Ala Asp Leu Ser Asp Gln Val Pro Asp Thr Glu Ser
   965 970 975
   Glu Thr Arg Ile Leu Leu Gln Gly Thr Pro Val Ala Gln Met Thr Glu
   980 985 990
   Asp Ala Val Asp Ala Glu Arg Leu Lys His Leu Ile Val Thr Pro Ser
   995 1000 1005
   Gly Cys Gly Glu Gln Asn Met Ile Gly Met Thr Pro Thr Val Ile
   1010 1015 1020
   Ala Val His Tyr Leu Asp Glu Thr Glu Gln Trp Glu Lys Phe Gly
   1025 1030 1035
   Leu Glu Lys Arg Gln Gly Ala Leu Glu Leu Ile Lys Lys Gly Tyr
   1040 1045 1050
   Thr Gln Gln Leu Ala Phe Arg Gln Pro Ser Ser Ala Phe Ala Ala
   1055 1060 1065
   Phe Val Lys Arg Ala Pro Ser Thr Trp Leu Thr Ala Tyr Val Val
   1070 1075 1080
   Lys Val Phe Ser Leu Ala Val Asn Leu Ile Ala Ile Asp Ser Gln
   1085 1090 1095
   Val Leu Cys Gly Ala Val Lys Trp Leu Ile Leu Glu Lys Gln Lys
   1100 1105 1110
   Pro Asp Gly Val Phe Gln Glu Asp Ala Pro Val Ile His Gln Glu
   1115 1120 1125
   Met Ile Gly Gly Leu Arg Asn Asn Asn Glu Lys Asp Met Ala Leu
   1130 1135 1140
   Thr Ala Phe Val Leu Ile Ser Leu Gln Glu Ala Lys Asp Ile Cys
   1145 1150 1155
   Glu Glu Gln Val Asn Ser Leu Pro Gly Ser Ile Thr Lys Ala Gly
   1160 1165 1170
   Asp Phe Leu Glu Ala Asn Tyr Met Asn Leu Gln Arg Ser Tyr Thr
   1175 1180 1185
   Val Ala Ile Ala Gly Tyr Ala Leu Ala Gln Met Gly Arg Leu Lys
   1190 1195 1200
   Gly Pro Leu Leu Asn Lys Phe Leu Thr Thr Ala Lys Asp Lys Asn
   1205 1210 1215
   Arg Trp Glu Asp Pro Gly Lys Gln Leu Tyr Asn Val Glu Ala Thr
   1220 1225 1230
   Ser Tyr Ala Leu Leu Ala Leu Leu Gln Leu Lys Asp Phe Asp Phe
   1235 1240 1245
   Val Pro Pro Val Val Arg Trp Leu Asn Glu Gln Arg Tyr Tyr Gly
   1250 1255 1260
   Gly Gly Tyr Gly Ser Thr Gln Ala Thr Phe Met Val Phe Gln Ala
   1265 1270 1275
   Leu Ala Gln Tyr Gln Lys Asp Ala Pro Asp His Gln Glu Leu Asn
   1280 1285 1290
   Leu Asp Val Ser Leu Gln Leu Pro Ser Arg Ser Ser Lys Ile Thr
   1295 1300 1305
   His Arg Ile His Trp Glu Ser Ala Ser Leu Leu Arg Ser Glu Glu
   1310 1315 1320
   Thr Lys Glu Asn Glu Gly Phe Thr Val Thr Ala Glu Gly Lys Gly
   1325 1330 1335
   Gln Gly Thr Leu Ser Val Val Thr Met Tyr His Ala Lys Ala Lys
   1340 1345 1350
   Asp Gln Leu Thr Cys Asn Lys Phe Asp Leu Lys Val Thr Ile Lys
   1355 1360 1365
   Pro Ala Pro Glu Thr Glu Lys Arg Pro Gln Asp Ala Lys Asn Thr
   1370 1375 1380
   Met Ile Leu Glu Ile Cys Thr Arg Tyr Arg Gly Asp Gln Asp Ala
   1385 1390 1395
   Thr Met Ser Ile Leu Asp Ile Ser Met Met Thr Gly Phe Ala Pro
   1400 1405 1410
   Asp Thr Asp Asp Leu Lys Gln Leu Ala Asn Gly Val Asp Arg Tyr
   1415 1420 1425
   Ile Ser Lys Tyr Glu Leu Asp Lys Ala Phe Ser Asp Arg Asn Thr
   1430 1435 1440
   Leu Ile Ile Tyr Leu Asp Lys Val Ser His Ser Glu Asp Asp Cys
   1445 1450 1455
   Leu Ala Phe Lys Val His Gln Tyr Phe Asn Val Glu Leu Ile Gln
   1460 1465 1470
   Pro Gly Ala Val Lys Val Tyr Ala Tyr Tyr Asn Leu Glu Glu Ser
   1475 1480 1485
   Cys Thr Arg Phe Tyr His Pro Glu Lys Glu Asp Gly Lys Leu Asn
   1490 1495 1500
   Lys Leu Cys Arg Asp Glu Leu Cys Arg Cys Ala Glu Glu Asn Cys
   1505 1510 1515
   Phe Ile Gln Lys Ser Asp Asp Lys Val Thr Leu Glu Glu Arg Leu
   1520 1525 1530
   Asp Lys Ala Cys Glu Pro Gly Val Asp Tyr Val Tyr Lys Thr Arg
   1535 1540 1545
   Leu Val Lys Val Gln Leu Ser Asn Asp Phe Asp Glu Tyr Ile Met
   1550 1555 1560
   Ala Ile Glu Gln Thr Ile Lys Ser Gly Ser Asp Glu Val Gln Val
   1565 1570 1575
   Gly Gln Gln Arg Thr Phe Ile Ser Pro Ile Lys Cys Arg Glu Ala
   1580 1585 1590
   Leu Lys Leu Glu Glu Lys Lys His Tyr Leu Met Trp Gly Leu Ser
   1595 1600 1605
   Ser Asp Phe Trp Gly Glu Lys Pro Asn Leu Ser Tyr Ile Ile Gly
   1610 1615 1620
   Lys Asp Thr Trp Val Glu His Trp Pro Glu Glu Asp Glu Cys Gln
   1625 1630 1635
   Asp Glu Glu Asn Gln Lys Gln Cys Gln Asp Leu Gly Ala Phe Thr
   1640 1645 1650
   Glu Ser Met Val Val Phe Gly Cys Pro Asn
   1655 1660
<210> 45
   <211> 93
   <212> PRT
   <213> Homo sapiens
<300>
   <308> Swiss-Prot/Q9H299
   <309> 2003-02-28
   <313> (1)..(93)
<400> 45
   Met Ser Gly Leu Arg Val Tyr Ser Thr Ser Val Thr Gly Ser Arg Glu
   1 5 10 15
   Ile Lys Ser Gln Gln Ser Glu Val Thr Arg Ile Leu Asp Gly Lys Arg
   20 25 30
   Ile Gln Tyr Gln Leu Val Asp Ile Ser Gln Asp Asn Ala Leu Arg Asp
   35 40 45
   Glu Met Arg Ala Leu Ala Gly Asn Pro Lys Ala Thr Pro Pro Gln Ile
   50 55 60
   Val Asn Gly Asp Gln Tyr Cys Gly Asp Tyr Glu Leu Phe Val Glu Ala
   65 70 75 80
   Val Glu Gln Asn Thr Leu Gln Glu Phe Leu Lys Leu Ala
   85 90
<210> 46
   <211> 567
   <212> PRT
   <213> Homo sapiens
<300>
   <308> swiss-Prot/Q96RQ9
   <309> 2003-02-28
   <313> (1)..(567)
<400> 46
   Met Ala Pro Leu Ala Leu His Leu Leu Val Leu Val Pro Ile Leu Leu
   1 5 10 15
   Ser Leu Val Ala Ser Gln Asp Trp Lys Ala Glu Arg Ser Gln Asp Pro
   20 25 30
   Phe Glu Lys Cys Met Gln Asp Pro Asp Tyr Glu Gln Leu Leu Lys Val
   35 40 45
   Val Thr Trp Gly Leu Asn Arg Thr Leu Lys Pro Gln Arg Val Ile Val
   50 55 60
   Val Gly Ala Gly Val Ala Gly Leu Val Ala Ala Lys Val Leu Ser Asp
   65 70 75 80
   Ala Gly His Lys Val Thr Ile Leu Glu Ala Asp Asn Arg Ile Gly Gly
   85 90 95
   Arg Ile Phe Thr Tyr Arg Asp Gln Asn Thr Gly Trp Ile Gly Glu Leu
   100 105 110
   Gly Ala Met Arg Met Pro Ser Ser His Arg Ile Leu His Lys Leu Cys
   115 120 125
   Gln Gly Leu Gly Leu Asn Leu Thr Lys Phe Thr Gln Tyr Asp Lys Asn
   130 135 140
   Thr Trp Thr Glu Val His Glu Val Lys Leu Arg Asn Tyr Val Val Glu
   145 150 155 160
   Lys Val Pro Glu Lys Leu Gly Tyr Ala Leu Arg Pro Gln Glu Lys Gly
   165 170 175
   His Ser Pro Glu Asp Ile Tyr Gln Met Ala Leu Asn Gln Ala Leu Lys
   180 185 190
   Asp Leu Lys Ala Leu Gly Cys Arg Lys Ala Met Lys Lys Phe Glu Arg
   195 200 205
   His Thr Leu Leu Glu Tyr Leu Leu Gly Glu Gly Asn Leu Ser Arg Pro
   210 215 220
   Ala Val Gln Leu Leu Gly Asp Val Met Ser Glu Asp Gly Phe Phe Tyr
   225 230 235 240
   Leu Ser Phe Ala Glu Ala Leu Arg Ala His Ser Cys Leu Ser Asp Arg
   245 250 255
   Leu Gln Tyr Ser Arg Ile Val Gly Gly Trp Asp Leu Leu Pro Arg Ala
   260 265 270
   Leu Leu Ser Ser Leu Ser Gly Leu Val Leu Leu Asn Ala Pro Val Val
   275 280 285
   Ala Met Thr Gln Gly Pro His Asp Val His Val Gln Ile Glu Thr Ser
   290 295 300
   Pro Pro Ala Arg Asn Leu Lys Val Leu Lys Ala Asp Val Val Leu Leu
   305 310 315 320
   Thr Ala Ser Gly Pro Ala Val Lys Arg Ile Thr Phe Ser Pro Pro Leu
   325 330 335
   Pro Arg His Met Gln Glu Ala Leu Arg Arg Leu His Tyr Val Pro Ala
   340 345 350
   Thr Lys Val Phe Leu Ser Phe Arg Arg Pro Phe Trp Arg Glu Glu His
   355 360 365
   Ile Glu Gly Gly His Ser Asn Thr Asp Arg Pro Ser Arg Met Ile Phe
   370 375 380
   Tyr Pro Pro Pro Arg Glu Gly Ala Leu Leu Leu Ala Ser Tyr Thr Trp
   385 390 395 400
   Ser Asp Ala Ala Ala Ala Phe Ala Gly Leu Ser Arg Glu Glu Ala Leu
   405 410 415
   Arg Leu Ala Leu Asp Asp Val Ala Ala Leu His Gly Pro Val Val Arg
   420 425 430
   Gln Leu Trp Asp Gly Thr Gly Val Val Lys Arg Trp Ala Glu Asp Gln
   435 440 445
   His Ser Gln Gly Gly Phe Val Val Gln Pro Pro Ala Leu Trp Gln Thr
   450 455 460
   Glu Lys Asp Asp Trp Thr Val Pro Tyr Gly Arg Ile Tyr Phe Ala Gly
   465 470 475 480
   Glu His Thr Ala Tyr Pro His Gly Trp Val Glu Thr Ala Val Lys Ser
   485 490 495
   Ala Leu Arg Ala Ala Ile Lys Ile Asn Ser Arg Lys Gly Pro Ala Ser
   500 505 510
   Asp Thr Ala Ser Pro Glu Gly His Ala Ser Asp Met Glu Gly Gln Gly
   515 520 525
   His Val His Gly Val Ala Ser Ser Pro Ser His Asp Leu Ala Lys Glu
   530 535 540
   Glu Gly Ser His Pro Pro Val Gln Gly Gln Leu Ser Leu Gln Asn Thr
   545 550 555 560
   Thr His Thr Arg Thr Ser His
   565
<210> 47
   <211> 462
   <212> PRT
   <213> Homo sapiens
<300>
   <308> Swiss-Prot/P02790
   <309> 1986-07-21
   <313> (1)..(462)
<400> 47
   Met Ala Arg Val Leu Gly Ala Pro Val Ala Leu Gly Leu Trp Ser Leu
   1               5                   10                  15
   Cys Trp Ser Leu Ala Ile Ala Thr Pro Leu Pro Pro Thr Ser Ala His
               20                  25                  30
   Gly Asn Val Ala Glu Gly Glu Thr Lys Pro Asp Pro Asp Val Thr Glu
           35                  40                  45
   Arg Cys Ser Asp Gly Trp Ser Phe Asp Ala Thr Thr Leu Asp Asp Asn
   50 55 60
   Gly Thr Met Leu Phe Phe Lys Gly Glu Phe Val Trp Lys Ser His Lys
   65 70 75 80
   Trp Asp Arg Glu Leu Ile Ser Glu Arg Trp Lys Asn Phe Pro Ser Pro
   85 90 95
   Val Asp Ala Ala Phe Arg Gln Gly His Asn Ser Val Phe Leu Ile Lys
   100 105 110
   Gly Asp Lys Val Trp Val Tyr Pro Pro Glu Lys Lys Glu Lys Gly Tyr
   115 120 125
   Pro Lys Leu Leu Gln Asp Glu Phe Pro Gly Ile Pro Ser Pro Leu Asp
   130 135 140
   Ala Ala Val Glu Cys His Arg Gly Glu Cys Gln Ala Glu Gly Val Leu
   145 150 155 160
   Phe Phe Gln Gly Asp Arg Glu Trp Phe Trp Asp Leu Ala Thr Gly Thr
   165 170 175
   Met Lys Glu Arg Ser Trp Pro Ala Val Gly Asn Cys Ser Ser Ala Leu
   180 185 190
   Arg Trp Leu Gly Arg Tyr Tyr Cys Phe Gln Gly Asn Gln Phe Leu Arg
   195 200 205
   Phe Asp Pro Val Arg Gly Glu Val Pro Pro Arg Tyr Pro Arg Asp Val
   210 215 220
   Arg Asp Tyr Phe Met Pro Cys Pro Gly Arg Gly His Gly His Arg Asn
   225 230 235 240
   Gly Thr Gly His Gly Asn Ser Thr His His Gly Pro Glu Tyr Met Arg
   245 250 255
   Cys Ser Pro His Leu Val Leu Ser Ala Leu Thr Ser Asp Asn His Gly
   260 265 270
   Ala Thr Tyr Ala Phe Ser Gly Thr His Tyr Trp Arg Leu Asp Thr Ser
   275 280 285
   Arg Asp Gly Trp His Ser Trp Pro Ile Ala His Gln Trp Pro Gln Gly
   290 295 300
   Pro Ser Ala Val Asp Ala Ala Phe Ser Trp Glu Glu Lys Leu Tyr Leu
   305 310 315 320
   Val Gln Gly Thr Gln Val Tyr Val Phe Leu Thr Lys Gly Gly Tyr Thr
   325 330 335
   Leu Val Ser Gly Tyr Pro Lys Arg Leu Glu Lys Glu Val Gly Thr Pro
   340 345 350
   His Gly Ile Ile Leu Asp Ser Val Asp Ala Ala Phe Ile Cys Pro Gly
   355 360 365
   Ser Ser Arg Leu His Ile Met Ala Gly Arg Arg Leu Trp Trp Leu Asp
   370 375 380
   Leu Lys Ser Gly Ala Gln Ala Thr Trp Thr Glu Leu Pro Trp Pro His
   385 390 395 400
   Glu Lys Val Asp Gly Ala Leu Cys Met Glu Lys Ser Leu Gly Pro Asn
   405 410 415
   Ser Cys Ser Ala Asn Gly Pro Gly Leu Tyr Leu Ile His Gly Pro Asn
   420 425 430
   Leu Tyr Cys Tyr Ser Asp Val Glu Lys Leu Asn Ala Ala Lys Ala Leu
   435 440 445
   Pro Gln Pro Gln Asn Val Thr Ser Leu Leu Gly Cys Thr His
   450 455 460
<210> 48
   <211> 369
   <212> PRT
   <213> Homo sapiens
<300>
   <308> swiss-Prot/P50502
   <309> 1996-10-02
   <313> (1)..(369)
<400> 48
   Met Asp Pro Arg Lys Val Asn Glu Leu Arg Ala Phe Val Lys Met Cys
   1               5                   10                  15
   Lys Gln Asp Pro Ser Val Leu His Thr Glu Glu Met Arg Phe Leu Arg
               20                  25                  30
   Glu Trp Val Glu Ser Met Gly Gly Lys Val Pro Pro Ala Thr Gln Lys
           35                  40                  45
   Ala Lys Ser Glu Glu Asn Thr Lys Glu Glu Lys Pro Asp Ser Lys Lys
       50                  55                  60
   Val Glu Glu Asp Leu Lys Ala Asp Glu Pro Ser Ser Glu Glu Ser Asp
   65                  70                  75                  80
   Leu Glu Ile Asp Lys Glu Gly Val Ile Glu Pro Asp Thr Asp Ala Pro
                   85                  90                  95
   Gln Glu Met Gly Asp Glu Asn Ala Glu Ile Thr Glu Glu Met Met Asp
   100 105 110
   Gln Ala Asn Asp Lys Lys Val Ala Ala Ile Glu Ala Leu Asn Asp Gly
   115 120 125
   Glu Leu Gln Lys Ala Ile Asp Leu Phe Thr Asp Ala Ile Lys Leu Asn
   130 135 140
   Pro Arg Leu Ala Ile Leu Tyr Ala Lys Arg Ala Ser Val Phe Val Lys
   145 150 155 160
   Leu Gln Lys Pro Asn Ala Ala Ile Arg Asp Cys Asp Arg Ala Ile Glu
   165 170 175
   Ile Asn Pro Asp Ser Ala Gln Pro Tyr Lys Trp Arg Gly Lys Ala His
   180 185 190
   Arg Leu Leu Gly His Trp Glu Glu Ala Ala His Asp Leu Ala Leu Ala
   195 200 205
   Cys Lys Leu Asp Tyr Asp Glu Asp Ala Ser Ala Met Leu Lys Glu Val
   210 215 220
   Gln Pro Arg Ala Gln Lys Ile Ala Glu His Arg Arg Lys Tyr Glu Arg
   225 230 235 240
   Lys Arg Glu Glu Arg Glu Ile Lys Glu Arg Ile Glu Arg Val Lys Lys
   245 250 255
   Ala Arg Glu Glu His Glu Arg Ala Gln Arg Glu Glu Glu Ala Arg Arg
   260 265 270
   Gln Ser Gly Ala Gln Tyr Gly Ser Phe Pro Gly Gly Phe Pro Gly Gly
   275 280 285
   Met Pro Gly Asn Phe Pro Gly Gly Met Pro Gly Met Gly Gly Gly Met
   290 295 300
   Pro Gly Met Ala Gly Met Pro Gly Leu Asn Glu Ile Leu Ser Asp Pro
   305 310 315 320
   Glu Val Leu Ala Ala Met Gln Asp Pro Glu Val Met Val Ala Phe Gln
   325 330 335
   Asp Val Ala Gln Asn Pro Ala Asn Met Ser Lys Tyr Gln Ser Asn Pro
   340 345 350
   Lys Val Met Asn Leu Ile Ser Lys Leu Ser Ala Lys Phe Gly Gly Gln
   355 360 365
   Ala
<210> 49
   <211> 9
   <212> PRT
   <213> Homo sapiens
<400> 49
   Met Gln Leu Met His Ala Asn Ala Gln
   1               5
<210> 50
   <211> 9
   <212> PRT
   <213> Homo sapiens
<400> 50
   Leu Thr Leu Asp Ser Asn Thr Lys Tyr
   1               5
<210> 51
   <211> 9
   <212> PRT
   <213> Homo sapiens
<400> 51
   Phe Val Ile Asp Lys Ser Gly Ser Met
   1               5
<210> 52
   <211> 9
   <212> PRT
   <213> Homo sapiens
<400> 52
   Tyr Leu Leu Asp Ser Asn Ser Trp Ile
   1               5
<210> 53
   <211> 9
   <212> PRT
   <213> Homo sapiens
<400> 53
   Tyr Glu Leu Asp Lys Ala Phe Ser Asp
   1               5
<210> 54
   <211> 9
   <212> PRT
   <213> Homo sapiens
<400> 54
   Ile Lys Ser Gln Gln Ser Glu Val Thr
   1 5
<210> 55
   <211> 9
   <212> PRT
   <213> Homo sapiens
<400> 55
   Val Gln Ile Glu Thr Ser Pro Pro Ala
   1 5
<210> 56
   <211> 9
   <212> PRT
   <213> Homo sapiens
<400> 56
   Ile Ile Leu Asp Ser Val Asp Ala Ala
   1 5
<210> 57
   <211> 9
   <212> PRT
   <213> Homo sapiens
<400> 57
   Ile Glu Pro Asp Thr Asp Ala Pro Gln
   1 5
<210> 58
   <211> 17
   <212> PRT
   <213> Homo sapiens
<400> 58
   Asn Ile Gln Pro Ile Phe Ala Val Thr Ser Arg Met Val Lys Thr Tyr
   1 5 10 15
   Glu
<210> 59
   <211> 19
   <212> PRT
   <213> Homo sapiens
<400> 59
   Glu Asn Asn Ile Gln Pro Ile Phe Ala Val Thr Ser Arg Met Val Lys
   1 5 10 15
   Thr Tyr Glu
<210> 60
   <211> 17
   <212> PRT
   <213> Homo sapiens
<400> 60
   Asn Lys Val Phe Gly Glu Asp Ser Val Gly Val Ile Phe Lys Asn Gly
   1 5 10 15
   Asp
<210> 61
   <211> 16
   <212> PRT
   <213> Homo sapiens
<400> 61
   Tyr Pro Glu Gln Leu Lys Met Thr Val Val Lys Leu Ile Ser His Arg
   1 5 10 15
<210> 62
   <211> 16
   <212> PRT
   <213> Homo sapiens
<400> 62
   Lys Asn Thr Leu Tyr Leu Gln Met Asn Ser Leu Arg Ala Glu Asp Thr
   1 5 10 15
<210> 63
   <211> 16
   <212> PRT
   <213> Homo sapiens
<400> 63
   Asn Gly Gly His Tyr Thr Tyr Ser Glu Asn Arg Val Glu Lys Asp Gly
   1 5 10 15
<210> 64
   <211> 15
   <212> PRT
   <213> Homo sapiens
<400> 64
   Gly Pro Asn Asn Tyr Tyr Ser Phe Ala Ser Gln Gln Gln Lys Pro
   1 5 10 15
<210> 65
   <211> 16
   <212> PRT
   <213> Homo sapiens
<400> 65
   Gly Pro Asn Asn Tyr Tyr Ser Phe Ala Ser Gln Gln Gln Lys Pro Glu
   1 5 10 15
<210> 66
   <211> 17
   <212> PRT
   <213> Homo sapiens
<400> 66
   Gly Pro Asn Asn Tyr Tyr Ser Phe Ala Ser Gln Gln Gln Lys Pro Glu
   1 5 10 15
   Asp
<210> 67
   <211> 18
   <212> PRT
   <213> Homo sapiens
<400> 67
   Gly Pro Asn Asn Tyr Tyr Ser Phe Ala Ser Gln Gln Gln Lys Pro Glu
   1               5                   10                  15
   Asp Thr
<210> 68
   <211> 16
   <212> PRT
   <213> Homo sapiens
<400> 68
   Glu Lys Leu Trp Phe Val Pro Ala Lys Val Glu Asp Ser Gly His Tyr
   1               5                   10                  15
<210> 69
   <211> 14
   <212> PRT
   <213> Homo sapiens
<400> 69
   Ser Leu Arg Glu Leu His Leu Asp His Asn Gln Ile Ser Arg
   1               5                   10
<210> 70
   <211> 13
   <212> PRT
   <213> Homo sapiens
<400> 70
   Leu Arg Glu Leu His Leu Asp His Asn Gln Ile Ser Arg
   1               5                   10
<210> 71
   <211> 15
   <212> PRT
   <213> Homo sapiens
<400> 71
   Glu Thr Met Lys Met Arg Tyr Glu His Ile Asp His Thr Phe Glu
   1 5 10 15
<210> 72
   <211> 17
   <212> PRT
   <213> Homo sapiens
<400> 72
   Glu Thr Met Lys Met Arg Tyr Glu His Ile Asp His Thr Phe Glu Ile
   1 5 10 15
   Gln
<210> 73
   <211> 16
   <212> PRT
   <213> Homo sapiens
<400> 73
   His Met Phe Leu Gln Asp Glu Ile Ile Asp Lys Ser Tyr Thr Pro Ser
   1 5 10 15
<210> 74
   <211> 17
   <212> PRT
   <213> Homo sapiens
<400> 74
   Val Asp Arg Tyr Ile Ser Lys Tyr Glu Leu Asp Lys Ala Phe Ser Asp
   1 5 10 15
   Arg
<210> 75
   <211> 14
   <212> PRT
   <213> Homo sapiens
<400> 75
   Arg Tyr Ile Ser Lys Tyr Glu Leu Asp Lys Ala Phe Ser Asp
   1 5 10
<210> 76
   <211> 19
   <212> PRT
   <213> Homo sapiens
<400> 76
   Leu Pro Val Gly Arg Thr Val Met Val Asn Ile Glu Asn Pro Glu Gly
   1 5 10 15
   Ile Pro Val
<210> 77
   <211> 20
   <212> PRT
   <213> Homo sapiens
<400> 77
   Leu Pro Val Gly Arg Thr Val Met Val Asn Ile Glu Asn Pro Glu Gly
   1 5 10 15
   Ile Pro Val Lys
   20
<210> 78
   <211> 18
   <212> PRT
   <213> Homo sapiens
<400> 78
   Gly Thr Pro His Gly Ile Ile Leu Asp Ser Val Asp Ala Ala Phe Ile
   1 5 10 15
   Cys Pro
<210> 79
   <211> 18
   <212> PRT
   <213> Homo sapiens
<400> 79
   Met Ala Thr Pro Leu Leu Met Gln Ala Leu Pro Met Gly Ala Leu Pro
   1               5                   10                  15
   Gln Gly
<210> 80
   <211> 17
   <212> PRT
   <213> Homo sapiens
<400> 80
   Met Ala Thr Pro Leu Leu Met Gln Ala Leu Pro Met Gly Ala Leu Pro
   1               5                   10                  15
   Gln
<210> 81
   <211> 16
   <212> PRT
   <213> Homo sapiens
<400> 81
   Met Ala Thr Pro Leu Leu Met Gln Ala Leu Pro Met Gly Ala Leu Pro
   1               5                   10                  15
<210> 82
   <211> 21
   <212> PRT
   <213> Homo sapiens
<400> 82
   Met Ala Thr Pro Leu Leu Met Gln Ala Leu Pro Met Gly Ala Leu Pro
   1               5                   10                  15
   Gln Gly Pro Met Gln
               20
<210> 83
   <211> 15
   <212> PRT
   <213> Homo sapiens
<400> 83
   Ala Thr Pro Leu Leu Met Gln Ala Leu Pro Met Gly Ala Leu Pro
   1 5 10 15
<210> 84
   <211> 23
   <212> PRT
   <213> Homo sapiens
<400> 84
   His Pro Pro Val Gln Trp Ala Phe Gln Glu Thr Ser Val Glu Ser Ala
   1 5 10 15
   Val Asp Thr Pro Phe Pro Ala
   20
<210> 85
   <211> 24
   <212> PRT
   <213> Homo sapiens
<400> 85
   His Pro Pro Val Gln Trp Ala Phe Gln Glu Thr Ser Val Glu Ser Ala
   1 5 10 15
   Val Asp Thr Pro Phe Pro Ala Gly
   20
<210> 86
   <211> 22
   <212> PRT
   <213> Homo sapiens
<400> 86
   His Pro Pro Val Gln Trp Ala Phe Gln Glu Thr Ser Val Glu Ser Ala
   1 5 10 15
   Val Asp Thr Pro Phe Pro
   20
<210> 87
   <211> 17
   <212> PRT
   <213> Homo sapiens
<400> 87
   Lys Ile Tyr Leu Tyr Thr Leu Asn Asp Asn Ala Arg Ser Ser Pro Val
   1 5 10 15
   Val
<210> 88
   <211> 16
   <212> PRT
   <213> Homo sapiens
<400> 88
   Lys Ile Tyr Leu Tyr Thr Leu Asn Asp Asn Ala Arg Ser Ser Pro Val
   1 5 10 15
<210> 89
   <211> 17
   <212> PRT
   <213> Homo sapiens
<400> 89
   Ile Tyr Leu Tyr Thr Leu Asn Asp Asn Ala Arg Ser Ser Pro Val Val
   1 5 10 15
   Ile
<210> 90
   <211> 15
   <212> PRT
   <213> Homo sapiens
<400> 90
   Ile Tyr Leu Tyr Thr Leu Asn Asp Asn Ala Arg Ser Ser Pro Val
   1 5 10 15
<210> 91
   <211> 16
   <212> PRT
   <213> Homo sapiens
<400> 91
   Ile Tyr Leu Tyr Thr Leu Asn Asp Asn Ala Arg Ser Ser Pro Val Val
   1 5 10 15
<210> 92
   <211> 15
   <212> PRT
   <213> Homo sapiens
<400> 92
   Tyr Asn Ser Tyr Ser Val Ser Asn Ser Glu Lys Asp Ile Met Ala
   1 5 10 15
<210> 93
   <211> 15
   <212> PRT
   <213> Homo sapiens
<400> 93
   Ala Gly Ser Leu Thr Leu Ser Lys Thr Glu Leu Gly Lys Lys Ala
   1 5 10 15
<210> 94
   <211> 16
   <212> PRT
   <213> Homo sapiens
<400> 94
   Ala Gly Ser Leu Thr Leu Ser Lys Thr Glu Leu Gly Lys Lys Ala Asp
   1 5 10 15
<210> 95
   <211> 14
   <212> PRT
   <213> Homo sapiens
<400> 95
   Val Pro Lys Asp Tyr Thr Gly Glu Asp Val Thr Pro Gln Asn
   1 5 10
<210> 96
   <211> 13
   <212> PRT
   <213> Homo sapiens
<400> 96
   Asp Ser Lys Phe His Gln Ala Ile Asn Asp Ala His Gln
   1 5 10
<210> 97
   <211> 15
   <212> PRT
   <213> Homo sapiens
<400> 97
   Met Pro Leu Glu Phe Lys Thr Leu Asn Val Leu His Asn Arg Gly
   1 5 10 15
<210> 98
   <211> 15
   <212> PRT
   <213> Homo sapiens
<400> 98
   Ala Thr Arg Ser Ile Gln Val Asp Gly Lys Thr Ile Lys Ala Gln
   1 5 10 15
<210> 99
   <211> 18 .
   <212> PRT
   <213> Homo sapiens
<400> 99
   Ala Thr Arg Ser Ile Gln Val Asp Gly Lys Thr Ile Lys Ala Gln Ile
   1 5 10 15
   Trp Asp
<210> 100
   <211> 14
   <212> PRT
   <213> Homo sapiens
<400> 100
   Thr Arg Ser Ile Gln Val Asp Gly Lys Thr Ile Lys Ala Gln
   1 5 10
<210> 101
   <211> 17
   <212> PRT
   <213> Homo sapiens
<400> 101
   Ala Thr Arg Ser Ile Gln Val Asp Gly Lys Thr Ile Lys Ala Gln Ile
   1               5                   10                  15
   Trp
<210> 102
   <211> 13
   <212> PRT
   <213> Homo sapiens
<400> 102
   Arg Ser Ile Gln Val Asp Gly Lys Thr Ile Lys Ala Gln
   1               5                   10
<210> 103
   <211> 9
   <212> PRT
   <213> Homo sapiens
<400> 103
   Ile Gln Pro Ile Phe Ala Val Thr Ser
   1               5
<210> 104
   <211> 9
   <212> PRT
   <213> Homo sapiens
<400> 104
   Val Phe Gly Glu Asp Ser Val Gly Val
   1               5
<210> 105
   <211> 9
   <212> PRT
   <213> Homo sapiens
<400> 105
   Leu Lys Met Thr Val Val Lys Leu Ile
   1               5
<210> 106
   <211> 9
   <212> PRT
   <213> Homo sapiens
<400> 106
   Tyr Leu Gln Met Asn Ser Leu Arg Ala
   1 5
<210> 107
   <211> 9
   <212> PRT
   <213> Homo sapiens
<400> 107
   Tyr Thr Tyr Ser Glu Asn Arg Val Glu
   1 5
<210> 108
   <211> 9
   <212> PRT
   <213> Homo sapiens
<400> 108
   Tyr Tyr Ser Phe Ala Ser Gln Gln Gln
   1 5
<210> 109
   <211> 9
   <212> PRT
   <213> Homo sapiens
<400> 109
   Phe Val Pro Ala Lys Val Glu Asp Ser
   1 5
<210> 110
   <211> 9
   <212> PRT
   <213> Homo sapiens
<400> 110
   Leu His Leu Asp His Asn Gln Ile Ser
   1 5
<210> 111
   <211> 9
   <212> PRT
   <213> Homo sapiens
<400> 111
   Met Arg Tyr Glu His Ile Asp His Thr
   1 5
<210> 112
   <211> 9
   <212> PRT
   <213> Homo sapiens
<400> 112
   Phe Leu Gln Asp Glu Ile Ile Asp Lys
   1 5
<210> 113
   <211> 9
   <212> PRT
   <213> Homo sapiens
<400> 113
   Met Val Asn Ile Glu Asn Pro Glu Gly
   1 5
<210> 114
   <211> 9
   <212> PRT
   <213> Homo sapiens
<400> 114
   Leu Met Gln Ala Leu Pro Met Gly Ala
   1 5
<210> 115
   <211> 9
   <212> PRT
   <213> Homo sapiens
<400> 115
   Trp Ala Phe Gln Glu Thr Ser Val Glu
   1 5
<210> 116
   <211> 9
   <212> PRT
   <213> Homo sapiens
<400> 116
   Tyr Thr Leu Asn Asp Asn Ala Arg Ser
   1               5
<210> 117
   <211> 9
   <212> PRT
   <213> Homo sapiens
<400> 117
   Tyr Ser Val Ser Asn Ser Glu Lys Asp
   1               5
<210> 118
   <211> 9
   <212> PRT
   <213> Homo sapiens
<400> 118
   Leu Thr Leu Ser Lys Thr Glu Leu Gly
   1               5
<210> 119
   <211> 9
   <212> PRT
   <213> Homo sapiens
<400> 119
   Tyr Thr Gly Glu Asp Val Thr Pro Gln
   1               5
<210> 120
   <211> 9
   <212> PRT
   <213> Homo sapiens
<400> 120
   Phe His Gln Ala Ile Asn Asp Ala His
   1               5
<210> 121
   <211> 9
   <212> PRT
   <213> Homo sapiens
<400> 121
   Phe Lys Thr Leu Asn Val Leu His Asn
   1 5
<210> 122
   <211> 9
   <212> PRT
   <213> Homo sapiens
<400> 122
   Ile Gln Val Asp Gly Lys Thr Ile Lys
   1 5
<210> 123
   <211> 769
   <212> PRT
   <213> Homo sapiens
<300>
   <308> Swissprot/P05107
   <309> 1987-08-13
   <313> (1)..(769)
<400> 123
<210> 124
   <211> 1070
   <212> PRT
   <213> Homo sapiens
<300>
   <308> Swissprot/P42338
   <309> 1995-11-01
   <313> (1)..(1070)
<400> 124
   Met Cys Phe Ser Phe Ile Met Pro Pro Ala Met Ala Asp Ile Leu Asp
   1 5 10 15
   Ile Trp Ala Val Asp Ser Gln Ile Ala Ser Asp Gly Ser Ile Pro Val
   20 25 30
   Asp Phe Leu Leu Pro Thr Gly Ile Tyr Ile Gln Leu Glu Val Pro Arg
   35 40 45
   Glu Ala Thr Ile Ser Tyr Ile Lys Gln Met Leu Trp Lys Gln Val His
   50 55 60
   Asn Tyr Pro Met Phe Asn Leu Leu Met Asp Ile Asp Ser Tyr Met Phe
   65 70 75 80
   Ala Cys Val Asn Gln Thr Ala Val Tyr Glu Glu Leu Glu Asp Glu Thr
   85 90 95
   Arg Arg Leu Cys Asp Val Arg Pro Phe Leu Pro Val Leu Lys Leu Val
   100 105 110
   Thr Arg Ser Cys Asp Pro Gly Glu Lys Leu Asp Ser Lys Ile Gly Val
   115 120 125
   Leu Ile Gly Lys Gly Leu His Glu Phe Asp Ser Leu Lys Asp Pro Glu
   130 135 140
   Val Asn Glu Phe Arg Arg Lys Met Arg Lys Phe Ser Glu Glu Lys Ile
   145 150 155 160
   Leu Ser Leu Val Gly Leu Ser Trp Met Asp Trp Leu Lys Gln Thr Tyr
   165 170 175
   Pro Pro Glu His Glu Pro Ser Ile Pro Glu Asn Leu Glu Asp Lys Leu
   180 185 190
   Tyr Gly Gly Lys Leu Ile Val Ala Val His Phe Glu Asn Cys Gln Asp
   195 200 205
   Val Phe Ser Phe Gln Val Ser Pro Asn Met Asn Pro Ile Lys Val Asn
   210 215 220
   Glu Leu Ala Ile Gln Lys Arg Leu Thr Ile His Gly Lys Glu Asp Glu
   225 230 235 240
   Val Ser Pro Tyr Asp Tyr Val Leu Gln Val Ser Gly Arg Val Glu Tyr
   245 250 255
   Val Phe Gly Asp His Pro Leu Ile Gln Phe Gln Tyr Ile Arg Asn Cys
   260 265 270
   Val Met Asn Arg Ala Leu Pro His Phe Ile Leu Val Glu Cys Cys Lys
   275 280 285
   Ile Lys Lys Met Tyr Glu Gln Glu Met Ile Ala Ile Glu Ala Ala Ile
   290 295 300
   Asn Arg Asn Ser Ser Asn Leu Pro Leu Pro Leu Pro Pro Lys Lys Thr
   305 310 315 320
   Arg Ile Ile Ser His Val Trp Glu Asn Asn Asn Pro Phe Gln Ile Val
   325 330 335
   Leu Val Lys Gly Asn Lys Leu Asn Thr Glu Glu Thr Val Lys Val His
   340 345 350
   Val Arg Ala Gly Leu Phe His Gly Thr Glu Leu Leu Cys Lys Thr Ile
   355 360 365
   Val Ser Ser Glu Val Ser Gly Lys Asn Asp His Ile Trp Asn Glu Pro
   370 375 380
   Leu Glu Phe Asp Ile Asn Ile Cys Asp Leu Pro Arg Met Ala Arg Leu
   385 390 395 400
   Cys Phe Ala Val Tyr Ala Val Leu Asp Lys Val Lys Thr Lys Lys Ser
   405 410 415
   Thr Lys Thr Ile Asn Pro Ser Lys Tyr Gln Thr Ile Arg Lys Ala Gly
   420 425 430
   Lys Val His Tyr Pro Val Ala Trp Val Asn Thr Met Val Phe Asp Phe
   435 440 445
   Lys Gly Gln Leu Arg Thr Gly Asp Ile Ile Leu His Ser Trp Ser Ser
   450 455 460
   Phe Pro Asp Glu Leu Glu Glu Met Leu Asn Pro Met Gly Thr Val Gln
   465 470 475 480
   Thr Asn Pro Tyr Thr Glu Asn Ala Thr Ala Leu His Val Lys Phe Pro
   485 490 495
   Glu Asn Lys Lys Gln Pro Tyr Tyr Tyr Pro Pro Phe Asp Lys Ile Ile
   500 505 510
   Glu Lys Ala Ala Glu Ile Ala Ser Ser Asp Ser Ala Asn Val Ser Ser
   515 520 525
   Arg Gly Gly Lys Lys Phe Leu Pro Val Leu Lys Glu Ile Leu Asp Arg
   530 535 540
   Asp Pro Leu Ser Gln Leu Cys Glu Asn Glu Met Asp Leu Ile Trp Thr
   545 550 555 560
   Leu Arg Gln Asp Cys Arg Glu Ile Phe Pro Gln Ser Leu Pro Lys Leu
   565 570 575
   Leu Leu Ser Ile Lys Trp Asn Lys Leu Glu Asp Val Ala Gln Leu Gln
   580 585 590
   Ala Leu Leu Gln Ile Trp Pro Lys Leu Pro Pro Arg Glu Ala Leu Glu
   595 600 605
   Leu Leu Asp Phe Asn Tyr Pro Asp Gln Tyr Val Arg Glu Tyr Ala Val
   610 615 620
   Gly Cys Leu Arg Gln Met Ser Asp Glu Glu Leu Ser Gln Tyr Leu Leu
   625 630 635 640
   Gln Leu Val Gln Val Leu Lys Tyr Glu Pro Phe Leu Asp Cys Ala Leu
   645 650 655
   Ser Arg Phe Leu Leu Glu Arg Ala Leu Gly Asn Arg Arg Ile Gly Gln
   660 665 670
   Phe Leu Phe Trp His Leu Arg Ser Glu Val His Ile Pro Ala Val Ser
   675 680 685
   Val Gln Phe Gly Val Ile Leu Glu Ala Tyr Cys Arg Gly Ser Val Gly
   690 695 700
   His Met Lys Val Leu Ser Lys Gln Val Glu Ala Leu Asn Lys Leu Lys
   705 710 715 720
   Thr Leu Asn Ser Leu Ile Lys Leu Asn Ala Val Lys Leu Asn Arg Ala
   725 730 735
   Lys Gly Lys Glu Ala Met His Thr Cys Leu Lys Gln Ser Ala Tyr Arg
   740 745 750
   Glu Ala Leu Ser Asp Leu Gln Ser Pro Leu Asn Pro Cys Val Ile Leu
   755 760 765
   Ser Glu Leu Tyr Val Glu Lys Cys Lys Tyr Met Asp Ser Lys Met Lys
   770 775 780
   Pro Leu Trp Leu Val Tyr Asn Asn Lys Val Phe Gly Glu Asp Ser Val
   785 790 795 800
   Gly Val Ile Phe Lys Asn Gly Asp Asp Leu Arg Gln Asp Met Leu Thr
   805 810 815
   Leu Gln Met Leu Arg Leu Met Asp Leu Leu Trp Lys Glu Ala Gly Leu
   820 825 830
   Asp Leu Arg Met Leu Pro Tyr Gly Cys Leu Ala Thr Gly Asp Arg Ser
   835 840 845
   Gly Leu Ile Glu Val Val Ser Thr Ser Glu Thr Ile Ala Asp Ile Gln
   850 855 860
   Leu Asn Ser Ser Asn Val Ala Ala Ala Ala Ala Phe Asn Lys Asp Ala
   865                 870                 875                 880
   Leu Leu Asn Trp Leu Lys Glu Tyr Asn Ser Gly Asp Asp Leu Asp Arg
                   885                 890                 895
   Ala Ile Glu Glu Phe Thr Leu Ser Cys Ala Gly Tyr Cys Val Ala Ser
               900                 905                 910
   Tyr Val Leu Gly Ile Gly Asp Arg His Ser Asp Asn Ile Met Val Lys
           915                 920                 925
   Lys Thr Gly Gln Leu Phe His Ile Asp Phe Gly His Ile Leu Gly Asn
       930                 935                 940
   Phe Lys Ser Lys Phe Gly Ile Lys Arg Glu Arg Val Pro Phe Ile Leu
   945                 950                 955                 960
   Thr Tyr Asp Phe Ile His Val Ile Gln Gln Gly Lys Thr Gly Asn Thr
                   965                 970                 975
   Glu Lys Phe Gly Arg Phe Arg Gln Cys Cys Glu Asp Ala Tyr Leu Ile
               980                 985                 990
   Leu Arg Arg His Gly Asn Leu Phe  Ile Thr Leu Phe Ala  Leu Met Leu
           995                 1000                 1005
   Thr Ala  Gly Leu Pro Glu Leu  Thr Ser Val Lys Asp  Ile Gln Tyr
       1010                 1015                 1020
   Leu Lys  Asp Ser Leu Ala Leu  Gly Lys Ser Glu Glu  Glu Ala Leu
       1025                 1030                 1035
   Lys Gln  Phe Lys Gln Lys Phe  Asp Glu Ala Leu Arg  Glu Ser Trp
       1040                 1045                 1050
   Thr Thr  Lys Val Asn Trp Met  Ala His Thr Val Arg  Lys Asp Tyr
       1055                 1060                 1065
   Arg Ser
       1070
<210> 125
   <211> 431
   <212> PRT
   <213> Homo sapiens
<300>
   <308> Swissprot/P00749
   <309> 1986-07-21
   <313> (1)..(431)
<400> 125
<210> 126
   <211> 117
   <212> PRT
   <213> Homo sapiens
<300>
   <308> Swissprot/P01764
   <309> 1986-07-21
   <313> (1)..(117)
<400> 126
   Met Glu Phe Gly Leu Ser Trp Leu Phe Leu Val Ala Ile Leu Lys Gly
   1 5 10 15
   Val Gln Cys Glu Val Gln Leu Leu Glu Ser Gly Gly Gly Leu Val Gln
   20 25 30
   Pro Gly Gly Ser Leu Arg Leu Ser Cys Ala Ala Ser Gly Phe Thr Phe
   35 40 45
   Ser Ser Tyr Ala Met Ser Trp Val Arg Gln Ala Pro Gly Lys Gly Leu
   50 55 60
   Glu Trp Val Ser Ala Ile Ser Gly Ser Gly Gly Ser Thr Tyr Tyr Gly
   65 70 75 80
   Asp Ser Val Lys Gly Arg Phe Thr Ile Ser Arg Asp Asn Ser Lys Asn
   85 90 95
   Thr Leu Tyr Leu Gln Met Asn Ser Leu Arg Ala Glu Asp Thr Ala Val
   100 105 110
   Tyr Tyr Cys Ala Lys
   115
<210> 127
   <211> 189
   <212> PRT
   <213> Homo sapiens
<300>
   <308> Swissprot/Q99497
   <309> 1997-05-01
   <313> (1)..(189)
<400> 127
<210> 128
   <211> 257
   <212> PRT
   <213> Homo sapiens
<300>
   <308> Swissprot/P48556
   <309> 1996-02-01
   <313> (1)..(257)
<400> 128
   Met Tyr Glu Gln Leu Lys Gly Glu Trp Asn Arg Lys Ser Pro Asn Leu
   1 5 10 15
   Ser Lys Cys Gly Glu Glu Leu Gly Arg Leu Lys Leu Val Leu Leu Glu
   20 25 30
   Leu Asn Phe Leu Pro Thr Thr Gly Thr Lys Leu Thr Lys Gln Gln Leu
   35 40 45
   Ile Leu Ala Arg Asp Ile Leu Glu Ile Gly Ala Gln Trp Ser Ile Leu
   50 55 60
   Arg Lys Asp Ile Pro Ser Phe Glu Arg Tyr Met Ala Gln Leu Lys Cys
   65 70 75 80
   Tyr Tyr Phe Asp Tyr Lys Glu Gln Leu Pro Glu Ser Ala Tyr Met His
   85 90 95
   Gln Leu Leu Gly Leu Asn Leu Leu Phe Leu Leu Ser Gln Asn Arg Val
   100 105 110
   Ala Glu Phe His Thr Glu Leu Glu Arg Leu Pro Ala Lys Asp Ile Gln
   115 120 125
   Thr Asn Val Tyr Ile Lys His Pro Val Ser Leu Glu Gln Tyr Leu Met
   130 135 140
   Glu Gly Ser Tyr Asn Lys Val Phe Leu Ala Lys Gly Asn Ile Pro Ala
   145 150 155 160
   Glu Ser Tyr Thr Phe Phe Ile Asp Ile Leu Leu Asp Thr Ile Arg Asp
   165 170 175
   Glu Ile Ala Gly Cys Ile Glu Lys Ala Tyr Glu Lys Ile Leu Phe Thr
   180 185 190
   Glu Ala Thr Arg Ile Leu Phe Phe Asn Thr Pro Lys Lys Met Thr Asp
   195 200 205
   Tyr Ala Lys Lys Arg Gly Trp Val Leu Gly Pro Asn Asn Tyr Tyr Ser
   210 215 220
   Phe Ala Ser Gln Gln Gln Lys Pro Glu Asp Thr Thr Ile Pro Ser Thr
   225 230 235 240
   Glu Leu Ala Lys Gln Val Ile Glu Tyr Ala Arg Gln Leu Glu Met Ile
   245 250 255
   Val
<210> 129
   <211> 569
   <212> PRT
   <213> Homo sapiens
<300>
   <308> Swissprot/P14778
   <309> 1990-04-01
   <313> (1)..(569)
<400> 129
   Met Lys Val Leu Leu Arg Leu Ile Cys Phe Ile Ala Leu Leu Ile Ser
   1               5                   10                  15
   Ser Leu Glu Ala Asp Lys Cys Lys Glu Arg Glu Glu Lys Ile Ile Leu
               20                  25                  30
   Val Ser Ser Ala Asn Glu Ile Asp Val Arg Pro Cys Pro Leu Asn Pro
           35                  40                  45
   Asn Glu His Lys Gly Thr Ile Thr Trp Tyr Lys Asp Asp Ser Lys Thr
       50                  55                  60
   Pro Val Ser Thr Glu Gln Ala Ser Arg Ile His Gln His Lys Glu Lys
   65                  70                  75                  80
   Leu Trp Phe Val Pro Ala Lys Val Glu Asp Ser Gly His Tyr Tyr Cys
                   85                  90                  95
   Val Val Arg Asn Ser Ser Tyr Cys Leu Arg Ile Lys Ile Ser Ala Lys
               100                 105                 110
   Phe Val Glu Asn Glu Pro Asn Leu Cys Tyr Asn Ala Gln Ala Ile Phe
           115                 120                 125
   Lys Gln Lys Leu Pro Val Ala Gly Asp Gly Gly Leu Val Cys Pro Tyr
       130                 135                 140
   Met Glu Phe Phe Lys Asn Glu Asn Asn Glu Leu Pro Lys Leu Gln Trp
   145                 150                 155                 160
   Tyr Lys Asp Cys Lys Pro Leu Leu Leu Asp Asn Ile His Phe Ser Gly
                   165                 170                 175
   Val Lys Asp Arg Leu Ile Val Met Asn Val Ala Glu Lys His Arg Gly
               180                 185                 190
   Asn Tyr Thr Cys His Ala Ser Tyr Thr Tyr Leu Gly Lys Gln Tyr Pro
   195 200 205
   Ile Thr Arg Val Ile Glu Phe Ile Thr Leu Glu Glu Asn Lys Pro Thr
   210 215 220
   Arg Pro Val Ile Val Ser Pro Ala Asn Glu Thr Met Glu Val Asp Leu
   225 230 235 240
   Gly Ser Gln Ile Gln Leu Ile Cys Asn Val Thr Gly Gln Leu Ser Asp
   245 250 255
   Ile Ala Tyr Trp Lys Trp Asn Gly Ser Val Ile Asp Glu Asp Asp Pro
   260 265 270
   Val Leu Gly Glu Asp Tyr Tyr Ser Val Glu Asn Pro Ala Asn Lys Arg
   275 280 285
   Arg Ser Thr Leu Ile Thr Val Leu Asn Ile Ser Glu Ile Glu Ser Arg
   290 295 300
   Phe Tyr Lys His Pro Phe Thr Cys Phe Ala Lys Asn Thr His Gly Ile
   305 310 315 320
   Asp Ala Ala Tyr Ile Gln Leu Ile Tyr Pro Val Thr Asn Phe Gln Lys
   325 330 335
   His Met Ile Gly Ile Cys Val Thr Leu Thr Val Ile Ile Val Cys Ser
   340 345 350
   Val Phe Ile Tyr Lys Ile Phe Lys Ile Asp Ile Val Leu Trp Tyr Arg
   355 360 365
   Asp Ser Cys Tyr Asp Phe Leu Pro Ile Lys Ala Ser Asp Gly Lys Thr
   370 375 380
   Tyr Asp Ala Tyr Ile Leu Tyr Pro Lys Thr Val Gly Glu Gly Ser Thr
   385 390 395 400
   Ser Asp Cys Asp Ile Phe Val Phe Lys Val Leu Pro Glu Val Leu Glu
   405 410 415
   Lys Gln Cys Gly Tyr Lys Leu Phe Ile Tyr Gly Arg Asp Asp Tyr Val
   420 425 430
   Gly Glu Asp Ile Val Glu Val Ile Asn Glu Asn Val Lys Lys Ser Arg
   435 440 445
   Arg Leu Ile Ile Ile Leu Val Arg Glu Thr Ser Gly Phe Ser Trp Leu
   450 455 460
   Gly Gly Ser Ser Glu Glu Gln Ile Ala Met Tyr Asn Ala Leu Val Gln
   465 470 475 480
   Asp Gly Ile Lys Val Val Leu Leu Glu Leu Glu Lys Ile Gln Asp Tyr
   485 490 495
   Glu Lys Met Pro Glu Ser Ile Lys Phe Ile Lys Gln Lys His Gly Ala
   500 505 510
   Ile Arg Trp Ser Gly Asp Phe Thr Gln Gly Pro Gln Ser Ala Lys Thr
   515 520 525
   Arg Phe Trp Lys Asn Val Arg Tyr His Met Pro Val Gln Arg Arg Ser
   530 535 540
   Pro Ser Ser Lys His Gln Leu Leu Ser Pro Ala Thr Lys Glu Lys Leu
   545 550 555 560
   Gln Arg Glu Ala His Val Pro Leu Gly
   565
<210> 130
   <211> 376
   <212> PRT
   <213> Homo sapiens
<300>
   <308> Swissprot/Q06828
   <309> 1994-06-01
   <313> (1)..(376)
<400> 130
<210> 131
   <211> 897
   <212> PRT
   <213> Homo sapiens
<300>
   <308> Swissprot/P32927
   <309> 1993-10-01
   <313> (1)..(897)
<400> 131
   Met Val Leu Ala Gln Gly Leu Leu Ser Met Ala Leu Leu Ala Leu Cys
   1               5                   10                  15
   Trp Glu Arg Ser Leu Ala Gly Ala Glu Glu Thr Ile Pro Leu Gln Thr
               20                  25                  30
   Leu Arg Cys Tyr Asn Asp Tyr Thr Ser His Ile Thr Cys Arg Trp Ala
           35                  40                  45
   Asp Thr Gln Asp Ala Gln Arg Leu Val Asn Val Thr Leu Ile Arg Arg
       50                  55                  60
   Val Asn Glu Asp Leu Leu Glu Pro Val Ser Cys Asp Leu Ser Asp Asp
   65                  70                  75                  80
   Met Pro Trp Ser Ala Cys Pro His Pro Arg Cys Val Pro Arg Arg Cys
                   85                  90                  95
   Val Ile Pro Cys Gln Ser Phe Val Val Thr Asp Val Asp Tyr Phe Ser
               100                 105                 110
   Phe Gln Pro Asp Arg Pro Leu Gly Thr Arg Leu Thr Val Thr Leu Thr
           115                 120                 125
   Gln His Val Gln Pro Pro Glu Pro Arg Asp Leu Gln Ile Ser Thr Asp
       130                 135                 140
   Gln Asp His Phe Leu Leu Thr Trp Ser Val Ala Leu Gly Ser Pro Gln
   145                 150                 155                 160
   Ser His Trp Leu Ser Pro Gly Asp Leu Glu Phe Glu Val Val Tyr Lys
                   165                 170                 175
   Arg Leu Gln Asp Ser Trp Glu Asp Ala Ala Ile Leu Leu Ser Asn Thr
               180                 185                 190
   Ser Gln Ala Thr Leu Gly Pro Glu His Leu Met Pro Ser Ser Thr Tyr
           195                 200                 205
   Val Ala Arg Val Arg Thr Arg Leu Ala Pro Gly Ser Arg Leu Ser Gly
       210                 215                 220
   Arg Pro Ser Lys Trp Ser Pro Glu Val Cys Trp Asp Ser Gln Pro Gly
   225                 230                 235                 240
   Asp Glu Ala Gln Pro Gln Asn Leu Glu Cys Phe Phe Asp Gly Ala Ala
                   245                 250                 255
   Val Leu Ser Cys Ser Trp Glu Val Arg Lys Glu Val Ala Ser Ser Val
   260 265 270
   Ser Phe Gly Leu Phe Tyr Lys Pro Ser Pro Asp Ala Gly Glu Glu Glu
   275 280 285
   Cys Ser Pro Val Leu Arg Glu Gly Leu Gly Ser Leu His Thr Arg His
   290 295 300
   His Cys Gln Ile Pro Val Pro Asp Pro Ala Thr His Gly Gln Tyr Ile
   305 310 315 320
   Val Ser Val Gln Pro Arg Arg Ala Glu Lys His Ile Lys Ser Ser Val
   325 330 335
   Asn Ile Gln Met Ala Pro Pro Ser Leu Asn Val Thr Lys Asp Gly Asp
   340 345 350
   Ser Tyr Ser Leu Arg Trp Glu Thr Met Lys Met Arg Tyr Glu His Ile
   355 360 365
   Asp His Thr Phe Glu Ile Gln Tyr Arg Lys Asp Thr Ala Thr Trp Lys
   370 375 380
   Asp Ser Lys Thr Glu Thr Leu Gln Asn Ala His Ser Met Ala Leu Pro
   385 390 395 400
   Ala Leu Glu Pro Ser Thr Arg Tyr Trp Ala Arg Val Arg Val Arg Thr
   405 410 415
   Ser Arg Thr Gly Tyr Asn Gly Ile Trp Ser Glu Trp Ser Glu Ala Arg
   420 425 430
   Ser Trp Asp Thr Glu Ser Val Leu Pro Met Trp Val Leu Ala Leu Ile
   435 440 445
   Val Ile Phe Leu Thr Ile Ala Val Leu Leu Ala Leu Arg Phe Cys Gly
   450 455 460
   Ile Tyr Gly Tyr Arg Leu Arg Arg Lys Trp Glu Glu Lys Ile Pro Asn
   465 470 475 480
   Pro Ser Lys Ser His Leu Phe Gln Asn Gly Ser Ala Glu Leu Trp Pro
   485 490 495
   Pro Gly Ser Met Ser Ala Phe Thr Ser Gly Ser Pro Pro His Gln Gly
   500 505 510
   Pro Trp Gly Ser Arg Phe Pro Glu Leu Glu Gly Val Phe Pro Val Gly
   515 520 525
   Phe Gly Asp Ser Glu Val Ser Pro Leu Thr Ile Glu Asp Pro Lys His
   530 535 540
   Val Cys Asp Pro Pro Ser Gly Pro Asp Thr Thr Pro Ala Ala Ser Asp
   545 550 555 560
   Leu Pro Thr Glu Gln Pro Pro Ser Pro Gln Pro Gly Pro Pro Ala Ala
   565 570 575
   Ser His Thr Pro Glu Lys Gln Ala Ser Ser Phe Asp Phe Asn Gly Pro
   580 585 590
   Tyr Leu Gly Pro Pro His Ser Arg Ser Leu Pro Asp Ile Leu Gly Gln
   595 600 605
   Pro Glu Pro Pro Gln Glu Gly Gly Ser Gln Lys Ser Pro Pro Pro Gly
   610 615 620
   Ser Leu Glu Tyr Leu Cys Leu Pro Ala Gly Gly Gln Val Gln Leu Val
   625 630 635 640
   Pro Leu Ala Gln Ala Met Gly Pro Gly Gln Ala Val Glu Val Glu Arg
   645 650 655
   Arg Pro Ser Gln Gly Ala Ala Gly Ser Pro Ser Leu Glu Ser Gly Gly
   660 665 670
   Gly Pro Ala Pro Pro Ala Leu Gly Pro Arg Val Gly Gly Gln Asp Gln
   675 680 685
   Lys Asp Ser Pro Val Ala Ile Pro Met Ser Ser Gly Asp Thr Glu Asp
   690 695 700
   Pro Gly Val Ala Ser Gly Tyr Val Ser Ser Ala Asp Leu Val Phe Thr
   705 710 715 720
   Pro Asn Ser Gly Ala Ser Ser Val Ser Leu Val Pro Ser Leu Gly Leu
   725 730 735
   Pro Ser Asp Gln Thr Pro Ser Leu Cys Pro Gly Leu Ala Ser Gly Pro
   740 745 750
   Pro Gly Ala Pro Gly Pro Val Lys Ser Gly Phe Glu Gly Tyr Val Glu
   755 760 765
   Leu Pro Pro Ile Glu Gly Arg Ser Pro Arg Ser Pro Arg Asn Asn Pro
   770 775 780
   Val Pro Pro Glu Ala Lys Ser Pro Val Leu Asn Pro Gly Glu Arg Pro
   785 790 795 800
   Ala Asp Val Ser Pro Thr Ser Pro Gln Pro Glu Gly Leu Leu Val Leu
   805 810 815
   Gln Gln Val Gly Asp Tyr Cys Phe Leu Pro Gly Leu Gly Pro Gly Pro
   820 825 830
   Leu Ser Leu Arg Ser Lys Pro Ser Ser Pro Gly Pro Gly Pro Glu Ile
   835 840 845
   Lys Asn Leu Asp Gln Ala Phe Gln Val Lys Lys Pro Pro Gly Gln Ala
   850 855 860
   Val Pro Gln Val Pro Val Ile Gln Leu Phe Lys Ala Leu Lys Gln Gln
   865 870 875 880
   Asp Tyr Leu Ser Leu Pro Pro Trp Glu Val Asn Lys Pro Gly Glu Val
   885 890 895
   Cys
<210> 132
   <211> 261
   <212> PRT
   <213> Homo sapiens
<300>
   <308> Swissprot/Q60493
   <309> 1996-11-01
   <313> (1)..(261)
<400> 132
   Met Ile Tyr Lys Cys Pro Met Cys Arg Glu Phe Phe Ser Glu Arg Ala
   1               5                   10                  15
   Asp Leu Phe Met His Gln Lys Val His Thr Ala Glu Lys Pro His Lys
               20                  25                  30
   Cys Asp Lys Cys Asp Lys Gly Phe Phe His Ile Ser Glu Leu His Ile
           35                  40                  45
   His Trp Arg Asp His Thr Gly Glu Lys Val Tyr Lys Cys Asp Asp Cys
       50                  55                  60
   Gly Lys Asp Phe Ser Thr Thr Thr Lys Leu Asn Arg His Lys Lys Ile
   65                  70                  75                  80
   His Thr Val Glu Lys Pro Tyr Lys Cys Tyr Glu Cys Gly Lys Ala Phe
                   85                  90                  95
   Asn Trp Ser Pro His Leu Gln Ile His Met Arg Val His Thr Gly Glu
               100                 105                 110
   Lys Pro Tyr Val Cys Ser Glu Cys Gly Arg Gly Phe Ser Asn Ser Ser
           115                 120                 125
   Asn Leu Cys Met His Gln Arg Val His Thr Gly Glu Lys Pro Phe Lys
   130 135 140
   Cys Glu Glu Cys Gly Lys Ala Phe Arg His Thr Ser Ser Leu Cys Met
   145 150 155 160
   His Gln Arg Val His Thr Gly Glu Lys Pro Tyr Lys Cys Tyr Glu Cys
   165 170 175
   Gly Lys Ala Phe Ser Gln Ser Ser Ser Leu Cys Ile His Gln Arg Val
   180 185 190
   His Thr Gly Glu Lys Pro Tyr Arg Cys Cys Gly Cys Gly Lys Ala Phe
   195 200 205
   Ser Gln Ser Ser Ser Leu Cys Ile His Gln Arg Val His Thr Gly Glu
   210 215 220
   Lys Pro Phe Lys Cys Asp Glu Cys Gly Lys Ala Phe Ser Gln Ser Thr
   225 230 235 240
   Ser Leu Cys Ile His Gln Arg Val His Thr Lys Glu Arg Asn His Leu
   245 250 255
   Lys Ile Ser Val Ile
   260
<210> 133
   <211> 296
   <212> PRT
   <213> Homo sapiens
<300>
   <308> Swissprot/P04233
   <309> 1987-03-20
   <313> (1)..(296)
<400> 133
   Met His Arg Arg Arg Ser Arg Ser Cys Arg Glu Asp Gln Lys Pro Val
   1               5                   10                  15
   Met Asp Asp Gln Arg Asp Leu Ile Ser Asn Asn Glu Gln Leu Pro Met
               20                  25                  30
   Leu Gly Arg Arg Pro Gly Ala Pro Glu Ser Lys Cys Ser Arg Gly Ala
           35                  40                  45
   Leu Tyr Thr Gly Phe Ser Ile Leu Val Thr Leu Leu Leu Ala Gly Gln
       50                  55                  60
   Ala Thr Thr Ala Tyr Phe Leu Tyr Gln Gln Gln Gly Arg Leu Asp Lys
   65                  70                  75                  80
   Leu Thr Val Thr Ser Gln Asn Leu Gln Leu Glu Asn Leu Arg Met Lys
                   85                  90                  95
   Leu Pro Lys Pro Pro Lys Pro Val Ser Lys Met Arg Met Ala Thr Pro
   100 105 110
   Leu Leu Met Gln Ala Leu Pro Met Gly Ala Leu Pro Gln Gly Pro Met
   115 120 125
   Gln Asn Ala Thr Lys Tyr Gly Asn Met Thr Glu Asp His Val Met His
   130 135 140
   Leu Leu Gln Asn Ala Asp Pro Leu Lys Val Tyr Pro Pro Leu Lys Gly
   145 150 155 160
   Ser Phe Pro Glu Asn Leu Arg His Leu Lys Asn Thr Met Glu Thr Ile
   165 170 175
   Asp Trp Lys Val Phe Glu Ser Trp Met His His Trp Leu Leu Phe Glu
   180 185 190
   Met Ser Arg His Ser Leu Glu Gln Lys Pro Thr Asp Ala Pro Pro Lys
   195 200 205
   Val Leu Thr Lys Cys Gln Glu Glu Val Ser His Ile Pro Ala Val His
   210 215 220
   Pro Gly Ser Phe Arg Pro Lys Cys Asp Glu Asn Gly Asn Tyr Leu Pro
   225 230 235 240
   Leu Gln Cys Tyr Gly Ser Ile Gly Tyr Cys Trp Cys Val Phe Pro Asn
   245 250 255
   Gly Thr Glu Val Pro Asn Thr Arg Ser Arg Gly His His Asn Cys Ser
   260 265 270
   Glu Ser Leu Glu Leu Glu Asp Pro Ser Ser Gly Leu Gly Val Thr Lys
   275 280 285
   Gln Asp Leu Gly Pro Val Pro Met
   290 295
<210> 134
   <211> 163
   <212> PRT
   <213> Homo sapiens
<300>
   <308> Swissprot/Q99969
   <309> 2000-05-30
   <313> (1)..(163)
<400> 134
   Met Arg Arg Leu Leu Ile Pro Leu Ala Leu Trp Leu Gly Ala Val Gly
   1               5                   10                  15
   Val Gly Val Ala Glu Leu Thr Glu Ala Gln Arg Arg Gly Leu Gln Val
   20                  25                  30
   Ala Leu Glu Glu Phe His Lys His Pro Pro Val Gln Trp Ala Phe Gln
           35                  40                  45
   Glu Thr Ser Val Glu Ser Ala Val Asp Thr Pro Phe Pro Ala Gly Ile
       50                  55                  60
   Phe Val Arg Leu Glu Phe Lys Leu Gln Gln Thr Ser Cys Arg Lys Arg
   65                  70                  75                  80
   Asp Trp Lys Lys Pro Glu Cys Lys Val Arg Pro Asn Gly Arg Lys Arg
                   85                  90                  95
   Lys Cys Leu Ala Cys Ile Lys Leu Gly Ser Glu Asp Lys Val Leu Gly
               100                 105                 110
   Arg Leu Val His Cys Pro Ile Glu Thr Gln Val Leu Arg Glu Ala Glu
           115                 120                 125
   Glu His Gln Glu Thr Gln Cys Leu Arg Val Gln Arg Ala Gly Glu Asp
       130                 135                 140
   Pro His Ser Phe Tyr Phe Pro Gly Gln Phe Ala Phe Ser Lys Ala Leu
   145                 150                 155                 160
   Pro Arg Ser
<210> 135
   <211> 2386
   <212> PRT
   <213> Homo sapiens
<300>
   <308> Swissprot/P02751
   <309> 1986-07-21
   <313> (1)..(2386)
<400> 135
   Met Leu Arg Gly Pro Gly Pro Gly Leu Leu Leu Leu Ala Val Gln Cys
   1               5                   10                  15
   Leu Gly Thr Ala Val Pro Ser Thr Gly Ala Ser Lys Ser Lys Arg Gln
               20                  25                  30
   Ala Gln Gln Met Val Gln Pro Gln Ser Pro Val Ala Val Ser Gln Ser
           35                  40                  45
   Lys Pro Gly Cys Tyr Asp Asn Gly Lys His Tyr Gln Ile Asn Gln Gln
       50                  55                  60
   Trp Glu Arg Thr Tyr Leu Gly Asn Ala Leu Val Cys Thr Cys Tyr Gly
   65                  70                  75                  80
   Gly Ser Arg Gly Phe Asn Cys Glu Ser Lys Pro Glu Ala Glu Glu Thr
   85 90 95
   Cys Phe Asp Lys Tyr Thr Gly Asn Thr Tyr Arg Val Gly Asp Thr Tyr
   100 105 110
   Glu Arg Pro Lys Asp Ser Met Ile Trp Asp Cys Thr Cys Ile Gly Ala
   115 120 125
   Gly Arg Gly Arg Ile Ser Cys Thr Ile Ala Asn Arg Cys His Glu Gly
   130 135 140
   Gly Gln Ser Tyr Lys Ile Gly Asp Thr Trp Arg Arg Pro His Glu Thr
   145 150 155 160
   Gly Gly Tyr Met Leu Glu Cys Val Cys Leu Gly Asn Gly Lys Gly Glu
   165 170 175
   Trp Thr Cys Lys Pro Ile Ala Glu Lys Cys Phe Asp His Ala Ala Gly
   180 185 190
   Thr Ser Tyr Val Val Gly Glu Thr Trp Glu Lys Pro Tyr Gln Gly Trp
   195 200 205
   Met Met Val Asp Cys Thr Cys Leu Gly Glu Gly Ser Gly Arg Ile Thr
   210 215 220
   Cys Thr Ser Arg Asn Arg Cys Asn Asp Gln Asp Thr Arg Thr Ser Tyr
   225 230 235 240
   Arg Ile Gly Asp Thr Trp Ser Lys Lys Asp Asn Arg Gly Asn Leu Leu
   245 250 255
   Gln Cys Ile Cys Thr Gly Asn Gly Arg Gly Glu Trp Lys Cys Glu Arg
   260 265 270
   His Thr Ser Val Gln Thr Thr Ser Ser Gly Ser Gly Pro Phe Thr Asp
   275 280 285
   Val Arg Ala Ala Val Tyr Gln Pro Gln Pro His Pro Gln Pro Pro Pro
   290 295 300
   Tyr Gly His Cys Val Thr Asp Ser Gly Val Val Tyr Ser Val Gly Met
   305 310 315 320
   Gln Trp Leu Lys Thr Gln Gly Asn Lys Gln Met Leu Cys Thr Cys Leu
   325 330 335
   Gly Asn Gly Val Ser Cys Gln Glu Thr Ala Val Thr Gln Thr Tyr Gly
   340 345 350
   Gly Asn Ser Asn Gly Glu Pro Cys Val Leu Pro Phe Thr Tyr Asn Gly
   355 360 365
   Arg Thr Phe Tyr Ser Cys Thr Thr Glu Gly Arg Gln Asp Gly His Leu
   370 375 380
   Trp Cys Ser Thr Thr Ser Asn Tyr Glu Gln Asp Gln Lys Tyr Ser Phe
   385 390 395 400
   Cys Thr Asp His Thr Val Leu Val Gln Thr Gln Gly Gly Asn Ser Asn
   405 410 415
   Gly Ala Leu Cys His Phe Pro Phe Leu Tyr Asn Asn His Asn Tyr Thr
   420 425 430
   Asp Cys Thr Ser Glu Gly Arg Arg Asp Asn Met Lys Trp Cys Gly Thr
   435 440 445
   Thr Gln Asn Tyr Asp Ala Asp Gln Lys Phe Gly Phe Cys Pro Met Ala
   450 455 460
   Ala His Glu Glu Ile Cys Thr Thr Asn Glu Gly Val Met Tyr Arg Ile
   465 470 475 480
   Gly Asp Gln Trp Asp Lys Gln His Asp Met Gly His Met Met Arg Cys
   485 490 495
   Thr Cys Val Gly Asn Gly Arg Gly Glu Trp Thr Cys Ile Ala Tyr Ser
   500 505 510
   Gln Leu Arg Asp Gln Cys Ile Val Asp Asp Ile Thr Tyr Asn Val Asn
   515 520 525
   Asp Thr Phe His Lys Arg His Glu Glu Gly His Met Leu Asn Cys Thr
   530 535 540
   Cys Phe Gly Gln Gly Arg Gly Arg Trp Lys Cys Asp Pro Val Asp Gln
   545 550 555 560
   Cys Gln Asp Ser Glu Thr Gly Thr Phe Tyr Gln Ile Gly Asp Ser Trp
   565 570 575
   Glu Lys Tyr Val His Gly Val Arg Tyr Gln Cys Tyr Cys Tyr Gly Arg
   580 585 590
   Gly Ile Gly Glu Trp His Cys Gln Pro Leu Gln Thr Tyr Pro Ser Ser
   595 600 605
   Ser Gly Pro Val Glu Val Phe Ile Thr Glu Thr Pro Ser Gln Pro Asn
   610 615 620
   Ser His Pro Ile Gln Trp Asn Ala Pro Gln Pro Ser His Ile Ser Lys
   625 630 635 640
   Tyr Ile Leu Arg Trp Arg Pro Lys Asn Ser Val Gly Arg Trp Lys Glu
   645 650 655
   Ala Thr Ile Pro Gly His Leu Asn Ser Tyr Thr Ile Lys Gly Leu Lys
   660 665 670
   Pro Gly Val Val Tyr Glu Gly Gln Leu Ile Ser Ile Gln Gln Tyr Gly
   675 680 685
   His Gln Glu Val Thr Arg Phe Asp Phe Thr Thr Thr Ser Thr Ser Thr
   690 695 700
   Pro Val Thr Ser Asn Thr Val Thr Gly Glu Thr Thr Pro Phe Ser Pro
   705 710 715 720
   Leu Val Ala Thr Ser Glu Ser Val Thr Glu Ile Thr Ala Ser Ser Phe
   725 730 735
   Val Val Ser Trp Val Ser Ala Ser Asp Thr Val Ser Gly Phe Arg Val
   740 745 750
   Glu Tyr Glu Leu Ser Glu Glu Gly Asp Glu Pro Gln Tyr Leu Asp Leu
   755 760 765
   Pro Ser Thr Ala Thr Ser Val Asn Ile Pro Asp Leu Leu Pro Gly Arg
   770 775 780
   Lys Tyr Ile Val Asn Val Tyr Gln Ile Ser Glu Asp Gly Glu Gln Ser
   785 790 795 800
   Leu Ile Leu Ser Thr Ser Gln Thr Thr Ala Pro Asp Ala Pro Pro Asp
   805 810 815
   Pro Thr Val Asp Gln Val Asp Asp Thr Ser Ile Val Val Arg Trp Ser
   820 825 830
   Arg Pro Gln Ala Pro Ile Thr Gly Tyr Arg Ile Val Tyr Ser Pro Ser
   835 840 845
   Val Glu Gly Ser Ser Thr Glu Leu Asn Leu Pro Glu Thr Ala Asn Ser
   850 855 860
   Val Thr Leu Ser Asp Leu Gln Pro Gly Val Gln Tyr Asn Ile Thr Ile
   865 870 875 880
   Tyr Ala Val Glu Glu Asn Gln Glu Ser Thr Pro Val Val Ile Gln Gln
   885 890 895
   Glu Thr Thr Gly Thr Pro Arg Ser Asp Thr Val Pro Ser Pro Arg Asp
   900 905 910
   Leu Gln Phe Val Glu Val Thr Asp Val Lys Val Thr Ile Met Trp Thr
   915 920 925
   Pro Pro Glu Ser Ala Val Thr Gly Tyr Arg Val Asp Val Ile Pro Val
   930 935 940
   Asn Leu Pro Gly Glu His Gly Gln Arg Leu Pro Ile Ser Arg Asn Thr
   945 950 955 960
   Phe Ala Glu Val Thr Gly Leu Ser Pro Gly Val Thr Tyr Tyr Phe Lys
   965 970 975
   Val Phe Ala Val Ser His Gly Arg Glu Ser Lys Pro Leu Thr Ala Gln
   980 985 990
   Gln Thr Thr Lys Leu Asp Ala Pro Thr Asn Leu Gln Phe Val Asn Glu
   995 1000 1005
   Thr Asp Ser Thr Val Leu Val Arg Trp Thr Pro Pro Arg Ala Gln
   1010 1015 1020
   Ile Thr Gly Tyr Arg Leu Thr Val Gly Leu Thr Arg Arg Gly Gln
   1025 1030 1035
   Pro Arg Gln Tyr Asn Val Gly Pro Ser Val Ser Lys Tyr Pro Leu
   1040 1045 1050
   Arg Asn Leu Gln Pro Ala Ser Glu Tyr Thr Val Ser Leu Val Ala
   1055 1060 1065
   Ile Lys Gly Asn Gln Glu Ser Pro Lys Ala Thr Gly Val Phe Thr
   1070 1075 1080
   Thr Leu Gln Pro Gly Ser Ser Ile Pro Pro Tyr Asn Thr Glu Val
   1085 1090 1095
   Thr Glu Thr Thr Ile Val Ile Thr Trp Thr Pro Ala Pro Arg Ile
   1100 1105 1110
   Gly Phe Lys Leu Gly Val Arg Pro Ser Gln Gly Gly Glu Ala Pro
   1115 1120 1125
   Arg Glu Val Thr Ser Asp Ser Gly Ser Ile Val Val Ser Gly Leu
   1130 1135 1140
   Thr Pro Gly Val Glu Tyr Val Tyr Thr Ile Gln Val Leu Arg Asp
   1145 1150 1155
   Gly Gln Glu Arg Asp Ala Pro Ile Val Asn Lys Val Val Thr Pro
   1160 1165 1170
   Leu Ser Pro Pro Thr Asn Leu His Leu Glu Ala Asn Pro Asp Thr
   1175 1180 1185
   Gly Val Leu Thr Val Ser Trp Glu Arg Ser Thr Thr Pro Asp Ile
   1190 1195 1200
   Thr Gly Tyr Arg Ile Thr Thr Thr Pro Thr Asn Gly Gln Gln Gly
   1205 1210 1215
   Asn Ser Leu Glu Glu Val Val His Ala Asp Gln Ser Ser Cys Thr
   1220 1225 1230
   Phe Asp Asn Leu Ser Pro Gly Leu Glu Tyr Asn Val Ser Val Tyr
   1235 1240 1245
   Thr Val Lys Asp Asp Lys Glu Ser Val Pro Ile Ser Asp Thr Ile
   1250 1255 1260
   Ile Pro Ala Val Pro Pro Pro Thr Asp Leu Arg Phe Thr Asn Ile
   1265 1270 1275
   Gly Pro Asp Thr Met Arg Val Thr Trp Ala Pro Pro Pro Ser Ile
   1280 1285 1290
   Asp Leu Thr Asn Phe Leu Val Arg Tyr Ser Pro Val Lys Asn Glu
   1295 1300 1305
   Glu Asp Val Ala Glu Leu Ser Ile Ser Pro Ser Asp Asn Ala Val
   1310 1315 1320
   Val Leu Thr Asn Leu Leu Pro Gly Thr Glu Tyr Val Val Ser Val
   1325 1330 1335
   Ser Ser Val Tyr Glu Gln His Glu Ser Thr Pro Leu Arg Gly Arg
   1340 1345 1350
   Gln Lys Thr Gly Leu Asp Ser Pro Thr Gly Ile Asp Phe Ser Asp
   1355 1360 1365
   Ile Thr Ala Asn Ser Phe Thr Val His Trp Ile Ala Pro Arg Ala
   1370 1375 1380
   Thr Ile Thr Gly Tyr Arg Ile Arg His His Pro Glu His Phe Ser
   1385 1390 1395
   Gly Arg Pro Arg Glu Asp Arg Val Pro His Ser Arg Asn Ser Ile
   1400 1405 1410
   Thr Leu Thr Asn Leu Thr Pro Gly Thr Glu Tyr Val Val Ser Ile
   1415 1420 1425
   Val Ala Leu Asn Gly Arg Glu Glu Ser Pro Leu Leu Ile Gly Gln
   1430 1435 1440
   Gln Ser Thr Val Ser Asp Val Pro Arg Asp Leu Glu Val Val Ala
   1445 1450 1455
   Ala Thr Pro Thr Ser Leu Leu Ile Ser Trp Asp Ala Pro Ala Val
   1460 1465 1470
   Thr Val Arg Tyr Tyr Arg Ile Thr Tyr Gly Glu Thr Gly Gly Asn
   1475 1480 1485
   Ser Pro Val Gln Glu Phe Thr Val Pro Gly Ser Lys Ser Thr Ala
   1490 1495 1500
   Thr Ile Ser Gly Leu Lys Pro Gly Val Asp Tyr Thr Ile Thr Val
   1505 1510 1515
   Tyr Ala Val Thr Gly Arg Gly Asp Ser Pro Ala Ser Ser Lys Pro
   1520 1525 1530
   Ile Ser Ile Asn Tyr Arg Thr Glu Ile Asp Lys Pro Ser Gln Met
   1535 1540 1545
   Gln Val Thr Asp Val Gln Asp Asn Ser Ile Ser Val Lys Trp Leu
   1550 1555 1560
   Pro Ser Ser Ser Pro Val Thr Gly Tyr Arg Val Thr Thr Thr Pro
   1565 1570 1575
   Lys Asn Gly Pro Gly Pro Thr Lys Thr Lys Thr Ala Gly Pro Asp
   1580 1585 1590
   Gln Thr Glu Met Thr Ile Glu Gly Leu Gln Pro Thr Val Glu Tyr
   1595 1600 1605
   Val Val Ser Val Tyr Ala Gln Asn Pro Ser Gly Glu Ser Gln Pro
   1610 1615 1620
   Leu Val Gln Thr Ala Val Thr Asn Ile Asp Arg Pro Lys Gly Leu
   1625 1630 1635
   Ala Phe Thr Asp Val Asp Val Asp Ser Ile Lys Ile Ala Trp Glu
   1640 1645 1650
   Ser Pro Gln Gly Gln Val Ser Arg Tyr Arg Val Thr Tyr Ser Ser
   1655 1660 1665
   Pro Glu Asp Gly Ile His Glu Leu Phe Pro Ala Pro Asp Gly Glu
   1670 1675 1680
   Glu Asp Thr Ala Glu Leu Gln Gly Leu Arg Pro Gly Ser Glu Tyr
   1685 1690 1695
   Thr Val Ser Val Val Ala Leu His Asp Asp Met Glu Ser Gln Pro
   1700 1705 1710
   Leu Ile Gly Thr Gln Ser Thr Ala Ile Pro Ala Pro Thr Asp Leu
   1715 1720 1725
   Lys Phe Thr Gln Val Thr Pro Thr Ser Leu Ser Ala Gln Trp Thr
   1730 1735 1740
   Pro Pro Asn Val Gln Leu Thr Gly Tyr Arg Val Arg Val Thr Pro
   1745 1750 1755
   Lys Glu Lys Thr Gly Pro Met Lys Glu Ile Asn Leu Ala Pro Asp
   1760 1765 1770
   Ser Ser Ser Val Val Val Ser Gly Leu Met Val Ala Thr Lys Tyr
   1775 1780 1785
   Glu Val Ser Val Tyr Ala Leu Lys Asp Thr Leu Thr Ser Arg Pro
   1790 1795 1800
   Ala Gln Gly Val Val Thr Thr Leu Glu Asn Val Ser Pro Pro Arg
   1805 1810 1815
   Arg Ala Arg Val Thr Asp Ala Thr Glu Thr Thr Ile Thr Ile Ser
   1820 1825 1830
   Trp Arg Thr Lys Thr Glu Thr Ile Thr Gly Phe Gln Val Asp Ala
   1835 1840 1845
   Val Pro Ala Asn Gly Gln Thr Pro Ile Gln Arg Thr Ile Lys Pro
   1850 1855 1860
   Asp Val Arg Ser Tyr Thr Ile Thr Gly Leu Gln Pro Gly Thr Asp
   1865 1870 1875
   Tyr Lys Ile Tyr Leu Tyr Thr Leu Asn Asp Asn Ala Arg Ser Ser
   1880 1885 1890
   Pro Val Val Ile Asp Ala Ser Thr Ala Ile Asp Ala Pro Ser Asn
   1895 1900 1905
   Leu Arg Phe Leu Ala Thr Thr Pro Asn Ser Leu Leu Val Ser Trp
   1910 1915 1920
   Gln Pro Pro Arg Ala Arg Ile Thr Gly Tyr Ile Ile Lys Tyr Glu
   1925 1930 1935
   Lys Pro Gly Ser Pro Pro Arg Glu Val Val Pro Arg Pro Arg Pro
   1940 1945 1950
   Gly Val Thr Glu Ala Thr Ile Thr Gly Leu Glu Pro Gly Thr Glu
   1955 1960 1965
   Tyr Thr Ile Tyr Val Ile Ala Leu Lys Asn Asn Gln Lys Ser Glu
   1970 1975 1980
   Pro Leu Ile Gly Arg Lys Lys Thr Asp Glu Leu Pro Gln Leu Val
   1985 1990 1995
   Thr Leu Pro His Pro Asn Leu His Gly Pro Glu Ile Leu Asp Val
   2000 2005 2010
   Pro Ser Thr Val Gln Lys Thr Pro Phe Val Thr His Pro Gly Tyr
   2015 2020 2025
   Asp Thr Gly Asn Gly Ile Gln Leu Pro Gly Thr Ser Gly Gln Gln
   2030 2035 2040
   Pro Ser Val Gly Gln Gln Met Ile Phe Glu Glu His Gly Phe Arg
   2045 2050 2055
   Arg Thr Thr Pro Pro Thr Thr Ala Thr Pro Ile Arg His Arg Pro
   2060 2065 2070
   Arg Pro Tyr Pro Pro Asn Val Gly Glu Glu Ile Gln Ile Gly His
   2075 2080 2085
   Ile Pro Arg Glu Asp Val Asp Tyr His Leu Tyr Pro His Gly Pro
   2090 2095 2100
   Gly Leu Asn Pro Asn Ala Ser Thr Gly Gln Glu Ala Leu Ser Gln
   2105 2110 2115
   Thr Thr Ile Ser Trp Ala Pro Phe Gln Asp Thr Ser Glu Tyr Ile
   2120 2125 2130
   Ile Ser Cys His Pro Val Gly Thr Asp Glu Glu Pro Leu Gln Phe
   2135 2140 2145
   Arg Val Pro Gly Thr Ser Thr Ser Ala Thr Leu Thr Gly Leu Thr
   2150 2155 2160
   Arg Gly Ala Thr Tyr Asn Ile Ile Val Glu Ala Leu Lys Asp Gln
   2165 2170 2175
   Gln Arg His Lys Val Arg Glu Glu Val Val Thr Val Gly Asn Ser
   2180 2185 2190
   Val Asn Glu Gly Leu Asn Gln Pro Thr Asp Asp Ser Cys Phe Asp
   2195 2200 2205
   Pro Tyr Thr Val Ser His Tyr Ala Val Gly Asp Glu Trp Glu Arg
   2210 2215 2220
   Met Ser Glu Ser Gly Phe Lys Leu Leu Cys Gln Cys Leu Gly Phe
   2225 2230 2235
   Gly Ser Gly His Phe Arg Cys Asp Ser Ser Arg Trp Cys His Asp
   2240 2245 2250
   Asn Gly Val Asn Tyr Lys Ile Gly Glu Lys Trp Asp Arg Gln Gly
   2255 2260 2265
   Glu Asn Gly Gln Met Met Ser Cys Thr Cys Leu Gly Asn Gly Lys
   2270 2275 2280
   Gly Glu Phe Lys Cys Asp Pro His Glu Ala Thr Cys Tyr Asp Asp
   2285 2290 2295
   Gly Lys Thr Tyr His Val Gly Glu Gln Trp Gln Lys Glu Tyr Leu
   2300 2305 2310
   Gly Ala Ile Cys Ser Cys Thr Cys Phe Gly Gly Gln Arg Gly Trp
   2315 2320 2325
   Arg Cys Asp Asn Cys Arg Arg Pro Gly Gly Glu Pro Ser Pro Glu
   2330 2335 2340
   Gly Thr Thr Gly Gln Ser Tyr Asn Gln Tyr Ser Gln Arg Tyr His
   2345 2350 2355
   Gln Arg Thr Asn Thr Asn Val Asn Cys Pro Ile Glu Cys Phe Met
   2360 2365 2370
   Pro Leu Asp Val Gln Ala Asp Arg Glu Asp Ser Arg Glu
   2375 2380 2385
<210> 136
   <211> 339
   <212> PRT
   <213> Homo sapiens
<300>
   <308> Swissprot/P07858
   <309> 1988-08-01
   <313> (1)..(339)
<400> 136
<210> 137
   <211> 1249
   <212> PRT
   <213> Homo sapiens
<300>
   <308> Swissprot/P29144
   <309> 1992-12-01
   <313> (1)..(1249)
<400> 137
   Met Ala Thr Ala Ala Thr Glu Glu Pro Phe Pro Phe His Gly Leu Leu
   1               5                   10                  15
   Pro Lys Lys Glu Thr Gly Ala Ala Ser Phe Leu Cys Arg Tyr Pro Glu
               20                  25                  30
   Tyr Asp Gly Arg Gly Val Leu Ile Ala Val Leu Asp Thr Gly Val Asp
           35                  40                  45
   Pro Gly Ala Pro Gly Met Gln Val Thr Thr Asp Gly Lys Pro Lys Ile
       50                  55                  60
   Val Asp Ile Ile Asp Thr Thr Gly Ser Gly Asp Val Asn Thr Ala Thr
   65                  70                  75                  80
   Glu Val Glu Pro Lys Asp Gly Glu Ile Val Gly Leu Ser Gly Arg Val
                   85                  90                  95
   Leu Lys Ile Pro Ala Ser Trp Thr Asn Pro Ser Gly Lys Tyr His Ile
               100                 105                 110
   Gly Ile Lys Asn Gly Tyr Asp Phe Tyr Pro Lys Ala Leu Lys Glu Arg
           115                 120                 125
   Ile Gln Lys Glu Arg Lys Glu Lys Ile Trp Asp Pro Val His Arg Val
       130                 135                 140
   Ala Leu Ala Glu Ala Cys Arg Lys Gln Glu Glu Phe Asp Val Ala Asn
   145                 150                 155                 160
   Asn Gly Ser Ser Gln Ala Asn Lys Leu Ile Lys Glu Glu Leu Gln Ser
                   165                 170                 175
   Gln Val Glu Leu Leu Asn Ser Phe Glu Lys Lys Tyr Ser Asp Pro Gly
               180                 185                 190
   Pro Val Tyr Asp Cys Leu Val Trp His Asp Gly Glu Val Trp Arg Ala
           195                 200                 205
   Cys Ile Asp Ser Asn Glu Asp Gly Asp Leu Ser Lys Ser Thr Val Leu
       210                 215                 220
   Arg Asn Tyr Lys Glu Ala Gln Glu Tyr Gly Ser Phe Gly Thr Ala Glu
   225 230 235 240
   Met Leu Asn Tyr Ser Val Asn Ile Tyr Asp Asp Gly Asn Leu Leu Ser
   245 250 255
   Ile Val Thr Ser Gly Gly Ala His Gly Thr His Val Ala Ser Ile Ala
   260 265 270
   Ala Gly His Phe Pro Glu Glu Pro Glu Arg Asn Gly Val Ala Pro Gly
   275 280 285
   Ala Gln Ile Leu Ser Ile Lys Ile Gly Asp Thr Arg Leu Ser Thr Met
   290 295 300
   Glu Thr Gly Thr Gly Leu Ile Arg Ala Met Ile Glu Val Ile Asn His
   305 310 315 320
   Lys Cys Asp Leu Val Asn Tyr Ser Tyr Gly Glu Ala Thr His Trp Pro
   325 330 335
   Asn Ser Gly Arg Ile Cys Glu Val Ile Asn Glu Ala Val Trp Lys His
   340 345 350
   Asn Ile Ile Tyr Val Ser Ser Ala Gly Asn Asn Gly Pro Cys Leu Ser
   355 360 365
   Thr Val Gly Cys Pro Gly Gly Thr Thr Ser Ser Val Ile Gly Val Gly
   370 375 380
   Ala Tyr Val Ser Pro Asp Met Met Val Ala Glu Tyr Ser Leu Arg Glu
   385 390 395 400
   Lys Leu Pro Ala Asn Gln Tyr Thr Trp Ser Ser Arg Gly Pro Ser Ala
   405 410 415
   Asp Gly Ala Leu Gly Val Ser Ile Ser Ala Pro Gly Gly Ala Ile Ala
   420 425 430
   Ser Val Pro Asn Trp Thr Leu Arg Gly Thr Gln Leu Met Asn Gly Thr
   435 440 445
   Ser Met Ser Ser Pro Asn Ala Cys Gly Gly Ile Ala Leu Ile Leu Ser
   450 455 460
   Gly Leu Lys Ala Asn Asn Ile Asp Tyr Thr Val His Ser Val Arg Arg
   465 470 475 480
   Ala Leu Glu Asn Thr Ala Val Lys Ala Asp Asn Ile Glu Val Phe Ala
   485 490 495
   Gln Gly His Gly Ile Ile Gln Val Asp Lys Ala Tyr Asp Tyr Leu Val
   500 505 510
   Gln Asn Thr Ser Phe Ala Asn Lys Leu Gly Phe Thr Val Thr Val Gly
   515 520 525
   Asn Asn Arg Gly Ile Tyr Leu Arg Asp Pro Val Gln Val Ala Ala Pro
   530 535 540
   Ser Asp His Gly Val Gly Ile Glu Pro Val Phe Pro Glu Asn Thr Glu
   545 550 555 560
   Asn Ser Glu Lys Ile Ser Leu Gln Leu His Leu Ala Leu Thr Ser Asn
   565 570 575
   Ser Ser Trp Val Gln Cys Pro Ser His Leu Glu Leu Met Asn Gln Cys
   580 585 590
   Arg His Ile Asn Ile Arg Val Asp Pro Arg Gly Leu Arg Glu Gly Leu
   595 600 605
   His Tyr Thr Glu Val Cys Gly Tyr Asp Ile Ala Ser Pro Asn Ala Gly
   610 615 620
   Pro Leu Phe Arg Val Pro Ile Thr Ala Val Ile Ala Ala Lys Val Asn
   625 630 635 640
   Glu Ser Ser His Tyr Asp Leu Ala Phe Thr Asp Val His Phe Lys Pro
   645 650 655
   Gly Gln Ile Arg Arg His Phe Ile Glu Val Pro Glu Gly Ala Thr Trp
   660 665 670
   Ala Glu Val Thr Val Cys Ser Cys Ser Ser Glu Val Ser Ala Lys Phe
   675 680 685
   Val Leu His Ala Val Gln Leu Val Lys Gln Arg Ala Tyr Arg Ser His
   690 695 700
   Glu Phe Tyr Lys Phe Cys Ser Leu Pro Glu Lys Gly Thr Leu Thr Glu
   705 710 715 720
   Ala Phe Pro Val Leu Gly Gly Lys Ala Ile Glu Phe Cys Ile Ala Arg
   725 730 735
   Trp Trp Ala Ser Leu Ser Asp Val Asn Ile Asp Tyr Thr Ile Ser Phe
   740 745 750
   His Gly Ile Val Cys Thr Ala Pro Gln Leu Asn Ile His Ala Ser Glu
   755 760 765
   Gly Ile Asn Arg Phe Asp Val Gln Ser Ser Leu Lys Tyr Glu Asp Leu
   770 775 780
   Ala Pro Cys Ile Thr Leu Lys Asn Trp Val Gln Thr Leu Arg Pro Val
   785 790 795 800
   Ser Ala Lys Thr Lys Pro Leu Gly Ser Arg Asp Val Leu Pro Asn Asn
   805 810 815
   Arg Gln Leu Tyr Glu Met Val Leu Thr Tyr Asn Phe His Gln Pro Lys
   820 825 830
   Ser Gly Glu Val Thr Pro Ser Cys Pro Leu Leu Cys Glu Leu Leu Tyr
   835 840 845
   Glu Ser Glu Phe Asp Ser Gln Leu Trp Ile Ile Phe Asp Gln Asn Lys
   850 855 860
   Arg Gln Met Gly Ser Gly Asp Ala Tyr Pro His Gln Tyr Ser Leu Lys
   865 870 875 880
   Leu Glu Lys Gly Asp Tyr Thr Ile Arg Leu Gln Ile Arg His Glu Gln
   885 890 895
   Ile Ser Asp Leu Glu Arg Leu Lys Asp Leu Pro Phe Ile Val Ser His
   900 905 910
   Arg Leu Ser Asn Thr Leu Ser Leu Asp Ile His Glu Asn His Ser Phe
   915 920 925
   Ala Leu Leu Gly Lys Lys Lys Ser Ser Asn Leu Thr Leu Pro Pro Lys
   930 935 940
   Tyr Asn Gln Pro Phe Phe Val Thr Ser Leu Pro Asp Asp Lys Ile Pro
   945 950 955 960
   Lys Gly Ala Gly Pro Gly Cys Tyr Leu Ala Gly Ser Leu Thr Leu Ser
   965 970 975
   Lys Thr Glu Leu Gly Lys Lys Ala Asp Val Ile Pro Val His Tyr Tyr
   980 985 990
   Leu Ile Pro Pro Pro Thr Lys Thr Lys Asn Gly Ser Lys Asp Lys Glu
   995 1000 1005
   Lys Asp Ser Glu Lys Glu Lys Asp Leu Lys Glu Glu Phe Thr Glu
   1010 1015 1020
   Ala Leu Arg Asp Leu Lys Ile Gln Trp Met Thr Lys Leu Asp Ser
   1025 1030 1035
   Ser Asp Ile Tyr Asn Glu Leu Lys Glu Thr Tyr Pro Asn Tyr Leu
   1040 1045 1050
   Pro Leu Tyr Val Ala Arg Leu His Gln Leu Asp Ala Glu Lys Glu
   1055 1060 1065
   Arg Met Lys Arg Leu Asn Glu Ile Val Asp Ala Ala Asn Ala Val
   1070 1075 1080
   Ile Ser His Ile Asp Gln Thr Ala Leu Ala Val Tyr Ile Ala Met
   1085 1090 1095
   Lys Thr Asp Pro Arg Pro Asp Ala Ala Thr Ile Lys Asn Asp Met
   1100 1105 1110
   Asp Lys Gln Lys Ser Thr Leu Val Asp Ala Leu Cys Arg Lys Gly
   1115 1120 1125
   Cys Ala Leu Ala Asp His Leu Leu His Thr Gln Ala Gln Asp Gly
   1130 1135 1140
   Ala Ile Ser Thr Asp Ala Glu Gly Lys Glu Glu Glu Gly Glu Ser
   1145 1150 1155
   Pro Leu Asp Ser Leu Ala Glu Thr Phe Trp Glu Thr Thr Lys Trp
   1160 1165 1170
   Thr Asp Leu Phe Asp Asn Lys Val Leu Thr Phe Ala Tyr Lys His
   1175 1180 1185
   Ala Leu Val Asn Lys Met Tyr Gly Arg Gly Leu Lys Phe Ala Thr
   1190 1195 1200
   Lys Leu Val Glu Glu Lys Pro Thr Lys Glu Asn Trp Lys Asn Cys
   1205 1210 1215
   Ile Gln Leu Met Lys Leu Leu Gly Trp Thr His Cys Ala Ser Phe
   1220 1225 1230
   Thr Glu Asn Trp Leu Pro Ile Met Tyr Pro Pro Asp Tyr Cys Val
   1235 1240 1245
   Phe
<210> 138
   <211> 433
   <212> PRT
   <213> Homo sapiens
<300>
   <308> Swissprot/Q99538
   <309> 1997-11-01
   <313> (1)..(433)
<400> 138
   Met Val Trp Lys Val Ala Val Phe Leu Ser Val Ala Leu Gly Ile Gly
   1               5                   10                  15
   Ala Val Pro Ile Asp Asp Pro Glu Asp Gly Gly Lys His Trp Val Val
   20 25 30
   Ile Val Ala Gly Ser Asn Gly Trp Tyr Asn Tyr Arg His Gln Ala Asp
   35 40 45
   Ala Cys His Ala Tyr Gln Ile Ile His Arg Asn Gly Ile Pro Asp Glu
   50 55 60
   Gln Ile Val Val Met Met Tyr Asp Asp Ile Ala Tyr Ser Glu Asp Asn
   65 70 75 80
   Pro Thr Pro Gly Ile Val Ile Asn Arg Pro Asn Gly Thr Asp Val Tyr
   85 90 95
   Gln Gly Val Pro Lys Asp Tyr Thr Gly Glu Asp Val Thr Pro Gln Asn
   100 105 110
   Phe Leu Ala Val Leu Arg Gly Asp Ala Glu Ala Val Lys Gly Ile Gly
   115 120 125
   Ser Gly Lys Val Leu Lys Ser Gly Pro Gln Asp His Val Phe Ile Tyr
   130 135 140
   Phe Thr Asp His Gly Ser Thr Gly Ile Leu Val Phe Pro Asn Glu Asp
   145 150 155 160
   Leu His Val Lys Asp Leu Asn Glu Thr Ile His Tyr Met Tyr Lys His
   165 170 175
   Lys Met Tyr Arg Lys Met Val Phe Tyr Ile Glu Ala Cys Glu Ser Gly
   180 185 190
   Ser Met Met Asn His Leu Pro Asp Asn Ile Asn Val Tyr Ala Thr Thr
   195 200 205
   Ala Ala Asn Pro Arg Glu Ser Ser Tyr Ala Cys Tyr Tyr Asp Glu Lys
   210 215 220
   Arg Ser Thr Tyr Leu Gly Asp Trp Tyr Ser Val Asn Trp Met Glu Asp
   225 230 235 240
   Ser Asp Val Glu Asp Leu Thr Lys Glu Thr Leu His Lys Gln Tyr His
   245 250 255
   Leu Val Lys Ser His Thr Asn Thr Ser His Val Met Gln Tyr Gly Asn
   260 265 270
   Lys Thr Ile Ser Thr Met Lys Val Met Gln Phe Gln Gly Met Lys Arg
   275 280 285
   Lys Ala Ser Ser Pro Val Pro Leu Pro Pro Val Thr His Leu Asp Leu
   290 295 300
   Thr Pro Ser Pro Asp Val Pro Leu Thr Ile Met Lys Arg Lys Leu Met
   305 310 315 320
   Asn Thr Asn Asp Leu Glu Glu Ser Arg Gln Leu Thr Glu Glu Ile Gln
   325 330 335
   Arg His Leu Asp Ala Arg His Leu Ile Glu Lys Ser Val Arg Lys Ile
   340 345 350
   Val Ser Leu Leu Ala Ala Ser Glu Ala Glu Val Glu Gln Leu Leu Ser
   355 360 365
   Glu Arg Ala Pro Leu Thr Gly His Ser Cys Tyr Pro Glu Ala Leu Leu
   370 375 380
   His Phe Arg Thr His Cys Phe Asn Trp His Ser Pro Thr Tyr Glu Tyr
   385 390 395 400
   Ala Leu Arg His Leu Tyr Val Leu Val Asn Leu Cys Glu Lys Pro Tyr
   405 410 415
   Pro Leu His Arg Ile Lys Leu Ser Met Asp His Val Cys Leu Gly His
   420 425 430
   Tyr
<210> 139
   <211> 342
   <212> PRT
   <213> Homo sapiens
<300>
   <308> Swissprot/P25105
   <309> 1992-05-01
   <313> (1)..(342)
<400> 139
   Met Glu Pro His Asp Ser Ser His Met Asp Ser Glu Phe Arg Tyr Thr
   1 5 10 15
   Leu Phe Pro Ile Val Tyr Ser Ile Ile Phe Val Leu Gly Val Ile Ala
   20 25 30
   Asn Gly Tyr Val Leu Trp Val Phe Ala Arg Leu Tyr Pro Cys Lys Lys
   35 40 45
   Phe Asn Glu Ile Lys Ile Phe Met Val Asn Leu Thr Met Ala Asp Met
   50 55 60
   Leu Phe Leu Ile Thr Leu Pro Leu Trp Ile Val Tyr Tyr Gln Asn Gln
   65 70 75 80
   Gly Asn Trp Ile Leu Pro Lys Phe Leu Cys Asn Val Ala Gly Cys Leu
                       85                  90                  95
   Phe Phe Ile Asn Thr Tyr Cys Ser Val Ala Phe Leu Gly Val Ile Thr
               100                 105                 110
   Tyr Asn Arg Phe Gln Ala Val Thr Arg Pro Ile Lys Thr Ala Gln Ala
           115                 120                 125
   Asn Thr Arg Lys Arg Gly Ile Ser Leu Ser Leu Val Ile Trp Val Ala
       130                 135                 140
   Ile Val Gly Ala Ala Ser Tyr Phe Leu Ile Leu Asp Ser Thr Asn Thr
   145                 150                 155                 160
   Val Pro Asp Ser Ala Gly Ser Gly Asn Val Thr Arg Cys Phe Glu His
                   165                 170                 175
   Tyr Glu Lys Gly Ser Val Pro Val Leu Ile Ile His Ile Phe Ile Val
               180                 185                 190
   Phe Ser Phe Phe Leu Val Phe Leu Ile Ile Leu Phe Cys Asn Leu Val
           195                 200                 205
   Ile Ile Arg Thr Leu Leu Met Gln Pro Val Gln Gln Gln Arg Asn Ala
       210                 215                 220
   Glu Val Lys Arg Arg Ala Leu Trp Met Val Cys Thr Val Leu Ala Val
   225                 230                 235                 240
   Phe Ile Ile Cys Phe Val Pro His His Val Val Gln Leu Pro Trp Thr
                   245                 250                 255
   Leu Ala Glu Leu Gly Phe Gln Asp Ser Lys Phe His Gln Ala Ile Asn
               260                 265                 270
   Asp Ala His Gln Val Thr Leu Cys Leu Leu Ser Thr Asn Cys Val Leu
           275                 280                 285
   Asp Pro Val Ile Tyr Cys Phe Leu Thr Lys Lys Phe Arg Lys His Leu
       290                 295                 300
   Thr Glu Lys Phe Tyr Ser Met Arg Ser Ser Arg Lys Cys Ser Arg Ala
   305                 310                 315                 320
   Thr Thr Asp Thr Val Thr Glu Val Val Val Pro Phe Asn Gln Ile Pro
                   325                 330                 335
   Gly Asn Ser Leu Lys Asn
               340
<210> 140
   <211> 359
   <212> PRT
   <213> Homo sapiens
<300>
   <308> Swissprot/Q92187
   <309> 1997-11-01
   <313> (1)..(359)
<400> 140
   Met Arg Ser Ile Arg Lys Arg Trp Thr Ile Cys Thr Ile Ser Leu Leu
   1 5 10 15
   Leu Ile Phe Tyr Lys Thr Lys Glu Ile Ala Arg Thr Glu Glu His Gln
   20 25 30
   Glu Thr Gln Leu Ile Gly Asp Gly Glu Leu Ser Leu Ser Arg Ser Leu
   35 40 45
   Val Asn Ser Ser Asp Lys Ile Ile Arg Lys Ala Gly Ser Ser Ile Phe
   50 55 60
   Gln His Asn Val Glu Gly Trp Lys Ile Asn Ser Ser Leu Val Leu Glu
   65 70 75 80
   Ile Arg Lys Asn Ile Leu Arg Phe Leu Asp Ala Glu Arg Asp Val Ser
   85 90 95
   Val Val Lys Ser Ser Phe Lys Pro Gly Asp Val Ile His Tyr Val Leu
   100 105 110
   Asp Arg Arg Arg Thr Leu Asn Ile Ser His Asp Leu His Ser Leu Leu
   115 120 125
   Pro Glu Val Ser Pro Met Lys Asn Arg Arg Phe Lys Thr Cys Ala Val
   130 135 140
   Val Gly Asn Ser Gly Ile Leu Leu Asp Ser Glu Cys Gly Lys Glu Ile
   145 150 155 160
   Asp Ser His Asn Phe Val Ile Arg Cys Asn Leu Ala Pro Val Val Glu
   165 170 175
   Phe Ala Ala Asp Val Gly Thr Lys Ser Asp Phe Ile Thr Met Asn Pro
   180 185 190
   Ser Val Val Gln Arg Ala Phe Gly Gly Phe Arg Asn Glu Ser Asp Arg
   195 200 205
   Glu Lys Phe Val His Arg Leu Ser Met Leu Asn Asp Ser Val Leu Trp
   210 215 220
   Ile Pro Ala Phe Met Val Lys Gly Gly Glu Lys His Val Glu Trp Val
   225 230 235 240
   Asn Ala Leu Ile Leu Lys Asn Lys Leu Lys Val Arg Thr Ala Tyr Pro
   245 250 255
   Ser Leu Arg Leu Ile His Ala Val Arg Gly Tyr Trp Leu Thr Asn Lys
   260 265 270
   Val Pro Ile Lys Arg Pro Ser Thr Gly Leu Leu Met Tyr Thr Leu Ala
   275 280 285
   Thr Arg Phe Cys Asp Glu Ile His Leu Tyr Gly Phe Trp Pro Phe Pro
   290 295 300
   Lys Asp Leu Asn Gly Lys Ala Val Lys Tyr His Tyr Tyr Asp Asp Leu
   305 310 315 320
   Lys Tyr Arg Tyr Phe Ser Asn Ala Ser Pro His Arg Met Pro Leu Glu
   325 330 335
   Phe Lys Thr Leu Asn Val Leu His Asn Arg Gly Ala Leu Lys Leu Thr
   340 345 350
   Thr Gly Lys Cys Val Lys Gln
   355
<210> 141
   <211> 218
   <212> PRT
   <213> Homo sapiens
<300>
   <308> Swissprot/Q15907
   <309> 1998-07-15
   <313> (1)..(218)
<400> 141